# EUROPEAN PATENT APPLICATION

(11) **EP 3 070 110 A1**
(43) Date of publication of application: **21.09.2016**
(21) Application number: 16000962.7
(22) Date of filing: 11.10.2013
(51) Int. Cl.: C08F 220/56, C08F 293/00, G01N 33/543

(54) **POLYMERS HAVING ORTHOGONAL REACTIVE GROUPS AND USES THEREOF**

(30) Priority: 12.10.2012 US 201261713329 P
(62) Divisional of application: 13789070.3
(71) Applicant: NVS Technologies Inc., Menlo Park, CA 94025 (US)
(72) Inventor: Lau, Aldrich N.K., Palo Alto, CA 94301 (US); Eason, Robert G., Los Gatos, CA 95033 (US)
(74) Representative: Grünecker Patent- und Rechtsanwälte PartG mbB

(57) **Abstract**

Polymers having orthogonal reactive groups and solid supports comprising polymers immobilized thereto are provided. The polymers find utility in any number of applications including immobilizing analyte molecules to solid supports for high throughput assays.

## Description

### STATEMENT OF GOVERNMENT INTEREST

Partial funding of the work described herein was provided by the U.S. Department of Homeland Security under Contract No. HSHQDC-10-C-00053. The U.S. Government has certain rights in this invention.

### BACKGROUND

### Field of the Invention

The present invention is generally directed to novel polymers, solid supports comprising the polymers and methods for use of the same.

### Description of the Related Art

Bioassays are used to probe for the presence and/or quantity of an analyte material in a biological sample. In surface-based assays, such as DNA microarrays, the analyte species-is-generally captured and detected on a solid support or substrate. The use of DNA microarrays has become widely adopted in the study of gene expression and genotyping due to the ability to monitor large numbers of genes simultaneously (Schena et al., Science 270:467-470 (1995); Pollack et al., Nat. Genet. 23:41-46 (1999)). Surface arrays can also be fabricated using other binding moieties such as carbohydrates, antibodies, proteins, haptens or aptamers, in order to facilitate a wide variety of bioassays in array format.

An effective functionalized material for bioassay applications must have adequate capacity to immobilize a sufficient amount of an analyte from relevant samples in order to provide a suitable signal when subjected to detection (e.g., polymerase chain reaction). Suitable functionalized materials must also provide a highly reproducible surface in order to be gainfully applied to profiling experiments, particularly in assay formats in which the sample and the control must be analyzed on disparate support surfaces with which they are associated, e.g., different supports or different locations on the same support. For example, supports that are not based on a highly reproducible surface chemistry can result in significant errors when undertaking assays (e.g., profiling comparisons), due to variations from support to support or different locations on the same support.

Surface arrays (e.g., "DNA chips") have been prepared by using polymers to attach the analyte to the solid support. In general, arrays that include a polymer are formed by the in situ polymerization of precursor monomers or prepolymers on a solid substrate (e.g., bead, particle, plate, etc.). The selectivity and reproducibility of arrays that include organic polymers is frequently highly dependent upon a number of experimental variables including, monomer concentration, monomer ratios, initiator type and concentration, solvent evaporation rate, ambient humidity (in the case when the solvent is water), crosslinker type and concentration, purity of the monomers/crosslinker/solvent, laboratory temperature, pipetting time, sparging conditions, reaction temperature (in the case of thermal polymerizations), reaction humidity, uniformity of ultraviolet radiation (in the case of UV photopolymerization) and ambient oxygen conditions. While many of these parameters can be controlled in a manufacturing setting, it is difficult if not impossible to control all of these parameters. As a result, in situ polymerization results in relatively poor reproducibility from spot-to-spot, chip-to-chip and lot-to-lot. Furthermore, residual monomer/crosslinker/initiator and by products call for additional purification steps that may not be easily implemented.

In addition, while a significant amount of work has been expended upon the development of array surfaces using silica based substrates, e.g., glass, quartz, fused silica, and silicon (See, e.g., D. Cuschin et al., Anal. Biochem. 1997, 250, 203-211; G.M. Harbers et al., Chem. Mater. 2007, 19, 4405-4414; and US Patent Nos. 6,790,613, to Shi et al., 5,932,711, to Boles et al., 6,994,972, to Bardhan, et al., 7,781,203, to Frutos et al., and 7,217,512 and 7,541,146 to Lewis et al.), certain advantages are derived from using less expensive, more easily manufactured substrates, such as polymeric substrates. However, additional challenges have been encountered both in the selection and preparation of such substrates for bioassay purposes. For example, polymeric substrates often suffer worse problems as a result of additional surface functionalization, such as increased auto fluorescence, increased hydrophobicity, as well as challenges in attaching or associating the in situ polymerized coating to the underlying polymer substrate.

Accordingly, while progress has been made in this field, there remains a need in the art for improved functionalized solid substrates, polymers and methods for attaching analytes to these solid substrates and solid support comprising such polymers for use in various assays, such as DNA microarrays. The present invention fulfills this need and provides further related advantages.

### BRIEF SUMMARY

In brief, the present invention is generally directed to polymers having orthogonal reactive groups. The polymers find utility in any number of applications, including immobilizing a capture probe on a solid substrate for use in analytical assays. Solid substrates comprising reactive groups suitable for reaction or interaction with the polymers, and solid supports comprising the polymers and optional capture probes are also provided. The presently disclosed polymers, solid substrates and solid supports are useful in a variety of analytical applications, for example DNA and protein microarrays for use in individual point of care situations (doctor's office, emergency room, home, in the field, etc.), high throughput testing and other applications

The presently described polymers, solid substrates, solid supports and related methods provide a number of advantages in various embodiments. For example, in certain embodiments the reactive groups described herein for immobilizing the polymers to the solid substrates are substantially inert except under specific conditions provided during the immobilization reaction, insuring a predictable and optimal level of reactivity during the surface coating process. Some embodiments also employ click chemistry for immobilizing a polymer to a solid substrate, and such chemistry is substantially pH-insensitive and produces limited or no reaction by-products. Related advantages are obtained in certain embodiments wherein functional groups having click reactivity are employed for conjugating the polymers to a capture probe (e.g., biomolecule such as DNA or an oligonucleotide) via click chemistry.

Further advantages are realized since the polymers comprise orthogonal reactive groups. For example, embodiments of the polymers include polymers having one or more reactive groups specific for immobilization ("immobilization group") of the polymer to the solid substrate and one or more functional groups specific for conjugation to a capture probe ("conjugation group"), such as a polynucleotide or antibody. Thus, only one type of reactive group can react with the capture probe (e.g., an amine-modified biomolecule) during array spotting. Any unreacted immobilization group (e.g., first reactive group) which is present on the polymer-coated surface is inert under conditions used for conjugation and will not affect bioassay performance. Certain embodiments of the present invention also employ coupling reactions which are nearly quantitative and almost instantaneous (minutes), whereas prior methods can take hours and may only couple a portion of the reactive groups owing to competitive hydrolysis.

In one embodiment, a polymer is provided, the polymer comprising A, B and C subunits, wherein:
the A subunit comprises, at each occurrence, independently a first reactive group having a reactivity specific for reaction with a target functional group;
the B subunit comprises, at each occurrence, independently a hydrophilic functional group; and
the C subunit comprises, at each occurrence, independently a second reactive group having a reactivity specific for covalent conjugation to a capture probe,
wherein the reactivity of the first reactive group and the second reactive group are orthogonal to each other.

The target functional group may be a functional group located on the exterior and/or interior surfaces of a solid substrate, for example on the surface of a porous monolith.

In another embodiment, the invention provides a solid support comprising a polymer immobilized to a solid substrate, wherein the polymer comprises B, D and E subunits, wherein:
the B subunit comprises, at each occurrence, independently a hydrophilic functional group;
the D subunit comprises, at each occurrence, independently a reactive group having a reactivity specific for covalent conjugation to an capture probe or the D subunit comprises a covalent bond to a capture probe; and
the E subunit comprises, at each occurrence, independently a click functional group and a covalent bond to either the solid substrate or an optional linker (L⁴) disposed between the D subunit and the solid substrate.

Solid substrates comprising reactive groups for immobilizing polymers thereto, compounds (e.g., polymers) and methods for preparation of such solid supports and related analytical methods are also provided.

These and other aspects of the invention will be apparent upon reference to the following detailed description. To this end, various references are set forth herein which describe in more detail certain background information, procedures, compounds and/or compositions, and are each hereby incorporated by reference in their entirety.

### BRIEF DESCRIPTION OF THE DRAWINGS

In the figures, identical reference numbers identify similar elements. The sizes and relative positions of elements in the figures are not necessarily drawn to scale and some of these elements are arbitrarily enlarged and positioned to improve figure legibility. Further, the particular shapes of the elements as drawn are not intended to convey any information regarding the actual shape of the particular elements, and have been solely selected for ease of recognition in the figures.
Figure 1 is a schematic showing immobilization of biomolecules on the surface of a solid support according to an embodiment of the invention.
Figures 2A, 2B and 2C illustrate exemplary methods.

### DETAILED DESCRIPTION OF THE INVENTION

In the following description, certain specific details are set forth in order to provide a thorough understanding of various embodiments of the invention. However, one skilled in the art will understand that the invention may be practiced without these details.

Unless the context requires otherwise, throughout the present specification and claims, the word "comprise" and variations thereof, such as, "comprises" and "comprising" are to be construed in an open, inclusive sense, that is as "including, but not limited to".

Reference throughout this specification to "one embodiment" or "an embodiment" means that a particular feature, structure or characteristic described in connection with the embodiment is included in at least one embodiment of the present invention. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

"Amino" refers to the -NH₂ radical.

"Cyano" refers to the -CN radical.

"Hydroxy" or "hydroxyl" refers to the -OH radical.

"Imino" refers to the =NH substituent.

"Nitro" refers to the -NO₂ radical.

"Oxo" refers to the =O substituent.

"Thioxo" refers to the =S substituent.

"Alkyl" refers to a straight or branched hydrocarbon chain radical consisting solely of carbon and hydrogen atoms, which is saturated or unsaturated (*i.e.,* contains one or more double and/or triple bonds), having from one to twelve carbon atoms (C₁-C₁₂ alkyl), preferably one to eight carbon atoms (C₁-C₈ alkyl) or one to six carbon atoms (C₁-C₆ alkyl), and which is attached to the rest of the molecule by a single bond, e.g., methyl, ethyl, *n*-propyl, 1-methylethyl (*iso*-propyl), *n*-butyl, *n*-pentyl, 1,1-dimethylethyl (*t*-butyl), 3-methylhexyl, 2-methylhexyl, ethenyl, prop-1-enyl, but-1-enyl, pent-1-enyl, penta-1,4-dienyl, ethynyl, propynyl, butynyl, pentynyl, hexynyl, and the like. Unless stated otherwise specifically in the specification, an alkyl group may be optionally substituted.

"Alkylene" or "alkylene chain" refers to a straight or branched divalent hydrocarbon chain linking the rest of the molecule to a radical group, consisting solely of carbon and hydrogen, which is saturated or unsaturated (*i.e.,* contains one or more double and/or triple bonds), and having from one to twelve carbon atoms, e.g., methylene, ethylene, propylene, *n*-butylene, ethenylene, propenylene, *n*-butenylene, propynylene, *n*-butynylene, and the like. The alkylene chain is attached to the rest of the molecule through a single or double bond and to the radical group through a single or double bond. The points of attachment of the alkylene chain to the rest of the molecule and to the radical group can be through one carbon or any two carbons within the chain. Unless stated otherwise specifically in the specification, an alkylene chain may be optionally substituted.

"Alkoxy" refers to a radical of the formula -ORₐ where Rₐ is an alkyl radical as defined above containing one to twelve carbon atoms. Unless stated otherwise specifically in the specification, an alkoxy group may be optionally substituted.

"Alkylamino" refers to a radical of the formula -NHRₐ or -NRₐRₐ where each Rₐ is, independently, an alkyl radical as defined above containing one to twelve carbon atoms. Unless stated otherwise specifically in the specification, an alkylamino group may be optionally substituted.

"Thioalkyl" refers to a radical of the formula -SRₐ where Rₐ is an alkyl radical as defined above containing one to twelve carbon atoms. Unless stated otherwise specifically in the specification, a thioalkyl group may be optionally substituted.

"Aryl" refers to a hydrocarbon ring system radical comprising hydrogen, 6 to 18 carbon atoms and at least one aromatic ring. For purposes of this invention, the aryl radical may be a monocyclic, bicyclic, tricyclic or tetracyclic ring system, which may include fused or bridged ring systems. Aryl radicals include, but are not limited to, aryl radicals derived from aceanthrylene, acenaphthylene, acephenanthrylene, anthracene, azulene, benzene, chrysene, fluoranthene, fluorene, *as*-indacene, *s*-indacene, indane, indene, naphthalene, phenalene, phenanthrene, pleiadene, pyrene, and triphenylene. Unless stated otherwise specifically in the specification, the term "aryl" or the prefix "ar-" (such as in "aralkyl") is meant to include aryl radicals that are optionally substituted.

"Aralkyl" refers to a radical of the formula -R_{b}-R_{c} where R_{b} is an alkylene chain as defined above and R_{c} is one or more aryl radicals as defined above, for example, benzyl, diphenylmethyl and the like. Unless stated otherwise specifically in the specification, an aralkyl group may be optionally substituted.

"Cycloalkyl" or "carbocyclic ring" refers to a stable non-aromatic monocyclic or polycyclic hydrocarbon radical consisting solely of carbon and hydrogen atoms, which may include fused or bridged ring systems, having from three to fifteen carbon atoms, preferably having from three to ten carbon atoms, and which is saturated or unsaturated and attached to the rest of the molecule by a single bond. Monocyclic radicals include, for example, cyclopropyl, cyclobutyl, cyclopentyl, cyclohexyl, cycloheptyl, and cyclooctyl. Polycyclic radicals include, for example, adamantyl, norbornyl, decalinyl, 7,7-dimethyl-bicyclo[2.2.1]heptanyl, and the like. Unless otherwise stated specifically in the specification, a cycloalkyl group may be optionally substituted.

"Cycloalkylalkyl" refers to a radical of the formula -R_{b}R_{d} where R_{b} is an alkylene chain as defined above and R_{d} is a cycloalkyl radical as defined above. Unless stated otherwise specifically in the specification, a cycloalkylalkyl group may be optionally substituted.

"Fused" refers to any ring structure described herein which is fused to an existing ring structure in the compounds of the invention. When the fused ring is a heterocyclyl ring or a heteroaryl ring, any carbon atom on the existing ring structure which becomes part of the fused heterocyclyl ring or the fused heteroaryl ring may be replaced with a nitrogen atom.

"Halo" or "halogen" refers to bromo, chloro, fluoro or iodo.

"Haloalkyl" refers to an alkyl radical, as defined above, that is substituted by one or more halo radicals, as defined above, e.g., trifluoromethyl, difluoromethyl, trichloromethyl, 2,2,2-trifluoroethyl, 1,2-difluoroethyl, 3-bromo-2-fluoropropyl, 1,2-dibromoethyl, and the like. Unless stated otherwise specifically in the specification, a haloalkyl group may be optionally substituted.

"Heterocyclyl" or "heterocyclic ring" refers to a stable 3- to 18-membered non-aromatic ring radical which consists of two to twelve carbon atoms and from one to six heteroatoms selected from the group consisting of nitrogen, oxygen and sulfur. Unless stated otherwise specifically in the specification, the heterocyclyl radical may be a monocyclic, bicyclic, tricyclic or tetracyclic ring system, which may include fused or bridged ring systems; and the nitrogen, carbon or sulfur atoms in the heterocyclyl radical may be optionally oxidized; the nitrogen atom may be optionally quaternized; and the heterocyclyl radical may be partially or fully saturated. Examples of such heterocyclyl radicals include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. Unless stated otherwise specifically in the specification, Unless stated otherwise specifically in the specification, a heterocyclyl group may be optionally substituted.

"*N*-heterocyclyl" refers to a heterocyclyl radical as defined above containing at least one nitrogen and where the point of attachment of the heterocyclyl radical to the rest of the molecule is through a nitrogen atom in the heterocyclyl radical. Unless stated otherwise specifically in the specification, a *N*-heterocyclyl group may be optionally substituted.

"Heterocyclylalkyl" refers to a radical of the formula -R_{b}Rₑ where R_{b} is an alkylene chain as defined above and Rₑ is a heterocyclyl radical as defined above, and if the heterocyclyl is a nitrogen-containing heterocyclyl, the heterocyclyl may be attached to the alkyl radical at the nitrogen atom. Unless stated otherwise specifically in the specification, a heterocyclylalkyl group may be optionally substituted.

"Heteroaryl" refers to a 5- to 14-membered ring system radical comprising hydrogen atoms, one to thirteen carbon atoms, one to six heteroatoms selected from the group consisting of nitrogen, oxygen and sulfur, and at least one aromatic ring. For purposes of this invention, the heteroaryl radical may be a monocyclic, bicyclic, tricyclic or tetracyclic ring system, which may include fused or bridged ring systems; and the nitrogen, carbon or sulfur atoms in the heteroaryl radical may be optionally oxidized; the nitrogen atom may be optionally quaternized. Examples include, but are not limited to, azepinyl, acridinyl, benzimidazolyl, benzothiazolyl, benzindolyl, benzodioxolyl, benzofuranyl, benzooxazolyl, benzothiazolyl, benzothiadiazolyl, benzo[*b*][1,4]dioxepinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzotriazolyl, benzo[4,6]imidazo[1,2-a]pyridinyl, carbazolyl, cinnolinyl, dibenzofuranyl, dibenzothiophenyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyridinyl, oxadiazolyl, 2-oxoazepinyl, oxazolyl, oxiranyl, 1-oxidopyridinyl, 1-oxidopyrimidinyl, 1-oxidopyrazinyl, 1-oxidopyridazinyl, 1-phenyl-1*H*-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, quinazolinyl, quinoxalinyl, quinolinyl, quinuclidinyl, isoquinolinyl, tetrahydroquinolinyl, thiazolyl, thiadiazolyl, triazolyl, tetrazolyl, triazinyl, and thiophenyl (i.e. thienyl). Unless stated otherwise specifically in the specification, a heteroaryl group may be optionally substituted.

"*N*-heteroaryl" refers to a heteroaryl radical as defined above containing at least one nitrogen and where the point of attachment of the heteroaryl radical to the rest of the molecule is through a nitrogen atom in the heteroaryl radical. Unless stated otherwise specifically in the specification, an *N*-heteroaryl group may be optionally substituted.

"Heteroarylalkyl" refers to a radical of the formula -R_{b}R_{f} where R_{b} is an alkylene chain as defined above and R_{f} is a heteroaryl radical as defined above. Unless stated otherwise specifically in the specification, a heteroarylalkyl group may be optionally substituted.

The term "substituted" used herein means any of the above groups (i.e., alkyl, alkylene, alkoxy, alkylamino, thioalkyl, aryl, aralkyl, cycloalkyl, cycloalkylalkyl, haloalkyl, heterocyclyl, *N*-heterocyclyl, heterocyclylalkyl, heteroaryl, *N*-heteroaryl and/or heteroarylalkyl) wherein at least one hydrogen atom is replaced by a bond to a non-hydrogen atoms such as, but not limited to: a halogen atom such as F, Cl, Br, and I; an oxygen atom in groups such as hydroxyl groups, alkoxy groups, and ester groups; a sulfur atom in groups such as thiol groups, thioalkyl groups, sulfone groups, sulfonyl groups, and sulfoxide groups; a nitrogen atom in groups such as amines, amides, alkylamines, dialkylamines, arylamines, alkylarylamines, diarylamines, N-oxides, imides, and enamines; a silicon atom in groups such as trialkylsilyl groups, dialkylarylsilyl groups, alkyldiarylsilyl groups, and triarylsilyl groups; and other heteroatoms in various other groups. "Substituted" also means any of the above groups in which one or more hydrogen atoms are replaced by a higher-order bond (e.g., a double- or triple-bond) to a heteroatom such as oxygen in oxo, carbonyl, carboxyl, and ester groups; and nitrogen in groups such as imines, oximes, hydrazones, and nitriles. For example, "substituted" includes any of the above groups in which one or more hydrogen atoms are replaced with -NR_{g}Rₕ, -NR_{g}C(=O)Rₕ, -NR_{g}C(=O)NR_{g}Rₕ, -NR_{g}C(=O)ORₕ, -NR_{g}SO₂Rₕ, -OC(=O)NR_{g}Rₕ, -ORg, -SRg, -SOR_{g}, -SO₂R_{g}, -OSO₂R_{g}, -SO₂OR_{g}, =NSO₂R_{g}, and -SO₂NR_{g}Rₕ. "Substituted also means any of the above groups in which one or more hydrogen atoms are replaced with -C(=O)Rg, -C(=O)OR_{g}, -C(=O)NR_{g}Rₕ, -CH₂SO₂R_{g}, -CH₂SO₂NR_{g}Rₕ. In the foregoing, Rg and Rₕ are the same or different and independently hydrogen, alkyl, alkoxy, alkylamino, thioalkyl, aryl, aralkyl, cycloalkyl, cycloalkylalkyl, haloalkyl, heterocyclyl, *N*-heterocyclyl, heterocyclylalkyl, heteroaryl, *N*-heteroaryl and/or heteroarylalkyl. "Substituted" further means any of the above groups in which one or more hydrogen atoms are replaced by a bond to an amino, cyano, hydroxyl, imino, nitro, oxo, thioxo, halo, alkyl, alkoxy, alkylamino, thioalkyl, aryl, aralkyl, cycloalkyl, cycloalkylalkyl, haloalkyl, heterocyclyl, *N*-heterocyclyl, heterocyclylalkyl, heteroaryl, *N*-heteroaryl and/or heteroarylalkyl group. In addition, each of the foregoing substituents may also be optionally substituted with one or more of the above substituents.

"Stable compound" and "stable structure" are meant to indicate a compound that is sufficiently robust to survive isolation to a useful degree of purity from a reaction mixture.

"Optional" or "optionally" means that the subsequently described event of circumstances may or may not occur, and that the description includes instances where said event or circumstance occurs and instances in which it does not. For example, "optionally substituted aryl" means that the aryl radical may or may not be substituted and that the description includes both substituted aryl radicals and aryl radicals having no substitution.

Often crystallizations or precipitations produce a solvate of the compound of the invention. As used herein, the term "solvate" refers to an aggregate that comprises one or more molecules of a compound of the invention with one or more molecules of solvent. The solvent may be water, in which case the solvate may be a hydrate. Alternatively, the solvent may be an organic solvent. Thus, the compounds of the present invention may exist as a hydrate, including a monohydrate, dihydrate, hemihydrate, sesquihydrate, trihydrate, tetrahydrate and the like, as well as the corresponding solvated forms. The compound of the invention may be true solvates, while in other cases, the compound of the invention may merely retain adventitious water or be a mixture of water plus some adventitious solvent.

The compounds of the invention, or their salts or tautomers may contain one or more asymmetric centers and may thus give rise to enantiomers, diastereomers, and other stereoisomeric forms that may be defined, in terms of absolute stereochemistry, as (R)- or (*S*)- or, as (D)- or (L)- for amino acids. The present invention is meant to include all such possible isomers, as well as their racemic and optically pure forms. Optically active (+) and (-), (R)- and (*S*)-, or (D)- and (L)-isomers may be prepared using chiral synthons or chiral reagents, or resolved using conventional techniques, for example, chromatography and fractional crystallization. Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC). When the compounds described herein contain olefinic double bonds or other centers of geometric asymmetry, and unless specified otherwise, it is intended that the compounds include both E and Z geometric isomers. Likewise, all tautomeric forms are also intended to be included.

A "stereoisomer" refers to a compound made up of the same atoms bonded by the same bonds but having different three-dimensional structures, which are not interchangeable. The present invention contemplates various stereoisomers and mixtures thereof and includes "enantiomers", which refers to two stereoisomers whose molecules are nonsuperimposeable mirror images of one another.

A "tautomer" refers to a proton shift from one atom of a molecule to another atom of the same molecule. The present invention includes tautomers of any said compounds.

A "polymer" refers to a molecule having one or more repeating subunit. The subunits ("monomers") may be the same or different and may occur in any position or order within the polymer. Polymers may be of natural or synthetic origin. The present invention includes various types of polymers, including polymers having ordered repeating subunits, random co-polymers and block co-polymers.

A "random co-polymer" refers to a polymer comprising more than one type of subunit wherein the subunits are connected in random order along a polymer chain. Random co-polymers may comprise any number of different subunits. That is, a random copolymer is a polymer in which the probability of finding a given monomeric unit at any given site in the polymer chain is independent of the nature of the adjacent units. A "statistical copolymer" is a copolymer in which the sequence of monomer subunits follows a statistical rule. If the probability of finding a given type of monomer residue at a particular point in the polymer chain is equal to the mole fraction of that monomer residue in the chain, then the polymer may be referred to as a truly random copolymer.

In certain embodiments, the polymers described herein are "random co-terpolymers", meaning that the polymers comprise three different subunits connected in random order. The individual subunits may be present in any molar ratio in the random polymer, for example each subunit may be present in from about 0.1 molar % to about 99.8 molar percent, relative to moles of other subunits in the polymer. In some embodiments, the subunits of a random co-terpolymer may be represented by the following general structure: 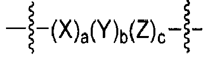 wherein X, Y and Z are independently unique subunits, and a, b and c are integers representing the number of each subunit within the polymer. For ease of illustration, the above structure depicts a linear connectivity of X, Y and Z; however, it is to be emphasized that random co-polymers (e.g., random co-terpolymers) of the present invention are not limited to polymers having the depicted connectivity of subunits, and the subunits in a random polymer can be connected in any random sequence, and the copolymer and co-terpolymer can be branched.

A "block co-polymer" refers to a polymer comprising repeating blocks of two or more subunits.

A "functional group" is a portion of a molecule having a specific type of reactivity (e.g., acidic, basic, nucleophilic, electrophilic, etc). "Reactive groups" are a type of functional group. Non-limiting examples of functional groups include azides, alkynes, amine, alcohols and the like. A "target functional group" is any functional group with which another functional group is intended to react. A "hydrophilic functional group" is a functional group having hydrophilic properties. A hydrophilic functional group generally tends to increase the overall molecule's solubility in polar solvents such as water.

"Covalent conjugation" refers to formation of a covalent bond by reaction of two or more functional groups.

"Orthogonal" or "orthogonal reactivity" refers to reactivity properties of functional groups and/or reactive groups. If two reactive groups have orthogonal reactivity it is meant that one of the reactive groups will react with a target functional group under conditions in which the second reactive group does not react to a substantial extent with the target functional group, and vice versa.

"Initiator" is a molecule used to initiate a polymerization reaction. Initiators for use in preparation of the disclosed polymers are well known in the art. Representative initiators include, but are not limited to, initiators useful in atom transfer radical polymerization, living polymerization, the AIBN family of initiators and benzophenone initiators. An "initiator residue" is that portion of an initiator which becomes attached to a polymer through radical or other mechanisms. In some embodiments, initiator residues are attached to the terminal end(s) of the disclosed polymers.

"Click chemistry" refers to reactions that have at least the following characteristics: (1) exhibits functional group orthogonality (i.e., the functional portion reacts only with a reactive site that is complementary to the functional portion, without reacting with other reactive sites); and (2) the resulting bond is irreversible (i.e., once the reactants have been reacted to form products, decomposition of the products into reactants is difficult) or in some instances the resulting bond may be reversible (i.e., revert back to reactants under appropriate conditions). Optionally, "click" chemistry can further have one or more of the following characteristics: (1) stereospecificity; (2) reaction conditions that do not involve stringent purification, atmospheric control, and the like; (3) readily available starting materials and reagents; (4) ability to utilize benign or no solvent; (5) product isolation by crystallization or distillation; (6) physiological stability; (7) large thermodynamic driving force (e.g., 10-20 kcal/mol); (8) a single reaction product; (9) high (e.g., greater than 50%) chemical yield; and (10) substantially no byproducts or byproducts that are environmentally benign byproducts.

Examples of reactions using "click" functionalities can include, but are not limited to, addition reactions, cycloaddition reactions, nucleophilic substitutions, and the like. Examples of cycloaddition reactions can include Huisgen 1,3-dipolar cycloaddition, Cu(I) catalyzed azide-alkyne cycloaddition, and Diels-Alder reactions. Examples of addition reactions include addition reactions to carbon-carbon double bonds such as epoxidation and dihydroxylation. Nucleophilic substitution examples can include nucleophilic substitution to strained rings such as epoxy and aziridine compounds. Other examples can include formation of ureas and amides. Some additional description of click chemistry can be found in Huisgen, Angew. Chem. Int. Ed., Vol. 2, No. 11, 1963, pp. 633-696; Lewis et al., Angew. Chem. Int. Ed., Vol. 41, No. 6, 2002, pp. 1053-1057; Rodionov et al., Angew. Chem. Int. Ed., Vol. 44, 2005, pp. 2210-2215; Punna et al., Angew. Chem. Int. Ed., Vol. 44, 2005, pp. 2215-2220; Li et al., J. Am. Chem. Soc., Vol. 127, 2005, pp. 14518-14524; Himo et al., J. Am. Chem. Soc., Vol. 127, 2005, pp. 210-216; Noodleman et al., Chem. Rev., Vol. 104, 2004, pp. 459-508; Sun et al., Bioconjugate Chem., Vol. 17, 2006, pp. 52-57; and Fleming et al., Chem. Mater., Vol. 18, 2006, pp. 2327-2334, the contents of which are hereby incorporated by reference herein in their entireties.

"Click reactivity" refers to a functional group capable of reacting under click chemistry conditions.

A "click functional group" is a functional group which results from reaction of two functional groups having click reactivity, for example a triazole moiety and the like.

"Solid substrate" refers to any solid substance having an outer surface. Generally the outer surface will have functional groups capable of immobilizing a polymer (e.g., by covalent attachment) or the outer surface will have functional groups which can be modified so that the surface is capable of immobilizing a polymer. Suitable solid substrates include any of the solid substrates known in the art such as glass and polymer supports. Solid substrates may be optically transparent, opaque or partially optically transparent. Solid substrates include planar substrates (i.e., shapes having at least one flat surface) as well as beads, particles, porous matrices, porous monoliths and the like. Examples of specific, but non-limiting, solid substrates are provided herein below.

A "solid support" as used herein refers to a solid substrate which comprises a polymer and/or capture probe immobilized thereto. Typically, the polymers will be immobilized to the solid substrate via covalent bonds, such as through azide functional groups or amide bonds resulting from reaction of an azide and alkyne or reactive ester and amine, respectively.

"Immobilizing" or "immobilized" with respect to a solid support includes covalent conjugation, non-specific association, ionic interactions and other means of adhering a substance (e.g., polymer) to a solid substrate.

A reactive group having "reactivity specific for" a target functional group means the reactive group will react preferentially with the target functional group under the reaction conditions and side reactions with other functional groups are minimized or absent. Similarly, a reactive group having reactivity specific for conjugation with a capture probe means the reactive group will conjugate preferentially with the capture probe under the reaction conditions and side reactions with other functional groups are minimized or absent.

"Analyte" or "analyte molecule" refers to a compound or molecule which is the subject of an analysis, for example an analyte molecule may be of unknown structure and the analysis includes identification of the structure. Analyte molecules include any number of common molecules, including DNA, proteins, peptides and carbohydrates, organic and inorganic molecules, metals (including radioactive isotopes), and the like. Analytes include viruses, bacteria, plasmodium, fungi, as well as metals and bio-warfare, bio-hazard and chemical warfare materials. Analytes also include analyte probes as defined herein.

A "capture probe" is a molecule capable of interacting with an analyte molecule, for example by hydrogen bonding (e.g., DNA hybridization), sequestering, covalent bonding, ionic interactions, and the like. Exemplary capture probes include oligonucleotides which are capable of sequence specific binding (hybridization) with oligonucleotide probes or flaps, oligosaccharides (e.g. lechtins) and proteins. In some embodiments capture probes comprise a fluorophore label. For example the capture probe may comprise a fluorophore label and an analyte molecule (e.g., analyte probe) may comprise a quencher, and the presence of the analyte molecule is detected by an absence of a fluorescent signal from the capture probe (since the fluorescence is quenched upon interaction with the quencher). In related embodiments, the capture probe comprises a quencher. In these embodiments, the fluorescence of a fluorescently labeled analyte molecule is quenched upon capture by the capture probe.

"Probe" or "analyte probe" refers to a molecule used for indirect identification of an analyte molecule. For example, a probe may carry sequence information which uniquely identifies an analyte molecule. Exemplary probes include oligonucleotides and the like.

"Flap" refers to an optional portion of a probe. In certain embodiments a flap contains sequence information to uniquely identify the probe (and thus the analyte molecule). A flap may be cleaved from the remainder of the probe (for example under PCR conditions) and hybridize with a capture probe on a solid support. The presence of the bound flap on the solid support indicates the presence of a particular analyte.

### A. Polymers

As noted above, one aspect of the present disclosure is directed to polymers having orthogonal reactive groups (i.e., "orthogonal polymers"). The polymers may be used in a variety of applications. In certain embodiments, the polymers may be covalently attached to a solid substrate by reaction of a first reactive group with a target functional group on a solid substrate, resulting in a solid support. In related embodiments, the substrate-bound polymers are used for covalently attaching a capture probe to a solid support by conjugation of a second reactive group with a target functional group on the capture probe. A capture probe covalently attached to the immobilized polymer, is capable of capturing/sequestering an analyte molecule from a sample, such as an analyte molecule dissolved in an aqueous sample. In certain embodiments, the mechanism of capturing may include complexation, hydrogen bonding (e.g. DNA hybridization) and/or covalent or non-covalent (e.g., antibody-streptavidin interaction) interactions.

In other embodiments, compounds useful for preparation of solid substrates having reactive groups for immobilizing polymers thereto are also provided. The solid supports comprising the polymers are useful in variety of methods, including high throughput analysis of polynucleotides in an array format. Methods for analysis in this regard are known in the art and are described in detail herein below.

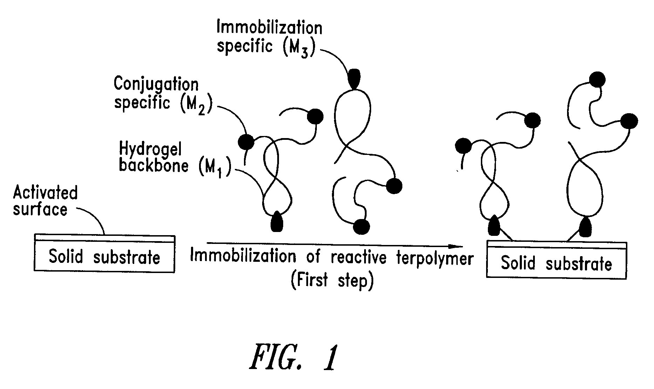

Certain embodiments of the disclosed polymers provide advantages over other means for immobilizing capture probes to a solid support. For example, since embodiments of the polymers include orthogonal reactive groups, precise control over the linkage between the solid support and the capture probe can be obtained. As illustrated in Figure 1, the polymers comprise one or more subunit for immobilization to the solid substrate, one or more subunit for conjugation to a capture probe (e.g., biomolecule, polynucleotide, DNA and the like) and one or more subunit for controlling the hydrophilicity of the polymer (noted as "hydrogel backbone" in Figure 1). The number and location of immobilization subunits and conjugation subunits can be modified to obtain a reactive surface (i.e., the surface of a solid support comprising one or more a reactive groups for conjugation to a capture probe) having the desired morphology and concentration of capture probes.

In contrast to the presently described methods and polymers, prior methods using polymers which lack orthogonal reactive groups cannot exert such control over the morphology of the reactive surface and random morphologies which can change from batch to batch are often obtained. Further, by controlling the number of hydrophilic subunits (e.g., subunit B) in the polymer, a desired hydrophilicity of the polymer can be obtained. Since conjugating a capture probe to a polymer immobilized on a solid substrate often requires reactions between an active surface and a capture probe dissolved in a solvent (i.e., an "interfacial reaction"), the ability to control the water contact angle of the active surface results in more facile interfacial reactions since the water contact angle can be altered according to the hydrophilic or hydrophobic nature of the reaction solvent.

Accordingly, in one embodiment the present invention provides a polymer comprising one or more subunits for immobilization to a solid substrate and one or more subunits for conjugation to a capture probe. The polymer may optionally further include subunits for controlling the hydrophilicity of the polymer. In one embodiment the present invention provides a polymer comprising A, B and C subunits, wherein:
the A subunit, at each occurrence, independently comprises a first reactive group having a reactivity specific for reaction with a target functional group;
the B subunit, at each occurrence, independently comprises a hydrophilic functional group; and
the C subunit, at each occurrence, independently comprises a second reactive group having a reactivity specific for covalent conjugation to a capture probe,
wherein the reactivity of the first reactive group and the second reactive group are orthogonal to each other.

In some embodiments, the target functional group is on a solid substrate, for example on an outer surface or within a porous network of the solid substrate.

In certain embodiments, the polymer is a random co-polymer, such as a random co-terpolymer. For example, in some embodiments the polymer has the following structure (I):

T¹-(A)ₓ(B)_{y}(C)_{z}-T² (I)

wherein:
T¹ and T² are each independently absent or polymer terminal groups selected from H, alkyl and an initiator residue; and
x, y and z are independently an integer from 1 to 350,000.

In some embodiments of the foregoing, x, y and z are independently an integer from 1 to 50,000,

The depicted connectivity of the A, B and C subunits of structure (I) is in no way limiting, and the actual structure of the polymer of structure (I) include embodiments wherein the polymer of structure (I) is a random co-polymer wherein each of the A, B, and C subunits occur at any position in the polymer.

In other embodiments, the polymer has the following structure (Ia):

T¹-[(A)ₓ₁(B)_{y1}(C)_{z1}]ₙ-T² (Ia)

wherein:
A, B and C are present at least once in the polymer of structure (Ia);
T¹ and T² are each independently absent or polymer terminal groups selected from H, alkyl and an initiator residue;
x1, y1 and z1 are, at each occurrence, independently 0 or 1; and
n is an integer from 1 to 700,000.

In other embodiments of the foregoing, n is an integer from 1 to 150,000

When present, the initiator residues result from reaction of an initiator and the polymer. The exact structure of the initiator residues can vary and will depend on the type of initiator used during the polymerization reaction. In certain embodiments, the initiator residue has one of the following structures: 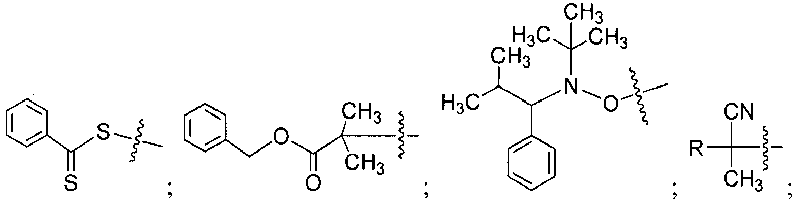 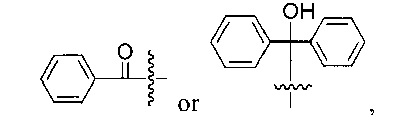 wherein R is an alkyl group comprising from 1-10 carbon atoms and optional nitrogen and oxygen atoms.

In any of the foregoing embodiments, the polymer comprises an A subunit at a terminal position of the polymer.

In other of the foregoing embodiments, the A subunit, at each occurrence, independently has the following structure (II): 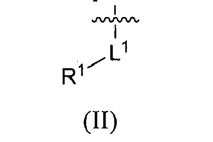 wherein:
R¹ is the first reactive group; and
L¹ is an optional linker up to 100 atoms in length.

In still other of the foregoing embodiments, the A subunit has, at each occurrence, independently the following structure (III): 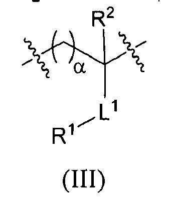 wherein:
R¹ is the first reactive group;
R² is hydrogen or alkyl
L¹ is an optional linker up to 100 atoms in length; and
α is an integer ranging from 0 to 10.

In further of the foregoing embodiments, L¹ comprises alkylene, ester, alkylene oxide, amide, imide, ether or dithio moieties, or combinations thereof. In other embodiments, L¹ is absent.

In some of the foregoing embodiments, α is 1. In some other of the foregoing embodiments, R² is H.

The actual structure or reactivity of the first reactive group is not limited, provided it has orthogonal reactivity to the second reactive group. In some embodiments, R¹ is an alkyne, alkylsilyl-protected alkyne, azide, nitrile, thiol, alkene, maleimide, epoxide, aziridine or thiirane functional group. In certain embodiments, R¹ is an alkyne or azide functional group.

In further of the foregoing embodiments, the A subunit has, at each occurrence, independently one of the following structures: 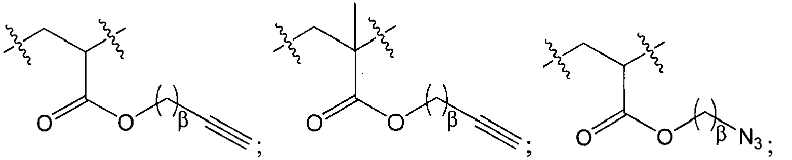 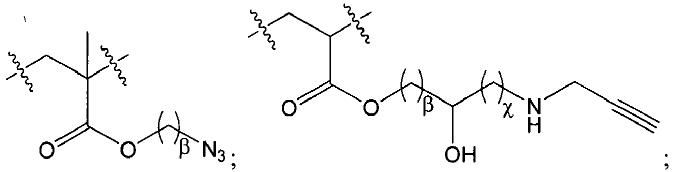 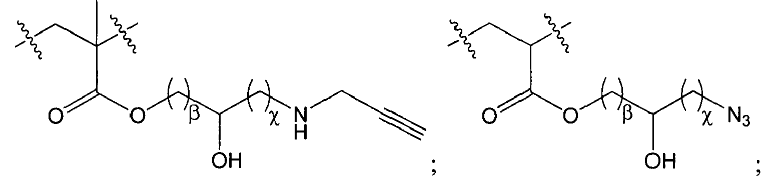 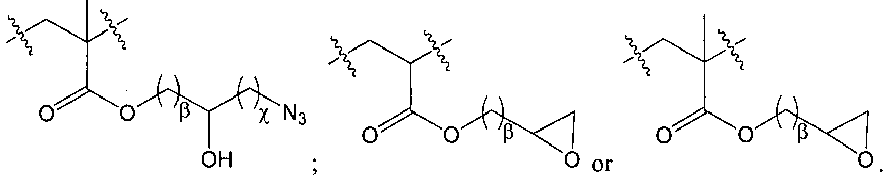 wherein β and χ are each independently integers ranging from 1 to 5.

In other embodiments, β is 1 or 3, and in other aspects χ is 1.

In other more specific embodiments of the foregoing, the A subunit has, at each occurrence, independently one of the following structures:

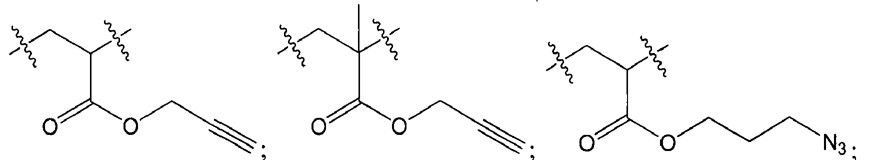

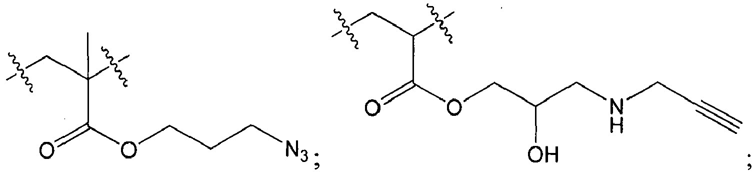

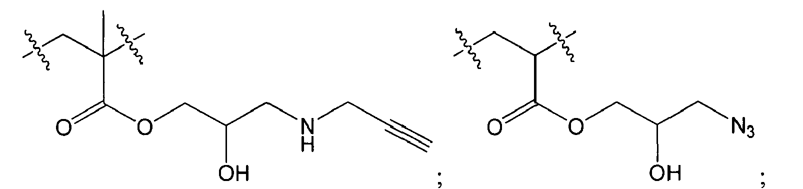

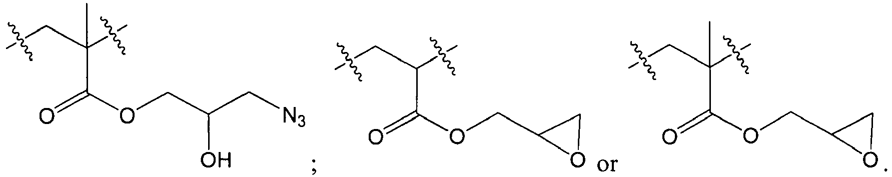

In other embodiments, at least one A subunit is at a terminal position covalently bound to T¹. For example, in some embodiments T¹ is H.

In other aspects at least one of the A subunits the following structure: 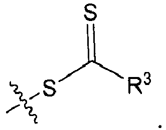 wherein:
R³ is aryl. For example, such structures may be useful for preparation of polymers comprising alkyne moieties. For example, at least one of the A subunits may have the following structure in certain embodiments:

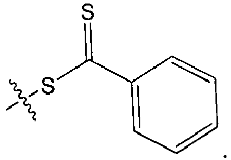

In some other of the foregoing embodiments, the first reactive group has, at each occurrence, independently click reactivity. For example, in certain embodiments the first reactive group is, at each occurrence, independently specific for reaction with an azide. For example, the first reactive group is, at each occurrence, independently an alkyne in some embodiments. In other embodiments, the first reactive group is, at each occurrence, independently specific for reaction with an alkyne. For example, in some embodiments, the first reactive group is, at each occurrence, independently an azide.

In certain other embodiments, the reactivity of the first reactive group is, at each occurrence, independently specific for an amine group on the solid substrate. In other embodiments, the first reactive group is, at each occurrence, independently a N-hydroxysuccinimide (NHS) ester, N-hydroxysulfosuccinimide (sulfo-NHS) ester, succinimidyl acetylthioacetate (SATA), carbodiimide, hydroxymethyl phosphine, maleimide, arylester, imidoester, isocyanate, psoralen, vinyl sulfone, pyridyl disulfide, azlactone or benzophenone. In some more specific embodiments, the first reactive group is, at each occurrence, independently a NHS ester, azlactone or arylester.

In some other of the foregoing embodiments, the first reactive group has, at each occurrence, independently one of the following structures: 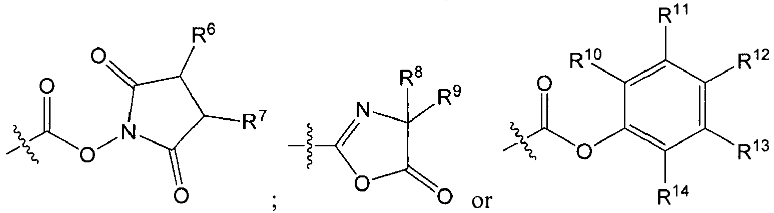 wherein:
R⁶, R⁷, R⁸ and R⁹ are each independently H or alkyl; and
R¹⁰, R¹¹, R¹², R¹³ and R¹⁴ are each independently, H, an electron withdrawing group, -NCS, -NCO, -CO₂H, -SO₃H, -L'-poly or salts thereof, wherein L' is an optional linker up to 100 atoms in length and poly is a water soluble polymer.

In some embodiments, each of R⁶, R⁷, R⁸ and R⁹ are H. In other embodiments, at least one of R¹⁰, R¹¹, R¹², R¹³ or R¹⁴ is an electron withdrawing group. In other embodiments, each of R¹⁰, R¹¹, R¹² , R¹³ or R¹⁴ is an electron 0withdrawing group. For example, in some embodiments the electron withdrawing group is halogen, nitro or nitrile, and in other more specific embodiments the electron withdrawing group is fluoro.

In some of the foregoing embodiments, at least one of the first reactive groups has the following structure:

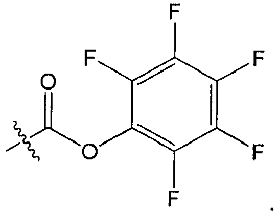

In some other embodiments, each of the first reactive groups has the above structure.

In other of the foregoing embodiments, the A subunit has, at each occurrence, independently one of the following structures:

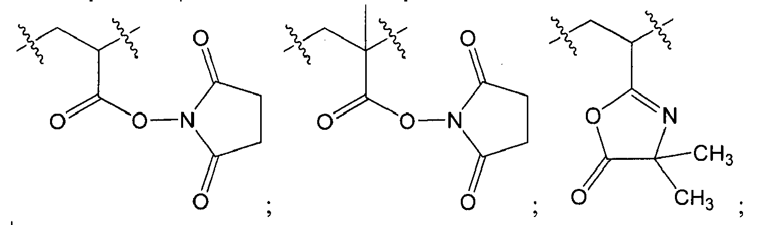

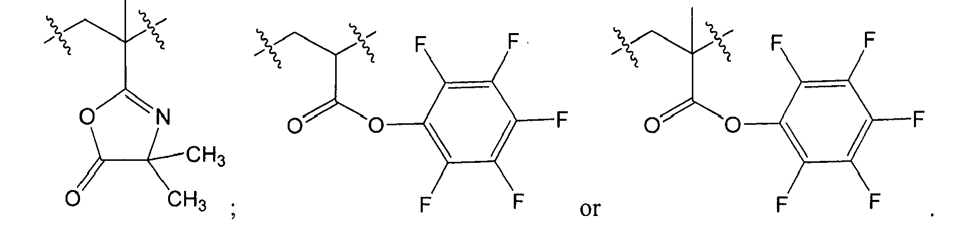

In some more specific aspects, the A subunits has, at each occurrence, independently one of the following structures:

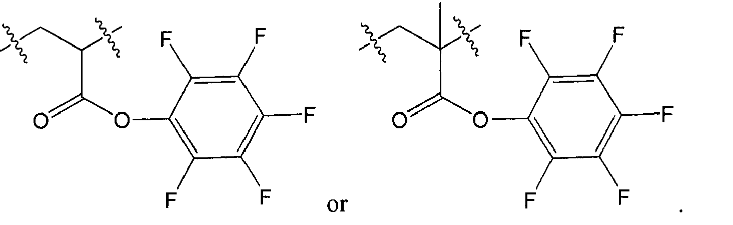

In some other of the foregoing embodiments, the C subunit has, at each occurrence, independently the following structure (IV): 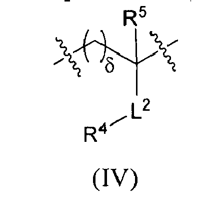 wherein:
R⁴ is the second reactive group;
R⁵ is hydrogen or alkyl;
L² is an optional linker up to 100 atoms in length; and
δ is an integer ranging from 0 to 10.

In some of the foregoing embodiments, L² comprises alkylene, ester, alkylene oxide, amide, imide, ether or dithio moieties, or combinations thereof. In other embodiments, L² is absent.

In some other embodiments of the foregoing, δ is 1, and in other embodiments R⁵ is H.

In certain embodiments, the reactivity of the second reactive group is, at each occurrence, independently specific for an amine group in the capture probe. In other embodiments, the second reactive group is, at each occurrence, independently a N-hydroxysuccinimide (NHS) ester, N-hydroxysulfosuccinimide (sulfo-NHS) ester, succinimidyl acetylthioacetate (SATA), carbodiimide, hydroxymethyl phosphine, maleimide, arylester, imidoester, isocyanate, psoralen, vinyl sulfone, pyridyl disulfide, azlactone or benzophenone. In some more specific embodiments, the second reactive group is, at each occurrence, independently a NHS ester, azlactone or arylester.

In some other of the foregoing embodiments, the second reactive groups has, at each occurrence, independently one of the following structures: 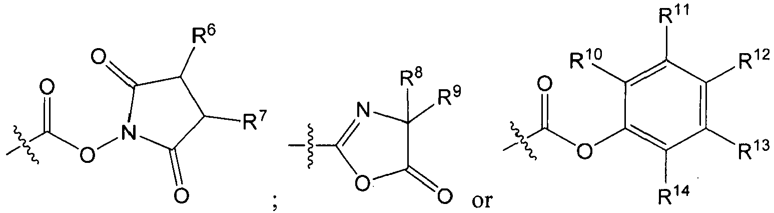 wherein:
R⁶, R⁷, R⁸ and R⁹ are each independently H or alkyl; and
R¹⁰, R¹¹, R¹², R¹³ and R¹⁴ are each independently, H, an electron withdrawing group, -NCS, -NCO, -CO₂H, -SO₃H, -L'-poly or salts thereof, wherein L' is an optional linker up to 100 atoms in length and poly is a water soluble polymer.

In some embodiments, each of R⁶, R⁷, R⁸ and R⁹ are H. In other embodiments, at least one of R¹⁰, R¹¹, R¹² , R¹³ or R¹⁴ is an electron withdrawing group. In other embodiments, each of R¹⁰, R¹¹, R¹² , R¹³ or R¹⁴ is an electron withdrawing group. For example, in some embodiments the electron withdrawing group is halogen, nitro or nitrile, and in other more specific embodiments the electron withdrawing group is fluoro.

In some of the foregoing embodiments, at least one of the second reactive groups has the following structure:

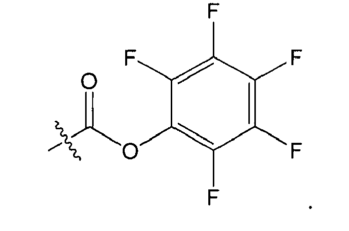

In other embodiments, each of the second reactive groups has the above structure.

In other of the foregoing embodiments, the C subunit has, at each occurrence, independently one of the following structures:

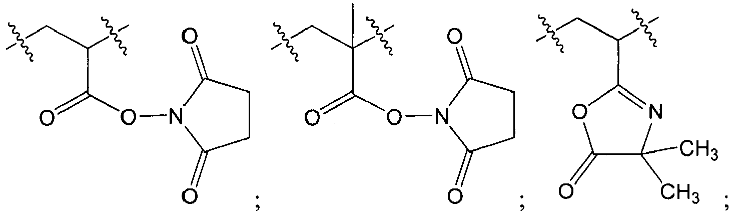

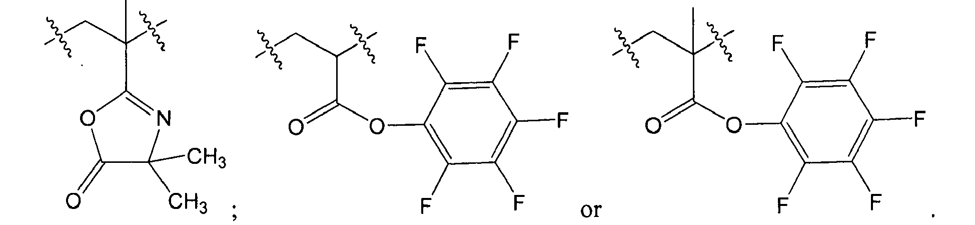

In some more specific aspects, the C subunit has, at each occurrence, independently one of the following structures:

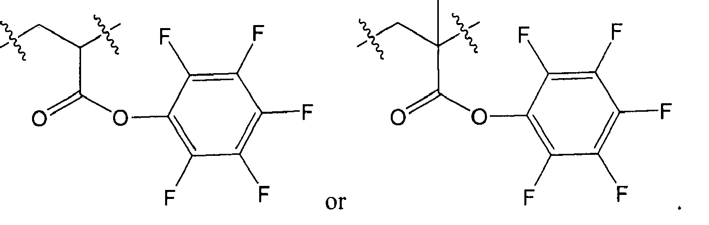

In some other embodiments, the C subunit has, at each occurrence, independently click reactivity. For example, the capture probe may include a click functional group and the capture probe is conjugated to the polymer using click chemistry. In some embodiments, R⁴ is an alkyne, alkylsilyl-protected alkyne, azide, nitrile, thiol, alkene, maleimide, epoxide, aziridine or thiirane functional group. In certain embodiments, R⁴ is an alkyne or azide functional group.

In further of the foregoing embodiments, the C subunit has, at each occurrence, independently one of the following structures: 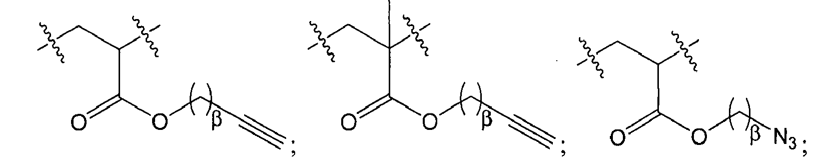 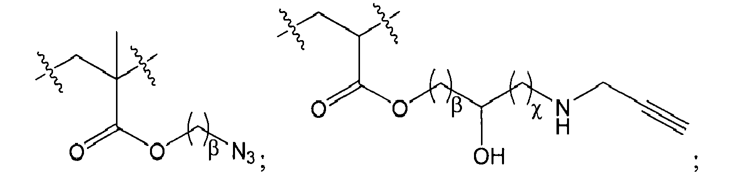 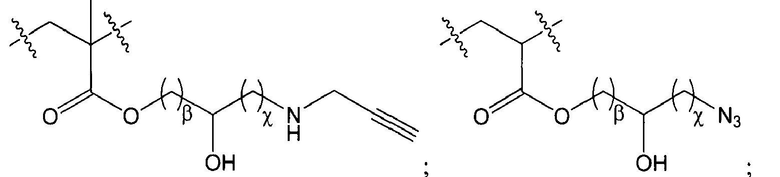 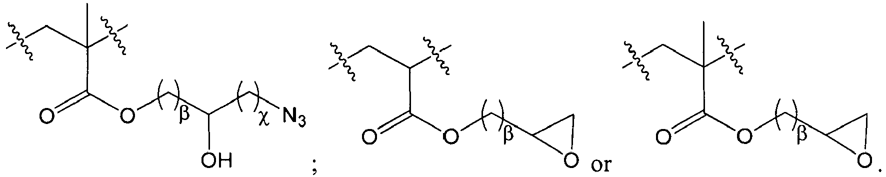 wherein β and χ are each independently integers ranging from 1 to 5.

In other embodiments, β is 1 or 3, and in other aspects χ is 1.

In other more specific embodiments of the foregoing, the C subunit has, at each occurrence, independently one of the following structures:

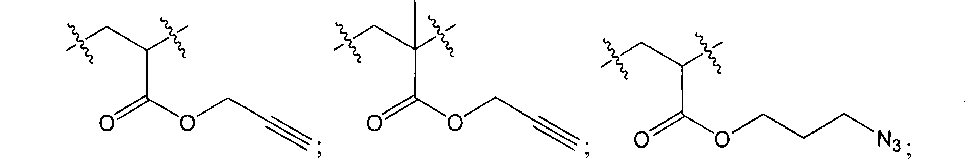

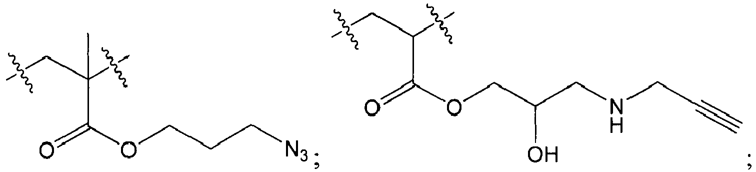

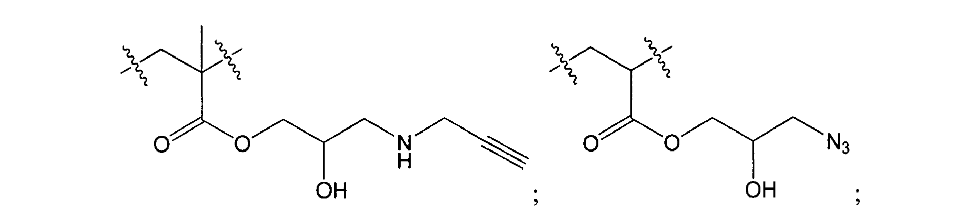

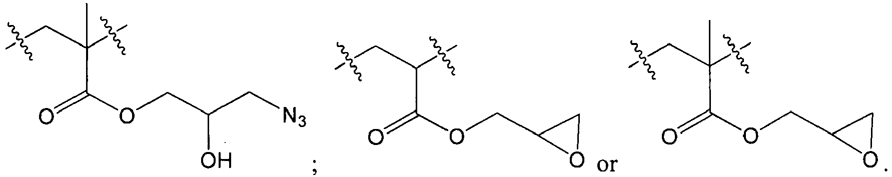

In other embodiments, at least one C subunit is at a terminal position covalently bound to T². For example, in some embodiments T² is H.

In other aspects, at least one of the C subunits has the following structure: 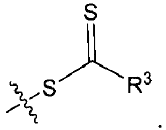 wherein:
R³ is aryl. For example, such structures may be useful for preparation of polymers comprising alkyne moieties. For example, at least one of the C subunits may have the following structure in certain embodiments:

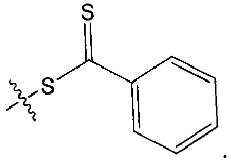

In some embodiments, poly is polyethylene glycol, polyacrylamide, poly(dimethylacrylamide), poly(acrylic acid), polyvinyl alcohol, polyvinyl pyrrolidone, poly(methyl vinyl ether), poly(ethyl vinyl ether), poly(*N*-vinylformamide), poyl(*N*-vinyl acetamide) or poly(*N*-methyl-*N*-vinylacetamide).

In other specific embodiments of the foregoing polymer, at least one of the C subunits is at a terminal position covalently bound to T². For example, in certain embodiments T² is H.

In some of the foregoing embodiments, the B subunit has, at each occurrence, independently the following structure (V): 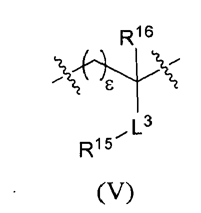 wherein:
R¹⁵ is the hydrophilic functional group;
R¹⁶ is hydrogen or alkyl;
L³ is an optional linker up to 100 atoms in length; and
ε is an integer ranging from 0 to 10.

In certain embodiments, L³ comprises alkylene, ester, alkylene oxide, amide, imide, ether or dithio moieties, or combinations thereof. In other embodiments, L³ is absent.

In some embodiments, ε is 1, and in other embodiments R¹⁶ is H.

In other of the foregoing embodiments, R¹⁵ has, at each occurrence, independently one of the following structures: 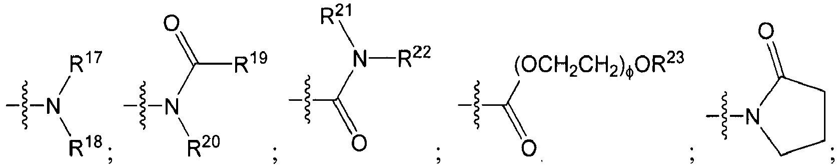 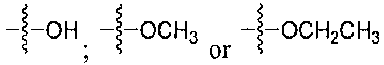 wherein:
R¹⁷, R¹⁸, R¹⁹, R²⁰, R²¹, R²² and R²³ are each independently H, alkyl or hydroxyl alkyl; and
φ is an integer ranging from 1 to 200.

In some embodiments of the foregoing, R¹⁷, R¹⁸, R¹⁹, R²⁰, R²¹, R²²and R²³ are each independently H or methyl. In other embodiments, R¹⁵ has, at each occurrence, independently the following structure:

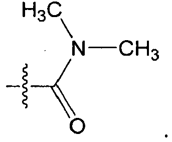

For example, in some embodiments the B subunit has, at each occurrence, independently one of the following structures:

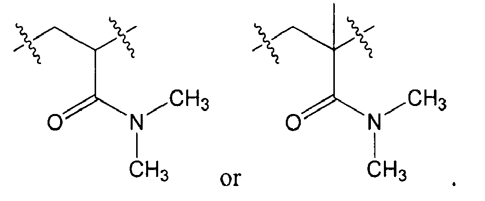

In any of the foregoing embodiments, the polymer has one of the following structures: 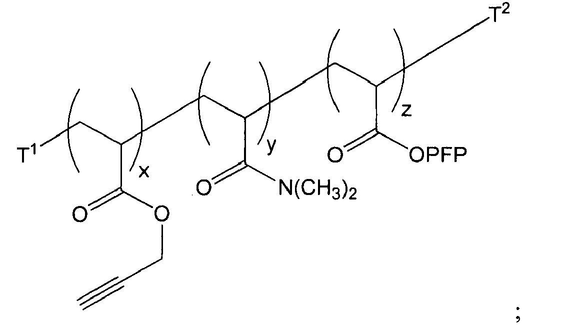 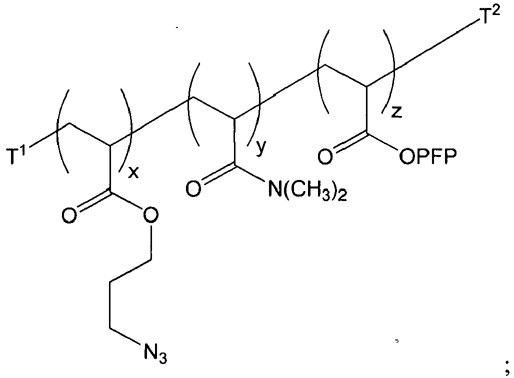 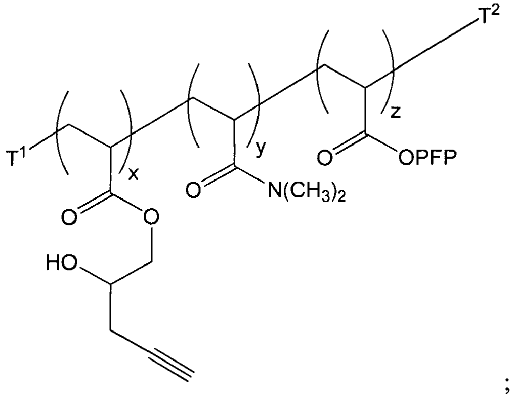 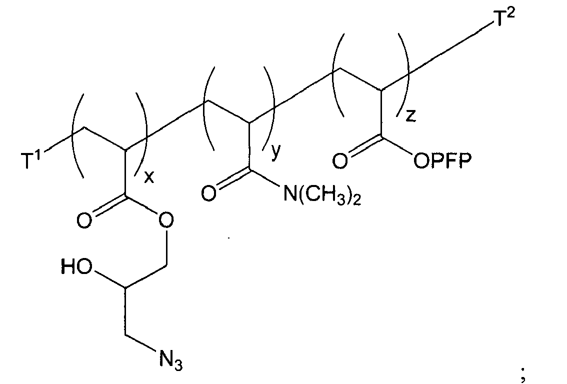 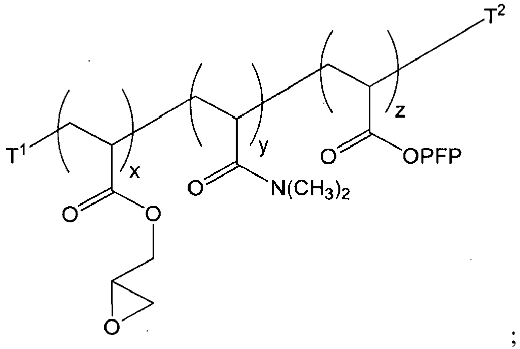 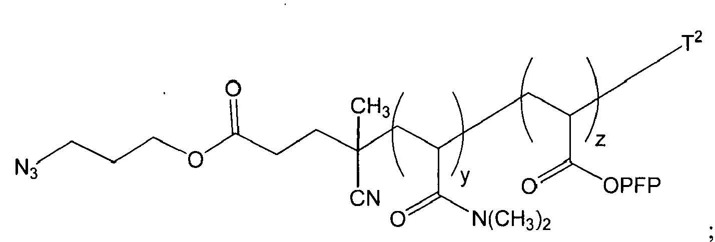 or 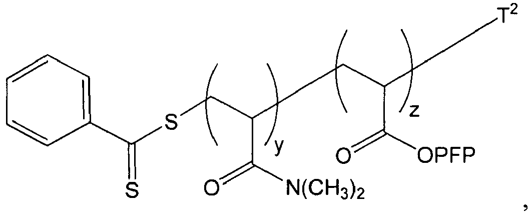 wherein:
the A, B and C subunits are present at least once in the polymer;
OPFP represents pentafluorophenoxy;
T¹ and T² are each independently absent or polymer terminal groups selected from H and alkyl; and
x, y and z are each independently an integer from 1 to 350,000.

In some of the foregoing embodiments, x, y and z are each independently an integer from 1 to 50,000.

In other exemplary embodiments, the polymer has one of the following structures: 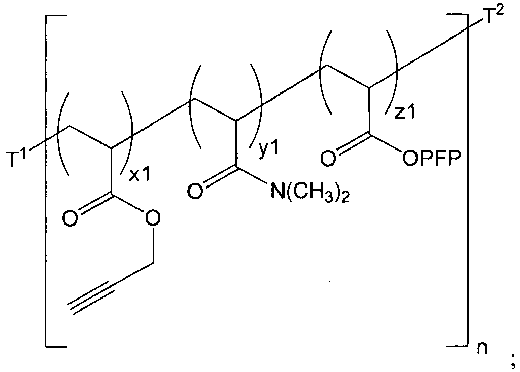 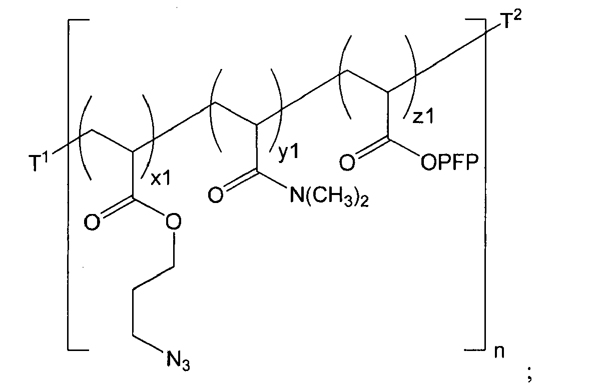 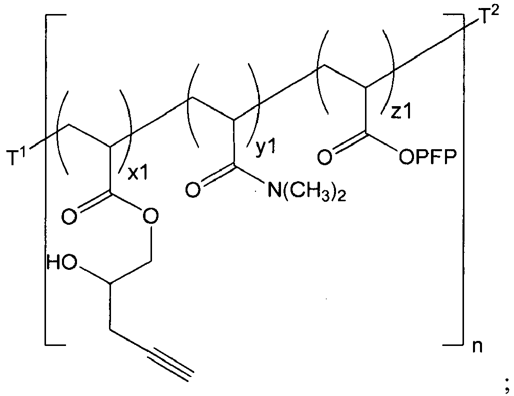 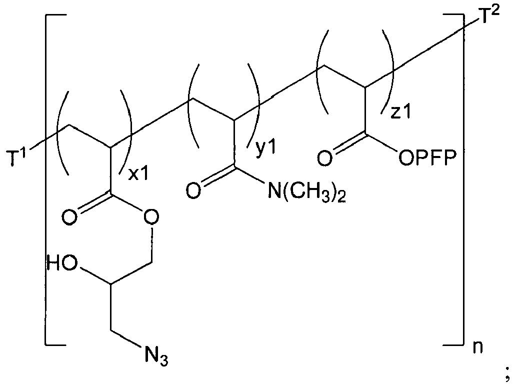 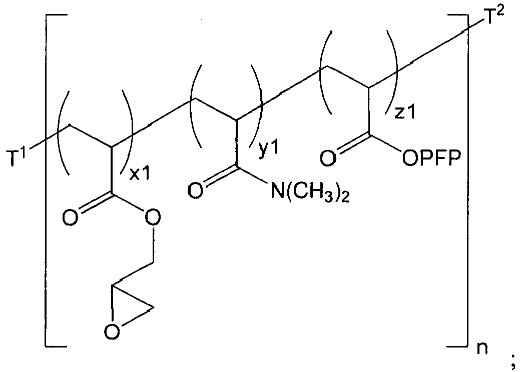 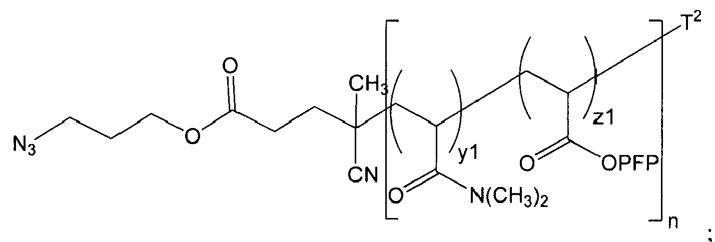 or 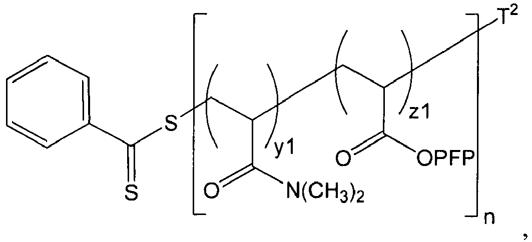 wherein:
the A, B and C subunits are present at least once in the polymer;
OPFP represents pentafluorophenoxy;
T¹ and T² are each independently absent or polymer terminal groups selected from H and alkyl;
x1, y1 and z1 are, at each occurrence, independently 0 or 1; and
n is an integer from 1 to 700,000.

In other embodiments of the foregoing, n is an integer from 1 to 150,000

As noted above, the hydrophilicity of the polymer, and thus the water contact angle of the resulting solid support surface, can be controlled at least in part by proper selection of the B subunit and the number of subunits incorporated into the polymer. Accordingly, in some embodiments at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95% or at least 99% of the subunits in the polymer are B subunits.

In other embodiments, the mole fraction of the A, B and C subunits can be varied. For example the mole fraction of each of the A, B and C subunits can vary from about 0.1 mole % to about 99.8 mole %. In some exemplary embodiments, the total mole percent of the sum of the A, B and C subunits is 100%.

In certain embodiments, the mole percent of the A subunit ranges from about 1% to about 30%, for example from about 15% to about 25%. In other embodiments, the mole percent of B subunits ranges from about 20% to about 60%, for example from about 35% to about 45%. In other embodiments, the mole percent of C subunits ranges from about 20% to about 60%, for example from about 35% to about 45%. In other further embodiments, the mole percent of the A subunit ranges from about 15% to about 25%, the mole percent of the B subunit ranges from about 35% to about 45% and the mole percent of the C subunit ranges from about 35% to about 45%. In still other embodiments, the mole percent of the A subunit is about 20%, the mole percent of the B subunit is about 40% and the mole percent of the C subunit is about 40%.

In certain embodiments, the polymer comprises only one A subunit. For example, in some embodiments the A subunit is at a terminal end of the polymer.

In various embodiments of any of the foregoing the capture probe is a polynucleotide, an oligonucleotide, a peptide, a polypeptide or a carbohydrate. For example, in some embodiments the capture probe is a polynucleotide. In other embodiments, the capture probe is DNA.

In certain embodiments, the invention also provides a compound useful for activating the surface of a solid substrate. The activated substrate can in turn be used in any number of applications, including immobilizing (e.g., via covalent attachment) the foregoing polymers to prepare solid supports. In some embodiments, the compounds comprise a photolizable azide moiety and either an alkyl azide or alkyne moiety, wherein the alkyne or alkyl azide are linked to the photolysable azide via a linker moiety (e.g., polymer). The photolysable azide may be any azide moiety which is capable of nitrene insertion into a C-H bond on the surface of the solid substrate upon irradiation with a suitable light source. Such methods are well-known in the art. In certain embodiments, the photolysable azide is an aryl azide.

In some embodiments, the compound for activating the surface of a solid substrate has the following structure (IX): 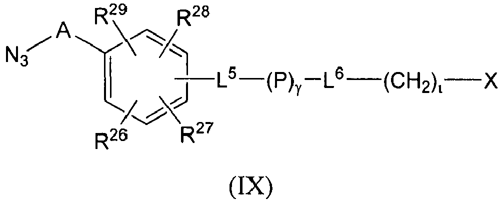 wherein:
X is an azide or alkyne moiety;
L⁵ and L⁶ are each independently optional linkers comprising alkylene, alkylene oxide, imide, ether, ester or amide moieties, or combinations thereof;
R²⁶, R²⁷, R²⁸ and R²⁹ are each independently, H, alkyl, halo, nitrile, nitro or ammonium;
P is -(OCH₂CH₂)- or -(CH₂)-;
A is a direct bond or -S(O)₂-;
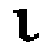 is an integer ranging from 0 to 10; and
γ is an integer ranging from 1 to 2000.

In some embodiments, each of R²⁶, R²⁷, R²⁸ and R²⁹ are H. In other embodiments, A is a direct bond.

In some embodiments, P is -(OCH₂CH₂)-. In other embodiments, P is -(CH₂)-. By careful selection of the P moiety, and the number of repeating subunits therein (i.e., γ), the present Applicants have discovered that the water contact angle of the resulting surface can be optimized for the interfacial reaction required for immobilization of the above polymers.

Accordingly, in some embodiments the compound has one of the following structures: 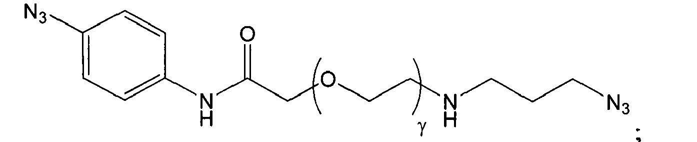 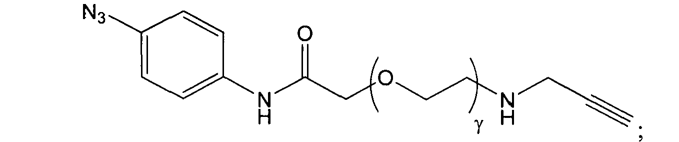 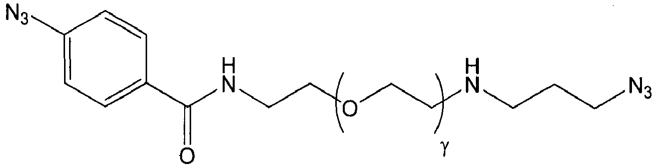 or

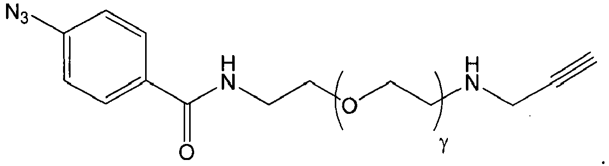

In some specific embodiments of the foregoing compounds, γ ranges from 1 to 100, for example from 55 to 90.

It is understood that any embodiments of the compounds and/or polymers, as set forth herein, and any specific substituent set forth herein in the compounds and/or polymers described herein, may be independently combined with other embodiments and/or substituents of the compounds and/or polymers described herein to form embodiments of the inventions not specifically set forth above. In addition, in the event that a list of substituents is listed for any particular R group in a particular embodiment and/or claim, it is understood that each individual substituent may be deleted from the particular embodiment and/or claim and that the remaining list of substituents will be considered to be within the scope of the invention.

It is understood that in the present description, combinations of substituents and/or variables of the depicted formulae are permissible only if such contributions result in stable compounds.

Methods for preparation of the disclosed compounds and polymers will be readily apparent to one of ordinary skill in the art. For example, in certain embodiments polymers of the present invention may be prepared by admixing the desired ratio of subunits and an optional activator (e.g., AIBN for thermal polymerization or a catalyst for ATRP). Subunits and polymers comprising click functional groups, such as azide or alkynes can be prepared according to methods known in the art or purchased from commercial sources (e.g., propargyl acrylate or 3-azidopropylacrylate). See e.g., S.R. Gondi, el at., Macromolecules 2007, 40, 474-481; P.J. Roth, el at., J. Polym. Sci. Part A: Polym. Chem. 2009, 47, 3118-3130; and C. Li, et al., Macromolecules, 2009, 42, 2916-2924, the disclosures of which is hereby incorporated by reference in their entirety. Exemplary methods are provided in the examples.

It will also be appreciated by those skilled in the art that in the processes described herein the functional groups of intermediate compounds may need to be protected by suitable protecting groups. Such functional groups include hydroxy, amino, mercapto and carboxylic acid. Suitable protecting groups for hydroxy include trialkylsilyl or diarylalkylsilyl (for example, *t*-butyldimethylsilyl, *t*-butyldiphenylsilyl or trimethylsilyl), tetrahydropyranyl, benzyl, and the like. Suitable protecting groups for amino, amidino and guanidino include *t*-butoxycarbonyl, benzyloxycarbonyl, and the like. Suitable protecting groups for mercapto include -C(O)-R" (where R" is alkyl, aryl or arylalkyl), *p*-methoxybenzyl, trityl and the like. Suitable protecting groups for carboxylic acid include alkyl, aryl or arylalkyl esters. Protecting groups may be added or removed in accordance with standard techniques, which are known to one skilled in the art and as described herein. The use of protecting groups is described in detail in Green, T.W. and P.G.M. Wutz, Protective Groups in Organic Synthesis (1999), 3rd Ed., Wiley. As one of skill in the art would appreciate, the protecting group may also be a polymer resin such as a Wang resin, Rink resin or a 2-chlorotrityl-chloride resin.

Furthermore, all compounds and/or polymers of the invention which exist in free base or acid form can be converted to salts by treatment with the appropriate inorganic or organic base or acid by methods known to one skilled in the art. Salts of the compounds of the invention can be converted to their free base or acid form by standard techniques.

### B. Solid Supports

As noted above, certain aspects of the present invention also include solid supports. The solid supports may comprise polymers immobilized thereto. In some embodiments, the polymers comprise functional groups for immobilization of further polymers on the solid support. In other embodiments, the immobilized polymers comprise functional groups for covalent conjugation with a capture probe or the immobilized polymers comprise a capture probe conjugated thereto. The solids supports can be used in a number of analytical assays, in particular embodiments such assays include assays in array format, such as DNA micro array assays.

Accordingly, in some embodiments the present disclosure provides a solid support comprising a polymer immobilized to an outer surface of a solid substrate, wherein the polymer comprises B, D and E subunits, wherein:
the B subunit comprises, at each occurrence, independently a hydrophilic functional group;
the D subunit comprises, at each occurrence, independently a reactive group having a reactivity specific for covalent conjugation to an capture probe or the D subunit comprises a covalent bond to a capture probe; and
the E subunit comprises, at each occurrence, independently a reaction product of two complementary click functional groups, wherein the reaction product comprises a covalent bond to either the outer surface of the solid substrate or an optional linker (L⁴) disposed between the E subunit and the outer surface of the solid substrate.

In some embodiments, the polymer is a random co-polymer, for example a random terpolymer.

In some embodiments of the foregoing solid support, the polymer is a random co-polymer, such as a random terpolymer. In some embodiments of the foregoing solid support, the polymer has the following structure (VI):

T³-(B)_{q}(D)ᵣ(E)ₛ-T⁴ (VI)

wherein:
T³ and T⁴ are each independently absent or polymer terminal groups selected from H, alkyl and an initiator residue; and
q, r and s are each independently an integer from 1 to 350,000.

In some other embodiments, q, r and s are each independently an integer from 1 to 50,000.

The depicted connectivity of the B, D and E subunits of structure (VI) is in no way limiting, and in certain embodiments, the actual structure of the polymer of structure (VI) is a random co-polymer wherein each of the B, D, and E subunits occur at any position in the polymer.

In some other embodiments of the foregoing solid support, the polymer has the following structure (VIa):

T³-[(B)_{q1}(D)ᵣ₁(E)ₛ₁]ₘ-T⁴ (VIa)

wherein:
B, D and E are present at least once in the polymer of structure (VIa);
T³ and T⁴ are each independently absent or polymer terminal groups selected from H, alkyl and an initiator residue;
q1, r1 and s1 are, at each occurrence, independently 0 or 1; and
m is an integer from 1 to 700,000.

In other embodiments, m is an integer from 1 to 150,000.

In other examples, the click functional group can be formed by reaction of an alkyne, amine, alkylsilyl-protected alkyne, azide, nitrile, thiol, alkene, maleimide, epoxide, aziridine or thiirane functional group with a complementary click reactive group.

In other embodiments of the foregoing solid support, the E subunit is at a terminal position in the polymer.

In other embodiments, the E subunit has, at each occurrence, independently the following structure (VI): 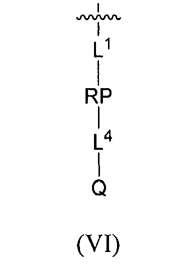 wherein:
L¹ is an optional linker up to 100 atoms in length;
RP is the reaction product;
L⁴ is an optional linker; and
Q represents the outer surface of the solid substrate.

In some embodiments, L⁴ is up to 100 atoms in length.

In other embodiments, each of the E subunits has the above structure (VI).

In other embodiments of the foregoing solid support, the E subunit has, at each occurrence, independently the following structure (VII): 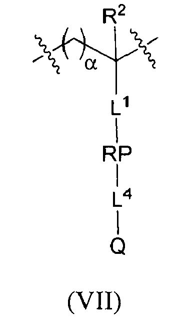 wherein:
L¹ is an optional linker up to 100 atoms in length;
RP is the reaction product;
L⁴ is an optional linker;
R² is H or alky; and
Q represents the outer surface of the solid substrate.

In other embodiments, each of the E subunits has the above structure (VII).

In still other embodiments of the foregoing solid support, wherein L¹ comprises alkylene, ester, ether or dithio moieties, or combinations thereof. In other embodiments L¹ is absent.

In some embodiments, α is 1. In other embodiments, R² is H.

In still other embodiments of the foregoing solid support, L⁴ comprises a silicon-oxygen bond, an alkylene chain, a polymer or combinations thereof. For example, in some embodiments the polymer is polyethylene glycol. In some aspects, the polyethylene glycol comprises from 1 to 50,000 (*e.g.,* 10 to 50,000) monomer subunits. In other embodiments the polyethylene glycol comprises from 1 to 90 monomer subunits. For example, in other embodiments the polyethylene glycol comprises from 55 to 90 monomer subunits.

In some embodiments of the foregoing solid support, L⁴ has one of the following structures: 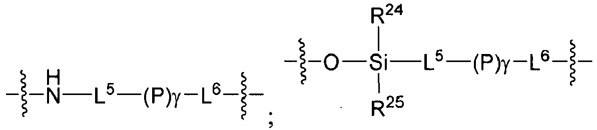 or 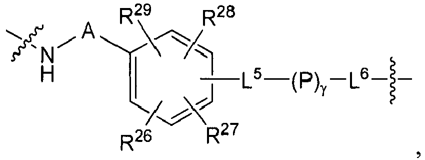 wherein:
L⁵ and L⁶ are each independently optional linkers comprising alkylene, alkylene oxide, imide, ether, ester or amide moieties, or combinations thereof;
R²⁴ and R²⁵ are each independently H, hydroxyl, alkyl, alkoxy or -OQ, wherein Q is the outer surface of the solid substrate;
R²⁶, R²⁷, R²⁸ and R²⁹ are each independently, H, alkyl, halo, nitrile, nitro or ammonium;
P represents a polymer subunit;
A is a direct bond or -S(O)₂-; and γ is an integer ranging from 1 to 2000,
where L⁴ is bound to the solid substrate via the terminal nitrogen or oxygen atom.

In certain embodiments of the foregoing, P is -CH₂- or -OCH₂CH₂-.

In other embodiments, L⁴ has one of the following structures: 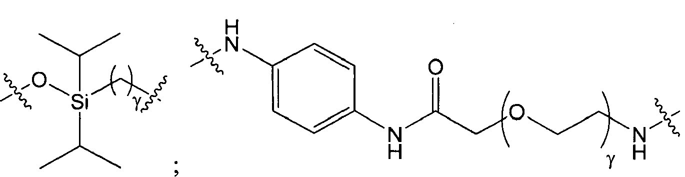 or

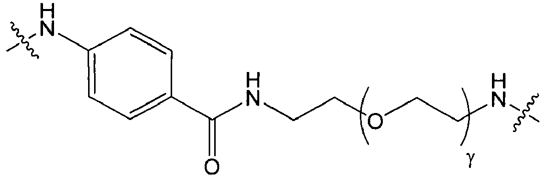

In certain embodiments, γ ranges from 1 to 90, for example from 55 to 90. In other embodiments, L⁴ is absent.

In some embodiments of the foregoing solid support, the click functional group is a triazole. For example, in some embodiments the E subunits has, at each occurrence, independently one of the following structures: 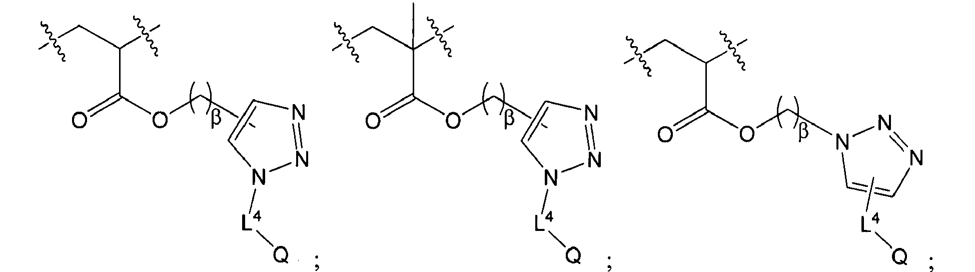 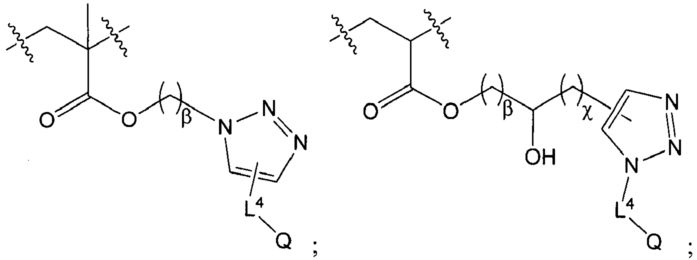 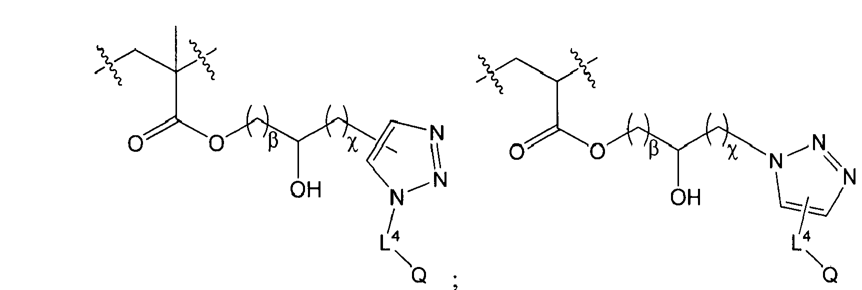 or 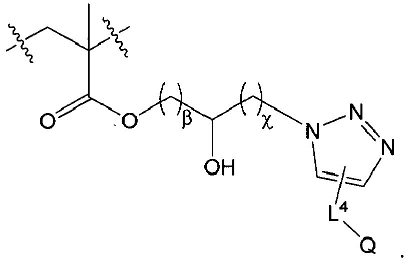 wherein:
β and χ are each independently integers ranging from 1 to 5;
L⁴ is an optional linker; and
Q represents the solid substrate.

In other embodiments, each of the E subunits has one of the above structures.

In other embodiments, at least one E subunit is at a terminal position covalently bound to T⁴. For example, in some embodiments T⁴ is H.

In certain embodiments, L⁴ comprises one or more polyethylene glycol repeating units.

In other embodiments of the foregoing solid support, the B subunit is as defined for the B subunit in any of the embodiments of the above described polymer. In some other embodiments of the foregoing solid support, the D subunit is as defined for the C subunit in any of the embodiments of the above described polymer.

In certain embodiments, at least one D subunit comprises a covalent bond to a capture probe. For example, in some examples at least one D subunit has the following structure (VIII): 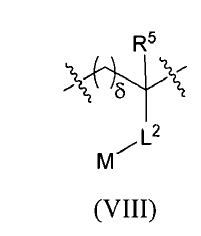 wherein:
M is the capture probe;
R⁵ is hydrogen or alkyl;
L² is an optional linker up to 100 atoms in length; and
δ is an integer ranging from 0 to 10.

In other embodiments, each of the D subunits has the above structure (VIII).

In some embodiments, L² comprises alkylene, ester, carbonyl, alkylene oxide, amide, imide ether or dithio moieties, or combinations thereof. In other embodiments, δ is 1. In still other embodiments, R⁵ is H.

In other embodiments of the foregoing solid support, at least one D subunit has one of the following structures:

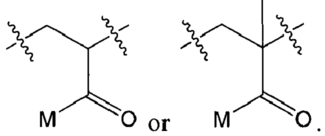

In other embodiments, each of the D subunits has one of the above structures.

In still other embodiments of the foregoing solid support, a surface of the solid support has one of the following structures: 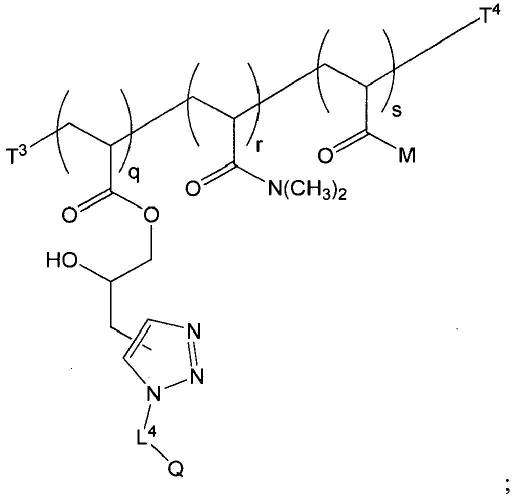 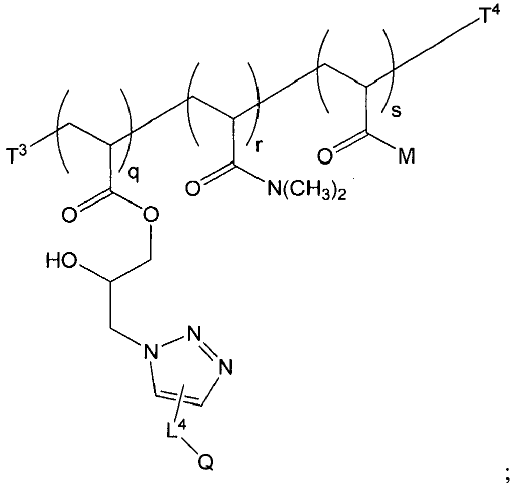 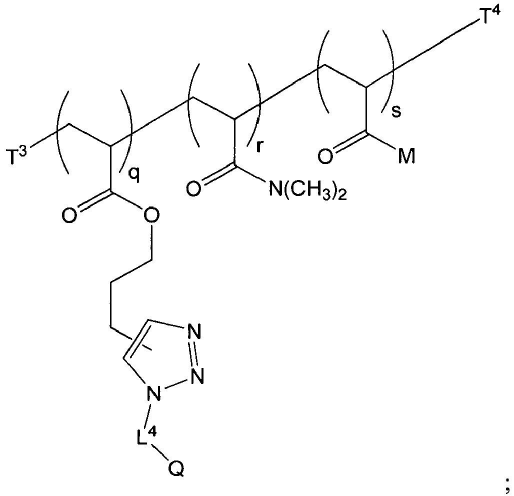 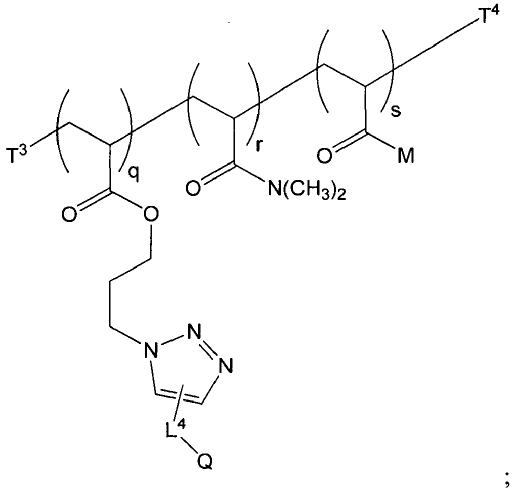 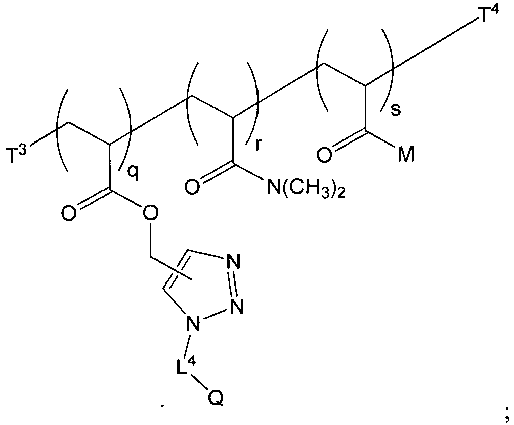 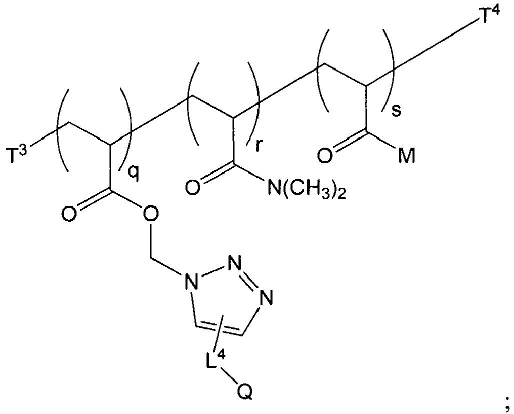 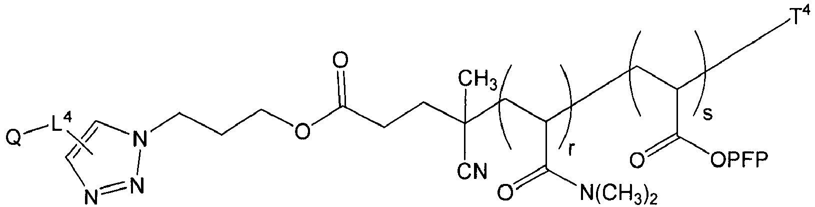 or 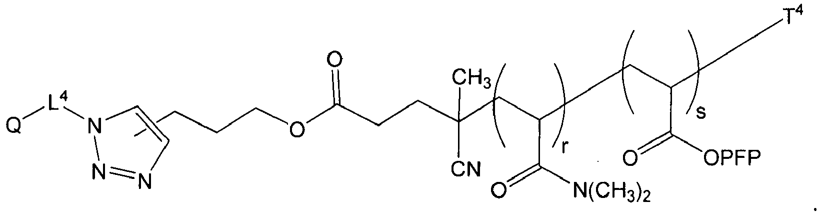 wherein:
the B, D and E subunits are present at least once in the polymer;
T³ and T⁴ are each independently absent or polymer terminal groups selected from H, alkyl and an initiator residue;
q, r and s are each independently an integer from 1 to 350,000;
L⁴ is an optional linker;
M, at each occurrence, independently represents a capture probe; and
Q represents the outer surface of the solid substrate.

In some other embodiments of the foregoing solid support, a surface of the solid support has one of the following structures: 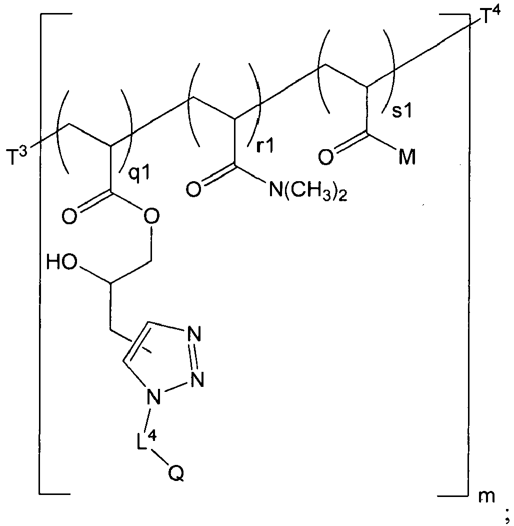 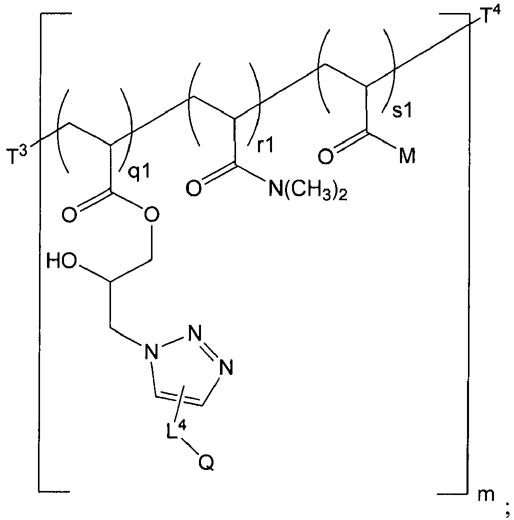 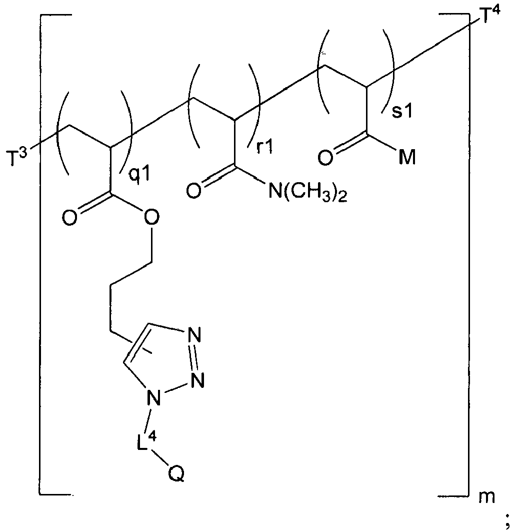 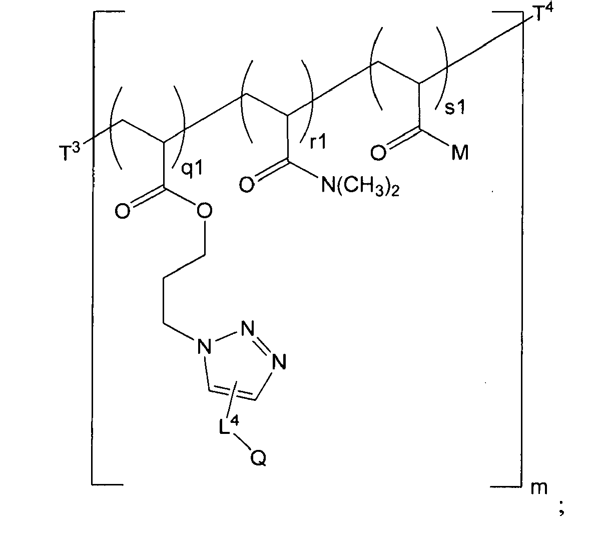 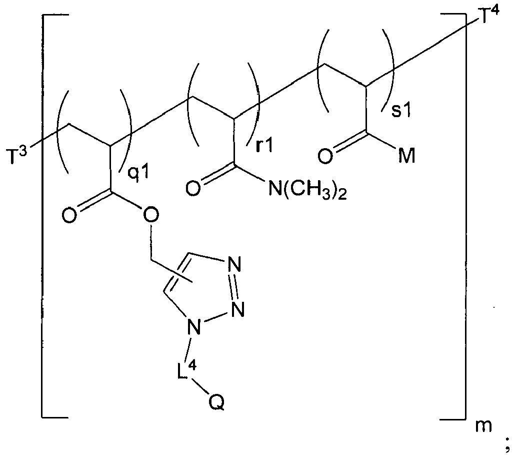 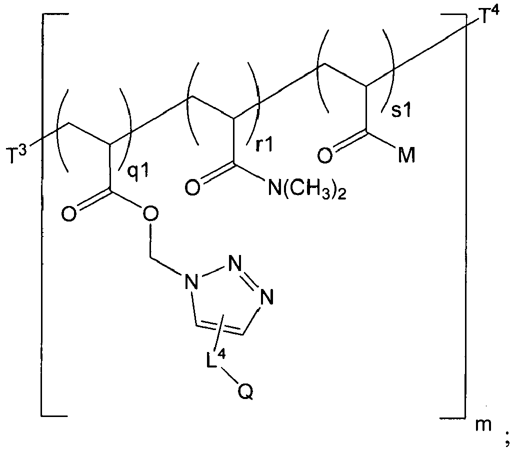 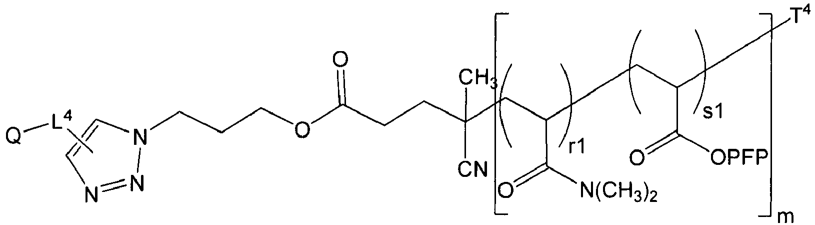 or 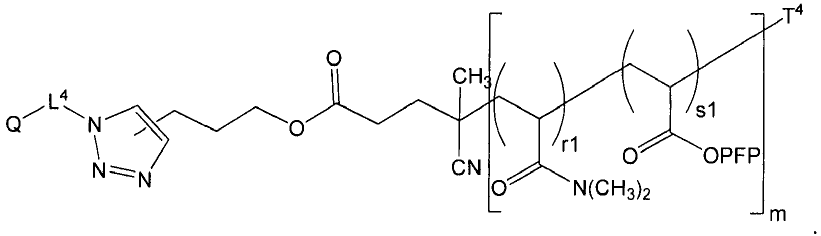 wherein:
the B, D and E subunits are present at least once in the polymer;
T³ and T⁴ are each independently absent or polymer terminal groups selected from H, alkyl and an initiator residue;
q1, r1 and s1 are, at each occurrence, independently 0 or 1;
L⁴ is an optional linker;
M, at each occurrence, independently represents a capture probe
Q represents the outer surface of the solid substrate; and
m is an integer from 1 to 150,000.

In certain embodiments of the foregoing, L⁴ is as defined in any of the above embodiments.

In other embodiments of the foregoing solid support, the capture probe is covalently bound to the D subunit via a nitrogen atom. In some aspects the capture probe is a peptide, protein, carbohydrate, polynucleotide, oligonucleotide or polypeptide. For example, in some embodiments the capture probe is a polynucleotide, such as DNA.

The water contact angle of the solid support is controlled (e.g., by controlling the number and type of B subunits) to enable interfacial reactions with an analyte molecule dissolved in a solvent, for example an aqueous solvent. In this regard, the water contact angle is generally tailored to enhance contact of the dissolved analyte and the reactive surface (i.e., the surface having the polymer immobilized thereto) of the solid support. In some embodiments, the water contact angle of the solid support ranges from about 50° to 90°, for example about 50° to 70°. In some embodiments the water contact angle ranges from about 55° to 65°, in other embodiments the water contact angle ranges from about 60° to 65°, and even other embodiments in other embodiments the water contact angle ranges from about 80° to 90° Methods for determination of the water contact angle are well known in the art.

Accordingly, in some embodiments at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95% or at least 99% of the subunits in the polymer are B subunits.

In other embodiments, the mole fraction of the B, D and E subunits can be varied. For example the mole fraction of each of the B, D and E subunits can vary from about 0.1 mole % to about 99.8 mole %. In some exemplary embodiments, the total mole percent of the sum of the B, D and E subunits is 100%.

In certain embodiments, the mole percent of the E subunit ranges from about 1% to about 30%, for example from about 15% to about 25%. In other embodiments, the mole percent of B subunits ranges from about 20% to about 60%, for example from about 35% to about 45%. In other embodiments, the mole percent of D subunits ranges from about 20% to about 60%, for example from about 35% to about 45%. In other further embodiments, the mole percent of the E subunit ranges from about 15% to about 25%, the mole percent of the B subunit ranges from about 35% to about 45% and the mole percent of the D subunit ranges from about 35% to about 45%. In still other embodiments, the mole percent of the E subunit is about 20%, the mole percent of the B subunit is about 40% and the mole percent of the D subunit is about 40%.

In certain embodiments, the polymer comprises only one E subunit. For example, in some embodiments the E subunit is at a terminal end of the polymer.

The type of solid substrate employed in practice of the invention is not limited. Generally the solid substrate will be of a type amenable to immobilization of the disclosed polymers and/or amenable to activation so that the polymers may be immobilized thereto. Solid substrates within the scope of the invention include, but are not limited to, optically transparent and opaque polymers. Solid substrates in the form of planar substrates, beads, particles, porous matrices and porous monoliths are also included in certain embodiments.

In some embodiments, the solid substrate comprises an organic polymer. For example, in some embodiments the solid support comprises poly(styrene), poly(carbonate), poly(ethersulfone), poly(ketone), poly(aliphatic ether), poly(aryl ether), poly(amide) poly(imide), poly(ester) poly(acrylate), poly(methacrylate), poly(olefin), poly(cyclic olefin), poly(vinyl alcohol) or copolymers, halogenated derivatives or crosslinked derivatives thereof. In certain embodiments, the halogenated derivatives are halogenated poly(aryl ether), halogenated poly(olefin) or halogenated poly(cyclic olefin). In some specific embodiments, the solid substrate comprises a poly(cyclic olefin).

In still other embodiments, the solid substrate comprises an oxide. For example, in some embodiments the solid substrate comprises silicon, fused silica, glass, quartz, indium-tin oxide, titanium dioxide, aluminum oxide or combinations thereof.

Solid substrate comprising organic polymers having an outer surface comprising an oxide layer are also included within the scope of the present invention.

Certain embodiments of the invention include use of the solid supports in analytical assays. Such assays often include an optical analysis step, such as fluorescence assay. Accordingly, in some embodiments the solid substrate is substantially optically transparent. In other embodiments, the solid substrate is substantially optically transparent between about 400 nm and about 800 nm. In still other embodiments, the solid substrate is at least about 90% optically transparent.

Other certain embodiments of the invention are directed to use of the solid supports in analytical array-type assays. Accordingly, some embodiments provide a solid support wherein the solid support comprises a systematic array of distinct locations, each distinct location independently comprising at least one of the polymers conjugated thereto. For example, in some embodiments each distinct location independently comprises a plurality of the polymers conjugated thereto. In more specific embodiments, at least one polymer at each distinct location comprises a capture probe covalently bound thereto via a D subunit, and in other embodiments each distinct location comprises at least one capture probe bound thereto, wherein the capture probe is structurally distinct from at least one capture probe bound at each of the other distinct locations. In further embodiments, each distinct location comprises a plurality of structurally distinct analyte molecules bound thereto.

In various embodiments of the above solid support, the capture probe is a peptide, protein, carbohydrate, polynucleotide, oligonucleotide, oligopeptide or polypeptide. In specific embodiments, the capture probe is a polynucleotide, such as DNA. In various embodiments, the polynucleotide or DNA comprises a sequence complementary to a sequence of an analyte polynucleotide or DNA molecule.

### C. Activated Solid Substrates

In another aspect, the present invention is directed to a solid substrate having an activated outer surface. The solid substrates may be used in any number of solid-supported applications. In some embodiments, the solid substrates comprise an azide or alkyne moiety covalently bound to an outer surface of the solid substrate. Such solid substrates may be used, for example, in methods for preparing solid supports comprising polymers and/or capture probes immobilized thereto. In specific embodiments, the solid substrates may be used for covalently attaching the disclosed polymers to the solid support via a click reaction.

Accordingly, in certain embodiments the present invention is directed to a solid substrate comprising an outer surface, wherein the solid substrate comprises an azide or alkyne moiety covalently bound to the outer surface.

In some embodiments, the solid substrate has one of the following structures: 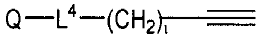 or Q-L⁴-(CH₂)-N₃ wherein:
Q represents the outer surface of the solid substrate;
L⁴ is an optional linker;
and 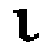 is an integer ranging from 0 to 10.

The present inventors have discovered that the L⁴ linker moiety can be selected such that the water contact angle of the solid substrate is optimized for interfacial reaction with a polymer in solvents having various polarities. Accordingly, in certain specific embodiments, L⁴ is present. The water contact angle of the solid substrate ranges from about 50° to 90°, for example from about 50° to 70°. In some embodiments the water contact angle is in the range of 55° to 65°. In certain embodiments the water contact angle ranges from 60° to 65°. In certain other embodiments the water contact angle ranges from 80° to 90°.

In some specific embodiments, L⁴ comprises a polyethylene glycol polymer, and in certain of these embodiments the polyethylene glycol polymer comprises from 1-90 ethylene glycol subunits, for example from 55-90 ethylene glycol subunits.

In some other embodiments, L⁴ is as defined above in any of the embodiments of the foregoing solid substrate comprising a polymer immobilized thereto. Further, the type of solid substrate used (e.g., polymer, oxide etc.) is not particularly limited. In certain embodiments, the composition of the solid support is as described in any of the embodiments of the foregoing solid support comprising a polymer immobilized on a solid substrate. In some other embodiments, 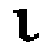 is 1, 2 or 3.

In some other embodiments, the present invention is directed to a solid substrate comprising an outer surface, wherein the solid substrate comprises an amino moiety covalently bound to the outer surface. In certain embodiments, the composition of a solid support is as described in any of the embodiments of the foregoing solid support comprising an ester- or azlactone-containing orthogonal polymer immobilized onto an aminated solid substrate. The orthogonal polymers comprise an azide or alkyne moiety for subsequent bioconjugation.

### D. Methods

Certain embodiments of the present invention are directed to methods. Such methods include, but are not limited to methods for preparation of the polymers, activated solid substrates and solid supports described herein. Methods for use of the solid supports in analytical assays are also provided. For example, the solid supports may be used in assays for the detection of any number of analytes, for example viruses, bacteria, plasmodium, fungi, as well as metals and unknown bio-warfare, bio-hazard and chemical warfare materials.

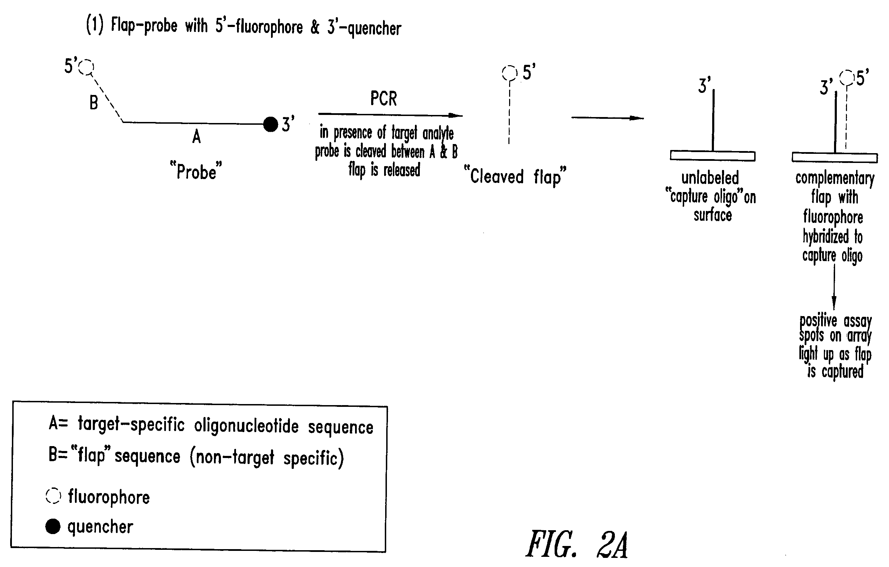

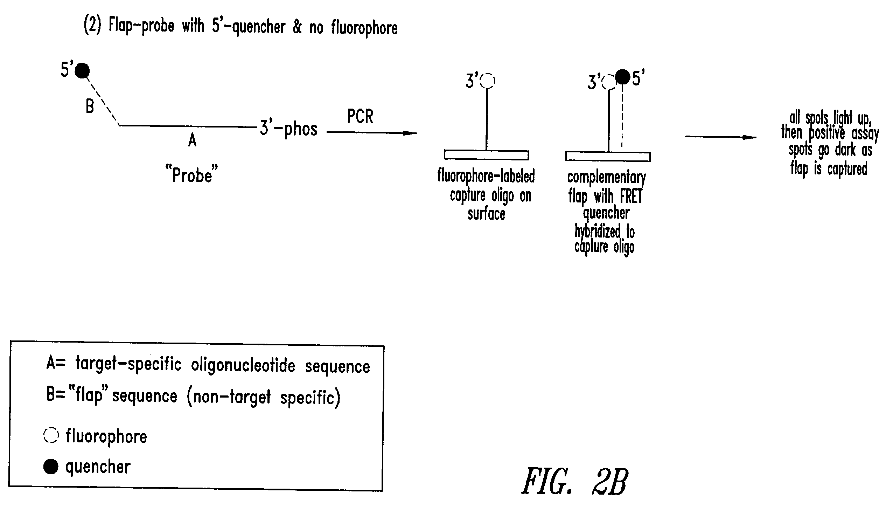

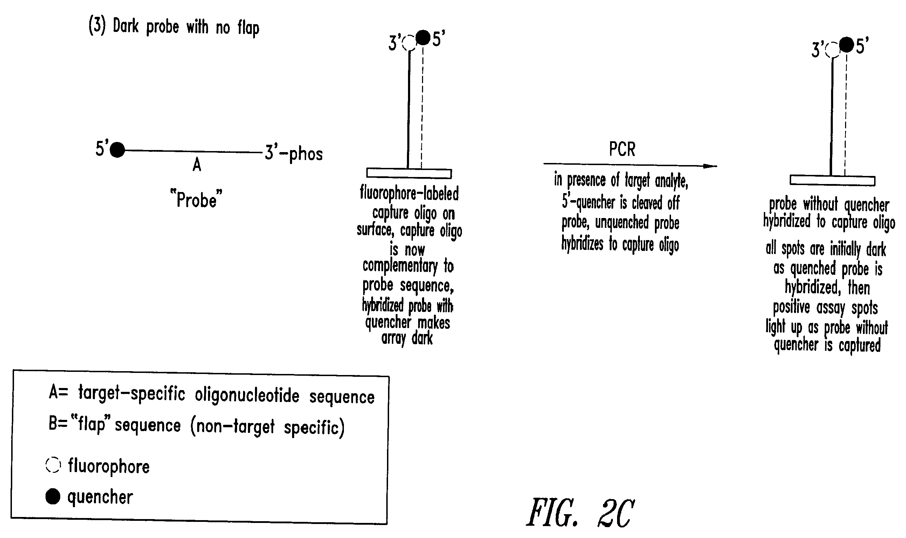

Methods for use of the solid supports for analysis of various analytes will be apparent to one of ordinary skill in the art. Such methods are described for example in Provisional U.S. Patent Application Nos. 61/463,580, 61/561,198, 1/684,104, 61/600,569, U.S. Patent Application No. 13/399,872 and U.S. Pub. No. 2012/0214686, the full disclosures of which are hereby incorporated herein by reference in their entirety for all purposes. Exemplary methods for use of the disclosed solid supports are depicted in schematically in Figure 2.

As depicted in Figure 2A, in one embodiment of the methods an analyte probe comprises sections A and B. The A section optionally comprises a quencher moiety, the quencher may be at the 3'end of the A section or at any other point within the A section. The A section is complementary to at least a portion of a target analyte sequence (e.g., pathogen DNA, etc.). The analyte probe also comprises section B (the "flap"). The flap comprises a fluorophore and a sequence complementary to at least a portion of a sequence of a capture probe bound to the solid support. Optionally, the sequence of the analyte probe is selected such that the A section and the flap have at least some complementarity so that the quencher and fluorophore are brought into close proximity, thus decreasing the fluorescent signal associated with the unbound analyte probe and increasing the overall sensitivity of the assay.

The assay conditions generally include a plurality of analyte probes having unique sequences specific for different target analytes. Under PCR conditions, and in the presence of a complementary (or at least partially complementary) target analyte, the flap is cleaved from the analyte probe. The cleaved flap is then hybridized to a solid support-bound capture probe complementary (or at least partially complementary) to the flap. The presence (or increase) of a fluorescent signal at the position to which the capture probe is bound indicates the presence of the target analyte sequence.

An alternate embodiment is depicted in Figure 2B. In this exemplary embodiment, the flap comprises a quencher and the support bound capture probe comprises a fluorophore. Again, the exact position of the quencher or fluorophore on the flap or capture probe, respectively, can be varied. Under PCR conditions in the presence of the target analyte sequence, the flap is cleaved from the probe. The flap is then hybridized to the capture probe and the fluorophore on the capture probe is thereby quenched. Accordingly, the absence (or decrease) of a fluorescent at the position which the capture probe is bound indicates the presence of the target analyte sequence.

Yet another exemplary method is provided in Figure 2C. Here, the probe comprises a sequence which is at least partially complementary to a target analyte sequence and does not comprise a cleavable flap. The probe in this embodiment comprises a quencher and the support-bound capture probe comprises a fluorophore. The probe is hybridized with the capture probe, resulting in a quenched signal at the position to which the capture probe is bound. The solid support is then subjected to PCR conditions. In the prescence of the target analyte sequence, the probe quencher is cleaved off and the fluorescent signal from the capture probe increases.

Accordingly, in one embodiment, the invention is generally directed to a method for determining the presence or absence of a target analyte molecule, the method comprising:
a) providing a solid support as described herein, wherein the D subunit, at each occurrence, independently comprises a capture probe covalently bound thereto;
b) contacting an analyte probe or fragment thereof with the solid support; and
c) detecting the presence or absence of a signal produced from interaction of the capture probe with the analyte probe.

In other related embodiments, the invention provides a method of detecting a target nucleic acid, the method comprising:
A) providing a detection chamber comprising at least one solid support described herein, the solid support comprising an array of capture probes;
B) loading a sample into the detection chamber, which sample comprises one or more copies of the target nucleic acid to be detected;
C) hybridizing an amplification primer and a probe to the one or more copies;
D) amplifying at least a portion of one or more of the target nucleic acid copies in an amplification primer dependent amplification reaction, wherein the amplification reaction results in cleavage of the probe and release of a first probe fragment;
E) hybridizing the first probe fragment to the high-efficiency array; and,
F) detecting a signal produced by binding the first probe fragment to the array, thereby detecting the target nucleic acid.

In certain embodiments, the detecting step(s) is carried out under conditions that reduce background signal proximal to the array.

In other embodiments, the methods comprising analyzing a sample for a plurality of target nucleic acid sequences, the method comprising:
A) contacting the sample with a first plurality of labeled probes, each of the first plurality of labeled probes comprising a first portion complementary to a different target sequence of interest in a first panel of target nucleic acid sequences and a second portion complementary to a different capture probe on a high efficiency probe array, the high efficiency probe array comprising a solid support as described herein, wherein the second portion has a label attached thereto and is not complementary to the target sequence of interest;
B) amplifying any target sequences from the first panel of target nucleic acid sequences that are present in the sample, in an amplification primer dependent amplification reaction, wherein the amplification reaction results in cleavage of labeled probes hybridized to the target sequences and release of the second portion of the labeled probes bearing the label;
C) hybridizing the released second portion of the labeled probes to the high-efficiency array;
D) detecting binding of the second portion of the labeled probe to a capture probe in the high efficiency array; and
E) identifying the target sequences present in the sample from the second portions of the labeled probes that hybridize to the high efficiency array.

In still other embodiments, the invention provides a method of detecting the presence of a target nucleic acid sequence in a sample, the method comprising:
A) performing an amplification reaction on the sample with a polymerase enzyme that possesses nuclease activity, in the presence of a first labeled probe that comprises a first portion complementary to a first target nucleic acid sequence and a second labeled portion not complementary to the first target nucleic acid sequence, such that the second portion is cleaved from the first portion when the target nucleic acid sequence is amplified;
B) hybridizing the second labeled portion to a capture probe complementary to the second portion; the capture probe being covalently bound to a solid support described herein; and
C) detecting the presence of the second labeled portion hybridized to the capture probe on the substrate.

Still other embodiments of the methods comprise a method of detecting a target nucleic acid sequence in a sample, the method comprising:
A) performing an amplification reaction on the sample with a polymerase enzyme that possesses nuclease activity, in the presence of a reagent comprising first probes that comprise a first portion complementary to the target nucleic acid sequence and a second portion not complementary to the first target nucleic acid sequence, the second portion comprising a first quencher moiety coupled to the second portion at a first position, such that the second portion is cleaved from the first portion as a first probe fragment, when the target nucleic acid sequence is amplified;
B) hybridizing the first probe fragment to capture probes immobilized upon a solid support described herein, wherein the capture probes comprise a fluorophore that is at least partially quenched by the first quencher moiety, the fluorophore coupled to a second position on the capture probes such that upon hybridization of the probe fragments to the capture probes, the fluorophore is at least partially quenched by the quencher; and
C) detecting the presence of the target sequence based upon the quenching of the fluorophore on the capture probes.

In another embodiments, the invention is directed to a method of detecting the presence of at least a first target nucleic acid sequence in a sample, the method comprising:
A) subjecting the sample to an amplification reaction capable of amplifying the target nucleic acid sequence in the presence of a solid support described herein, wherein the solid support comprises at least a first set of nucleic acid probes, the first set of nucleic acid probes comprising a capture probe comprising a fluorophore attached thereto, and a target specific nucleic acid probe complementary to at least a portion of the capture probe and the target nucleic acid sequence and comprising a quencher attached thereto, such that the quencher quenches fluorescence from the fluorophore when the target specific probe is hybridized to the capture probe; and
B) detecting fluorescence from the sample following one or more cycles of the polymerase chain reaction, an increase in fluorescence being indicative of the presence of the target nucleic acid sequence.

The present incention also provides devices and consumables comprising the solid supports and soild substrates described herein. In one embodiments, the invention provides a nucleic acid detection device, the nucleic acid detection device comprising:
A) a detection chamber that comprises at least one high efficiency nucleic acid detection array on at least one surface of the chamber, the nucleic acid detection array comprising a solid support described herein, wherein the chamber is configured to reduce signal background for signals detected from the array;
B) a thermo-regulatory module operably coupled to the detection chamber, which module regulates temperature within the chamber during operation of the device; and,
C) an optical train that detects a signal produced at the array during operation of the device.

In other embodiments, the invention provides a nucleic acid detection consumable, the nucleic acid detection consumable comprising: a thin chamber less than about 500µm in depth, which chamber comprises an optically transparent window that comprises a high efficiency capture nucleic acid array disposed on an inner surface of the window, which chamber additionally comprises at least one reagent delivery port fluidly coupled to the chamber, wherein the consumable is configured to permit thermocycling of fluid within the chamber, wherein the high efficiency capture nucleic acid array comprises a solid support described herein.

In certain embodiments, the target analyte molecule is a DNA sequence, the DNA sequence having a sequence which indicates the presence of a pathogen, for example a virus, bacteria, plasmodium or fungus.

In some embodiments, the analyte probe is a flap. In some other embodiments, the analyte probe comprises a quencher. In some other embodiments, the analyte probe comprises a fluorophore. In still other embodiments, the capture probe comprises a fluorophore. In still other embodiments, the probe comprises an oligonucleotide.

The solid support may be any of the solid supports described herein. Further, in certain embodiments, the capture probe is a polynucleotide, and in other embodiments the target analyte molecule is a polynucleotide. In still other embodiments, the target analyte molecule is prepared via a polymerase chain reaction.

In some other embodiments, the signal is a fluorescent signal. For example, in some embodiments the fluorescent signal is produced as a result of specific hybridization of a target analyte molecule with a capture probe.

In other related embodiments, the invention provides a method for detecting an analyte in a sample. The method includes contacting the analyte with a solid support of the invention to allow capture of the analyte by the capture probe of the solid support of the invention and detecting capture of the analyte. In certain embodiments, the analyte is a biomolecule, such as a polypeptide, a nucleic acid, a carbohydrate, a lipid, or hybrids thereof. In other embodiments, the analyte is an organic molecule such as a drug, drug candidate, cofactor or metabolite. In another embodiment, the analyte is an inorganic molecule, such as a metal complex or cofactor. In an exemplary embodiment, the analyte is a nucleic acid which is a labeled probe. In another exemplary embodiment, the invention provides a reactive surface that covalently immobilizes a protein, an enzyme, an antibody, an antigen, a hormone, a carbohydrate, a glycoconjugate or a synthetically produced analyte target such as synthetically produced epitope that may be used to capture and detect an analyte in a subsequent step.

In various other embodiments, the invention provides a method of detecting a target nucleic acid using a solid support of the invention. The methods include binding a detectably labeled nucleic acid probe fragment to a nucleic acid of complementary sequence immobilized on the polymer of the solid support of the invention. An exemplary method includes:
A) hybridizing an amplification primer and a detectably labeled probe to the target nucleic acid;
B) amplifying at least a portion of the target nucleic acid in a primer dependent amplification reaction, wherein the amplification reaction results in cleavage of the labeled probe and release of a labeled probe fragment; and
C) hybridizing the labeled probe fragment to the immobilized assay component, wherein said component is a nucleic acid at least partially complementary to said labeled probe fragment, thereby detecting said nucleic acid.

Detection of the analyte can be accomplished by any art-recognized method or device. In certain embodiments, the analyte is detected by a fluorescent signal arising from an analyte or probe immobilized on the solid support. In an exemplary embodiment, the solid support of the invention is a nucleic acid array, and the signal arises from a fluorescently labeled nucleic acid hybridized to an assay component immobilized on the polymer of the solid support. In various embodiments, the immobilized assay component is a nucleic acid with a sequence at least partially complementary to the sequence of the fluorescently labeled nucleic acid. In selected embodiments in which the analyte is fluorescently labeled, it is detected by a fluorescence detector such as a CCD array. In certain embodiments the method involves profiling a certain class of analytes (e.g., biomolecules, e.g., nucleic acids) in a sample by applying the sample to one or more addressable locations of the solid support and detecting analytes captured at the addressable location or locations. Examples of methods useful for implementing the present invention include those described in Provisional U.S. Patent Application No. 61/561,198, and USSN 13/399,872, the full disclosures of which are hereby incorporated herein by reference in their entirety for all purposes.

In some embodiments, the solid supports of the present invention are useful for the isolation and detection of analytes in an assay mixture. In particular, solid supports of the invention are useful in performing assays of substantially any format including, but not limited to the polymerase chain reaction (PCR), chromatographic capture, immunoassays, competitive assays, DNA or RNA binding assays, fluorescence in situ hybridization (FISH), protein and nucleic acid profiling assays, sandwich assays and the like. The following discussion focuses on the use of a solid support of the invention to practice exemplary assays. This focus is for clarity of illustration only and is not intended to define or limit the scope of the invention. Those of skill in the art will appreciate that the method of the invention is broadly applicable to any assay technique for detecting the presence and/or amount of an analyte.

In various embodiments, the invention provides a method of detecting a target nucleic acid using a solid support of the invention. The methods includes binding a detectably labeled nucleic acid probe fragment to a nucleic acid of complementary sequence immobilized on the reactive polymer of the solid support of the invention. An exemplary method includes:
A) hybridizing an amplification primer and a detectably labeled probe to the target nucleic acid;
B) amplifying at least a portion of the target nucleic acid in a primer dependent amplification reaction, wherein the amplification reaction results in cleavage of the labeled probe and release of a labeled probe fragment; and
C) hybridizing the labeled probe fragment to the immobilized assay component, wherein said component is a nucleic acid at least partially complementary to said labeled probe fragment, thereby detecting said nucleic acid.

A sample can be from any source, and can be a biological sample, such as a sample from an organism or a group of organisms from the same or different species. A biological sample can be a sample of bodily fluid, for example, a blood sample, serum sample, lymph sample, a bone marrow sample, ascites fluid, pleural fluid, pelvic wash fluid, ocular fluid, urine, semen, sputum, or saliva. A biological sample can also be an extract from cutaneous, nasal, throat, or genital swabs, or extracts of fecal material. Biological samples can also be samples of organs or tissues, including tumors. Biological samples can also be samples of cell cultures, including both cell lines and primary cultures of both prokaryotic and eukaryotic cells.

A sample can be from the environment, such as from a body of water or from the soil, or from a food, beverage, or water source, an industrial source, workplace area, public area, or living area. A sample can be an extract, for example a liquid extract of a soil or food sample. A sample can be a solution made from washing or soaking, or suspending a swab from, articles such as tools, articles of clothing, artifacts, or other materials. Samples also include samples for identification of biowarfare agents, for example samples of powders or liquids of known or unknown origin.

A sample can be an unprocessed or a processed sample; processing can involve steps that increase the purity, concentration, or accessibility of components of the sample to facilitate the analysis of the sample. As non-limiting examples, processing can include steps that reduce the volume of a sample, remove or separate components of a sample, solubilize a sample or one or more sample components, or disrupt, modify, expose, release, or isolate components of a sample. Non-limiting examples of such procedures are centrifugation, precipitation, filtration, homogenization, cell lysis, binding of antibodies, cell separation, etc. For example, in some preferred embodiments of the present invention, the sample is a blood sample that is at least partially processed, for example, by the removal of red blood cells, by concentration, by selection of one or more cell or virus types (for example, white blood cells or pathogenic cells), or by lysis of cells, etc.

Exemplary samples include a solution of at least partially purified nucleic acid molecules. The nucleic acid molecules can be from a single source or multiple sources, and can comprise DNA, RNA, or both. For example, a solution of nucleic acid molecules can be a sample that was subjected to any of the steps of cell lysis, concentration, extraction, precipitation, nucleic acid selection (such as, for example, poly A RNA selection or selection of DNA sequences comprising Alu elements), or treatment with one or more enzymes. The sample can also be a solution that comprises synthetic nucleic acid molecules.

In an exemplary embodiment, when the solid support of the invention is used to detect and/or characterize a nucleic acid, the solid support of the invention is a nucleic acid array having a plurality of nucleic acids of different sequences covalently bound to the surface-bound polymer at known locations on the solid support. In various embodiments, the solid support is a component of a reaction vessel in which PCR is performed on a target nucleic acid sample contained in an assay mixture. In an exemplary method, one or more nucleic acid primer and a detectably labeled nucleic acid probe are hybridized to the target nucleic acid. During PCR template extension, the probe is cleaved, producing a probe fragment. The probe fragment is released from the target nucleic acid and is captured by an immobilized analyte component, which is a nucleic acid, on the surface bound polymer. The probe sequence is determined by its binding location on the array.

In various embodiments the solid supports of the invention are utilized as a component of a multiplex assay for detecting one or more species in an assay mixture. The solid supports of the invention are particularly useful in performing multiplex-type analyses and assays. In an exemplary multiplex analysis, two or more distinct species (or regions of one or more species) are detected using two or more probes, wherein each of the probes is labeled with a different fluorophore. The solid supports of the invention allow for the design of multiplex assays in which more than one detectably labeled probe structure is used in the assay. A number of different multiplex assays using the solid supports of the invention will be apparent to one of skill in the art. In one exemplary assay, each of at least two distinct fluorophores is used to signal hybridization of a nucleic acid probe fragment to a surface immobilized nucleic acid.

Exemplary labeled probes of use in practicing the methods of the invention are nucleic acid probes. Useful nucleic-acid probes include those that can be used as components of detection agents in a variety of DNA amplification/quantification strategies including, for example, 5'-nuclease assay, Strand Displacement Amplification (SDA), Nucleic Acid Sequence-Based Amplification (NASBA), Rolling Circle Amplification (RCA), as well as for direct detection of targets in solution phase or solid phase (e.g., array) assays. Furthermore, the solid supports and oligomers can be used in probes of substantially any format, including, for example, format selected from molecular beacons, Scorpion probes™, Sunrise probes™, conformationally assisted probes, light up probes, Invader Detection probes, and TaqMan™ probes. See, for example, Cardullo, R., et al., Proc. Natl. Acad. Sci. USA, 85:8790-8794 (1988); Dexter, D.L., J. Chem. Physics, 21:836-850 (1953); Hochstrasser, R.A., et al., Biophysical Chemistry, 45:133-141 (1992) ; Selvin, P., Methods in Enzymology, 246:300-334 (1995); Steinberg, I., Ann. Rev. Biochem., 40:83-114 (1971); Stryer, L., Ann. Rev. Biochem., 47:819-846 (1978); Wang, G., et al., Tetrahedron Letters, 31:6493-6496 (1990); Wang, Y., et al., Anal. Chem., 67:1197-1203 (1995); Debouck, C., et al., in supplement to nature genetics, 21:48-50 (1999); Rehman, F.N., et al., Nucleic Acids Research, 27:649-655 (1999); Cooper, J.P., et al., Biochemistry, 29:9261-9268 (1990); Gibson, E.M., et al., Genome Methods, 6:995-1001 (1996); Hochstrasser, R.A., et al., Biophysical Chemistry, 45:133-141 (1992); Holland, P.M., et al., Proc Natl. Acad. Sci USA, 88:7276-7289 (1991); Lee, L.G., et al., Nucleic Acids Rsch., 21:3761-3766 (1993); Livak, K.J., et al., PCR Methods and Applications, Cold Spring Harbor Press (1995); Vamosi, G., et al., Biophysical Journal, 71:972-994 (1996); Wittwer, C.T., et al., Biotechniques, 22:176-181 (1997); Wittwer, C.T., et al., Biotechniques, 22:130-38 (1997); Giesendorf, B.A.J., et al., Clinical Chemistry, 44:482-486 (1998); Kostrikis, L.G., et al., Science, 279:1228-1229 (1998); Matsuo, T., Biochemica et Biophysica Acta, 1379:178-184 (1998); Piatek, A.S., et al., Nature Biotechnology, 16:359-363 (1998); Schofield, P., et al., Appl. Environ. Microbiology, 63:1143-1147 (1997); Tyagi S., et al., Nature Biotechnology, 16:49-53 (1998); Tyagi, S., et al., Nature Biotechnology, 14:303-308 (1996); Nazarenko, I.A., et al., Nucleic Acids Research, 25:2516-2521 (1997); Uehara, H., et al., Biotechniques, 26:552-558 (1999); D. Whitcombe, et al., Nature Biotechnology, 17:804-807 (1999); Lyamichev, V., et al., Nature Biotechnology, 17:292 (1999); Daubendiek, et al., Nature Biotechnology, 15:273-277 (1997); Lizardi, P.M., et al., Nature Genetics, 19:225-232 (1998); Walker, G., et al., Nucleic Acids Res., 20:1691-1696 (1992); Walker, G.T., et al., Clinical Chemistry, 42:9-13 (1996); and Compton, J., Nature, 350:91-92 (1991), the disclosures of which are each incorporated herein by reference in their entireties for all purposes.

In various embodiments, the present invention provides methods of detecting polymorphism in target nucleic acid sequences. Polymorphism refers to the occurrence of two or more genetically determined alternative sequences or alleles in a population. A polymorphic marker or site is the locus at which divergence occurs. Exemplary markers have at least two alleles, each occurring at frequency of greater than 1%, and more preferably greater than 10% or 20% of a selected population. A polymorphic locus may be as small as one base pair. Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The first identified allelic form is arbitrarily designated as the reference form and other allelic forms are designated as alternative or variant alleles. The allelic form occurring most frequently in a selected population is sometimes referred to as the wildtype form. Diploid organisms may be homozygous or heterozygous for allelic forms. A diallelic polymorphism has two forms. A triallelic polymorphism has three forms.

In an exemplary embodiment, solid support of the invention is utilized to detect a single nucleotide polymorphism. A single nucleotide polymorphism occurs at a polymorphic site occupied by a single nucleotide, which is the site of variation between allelic sequences. The site is usually preceded by and followed by highly conserved sequences of the allele (e.g., sequences that vary in less than 1/100 or 1/1000 members of the populations). A single nucleotide polymorphism usually arises due to substitution of one nucleotide for another at the polymorphic site. A transition is the replacement of one purine by another purine or one pyrimidine by another pyrimidine. A transversion is the replacement of a purine by a pyrimidine or vice versa. Single nucleotide polymorphisms can also arise from a deletion of a nucleotide or an insertion of a nucleotide relative to a reference allele.

In embodiments in which polymorphism is detected, polymorphic nucleic acids are bound to the solid support at addressable locations. Occurrence of a detectable signal at a particular location is indicative of the presence of a polymorphism in the target nucleic acid sequence.

In an exemplary embodiment, the probe is detectably labeled with a fluorophore moiety. There is a great deal of practical guidance available in the literature for selecting appropriate fluorophores for particular probes, as exemplified by the following references: Pesce et al., Eds., FLUORESCENCE SPECTROSCOPY (Marcel Dekker, New York, 1971); White et al., FLUORESCENCE ANALYSIS: A PRACTICAL APPROACH (Marcel Dekker, New York, 1970); and the like. The literature also includes references providing exhaustive lists of fluorescent and chromogenic molecules and their relevant optical properties for choosing fluorophores (see, for example, Berlman, HANDBOOK OF FLUORESCENCE SPECTRA OF AROMATIC MOLECULES, 2nd Edition (Academic Press, New York, 1971); Griffiths, COLOUR AND CONSTITUTION OF ORGANIC MOLECULES (Academic Press, New York, 1976); Bishop, Ed., INDICATORS (Pergamon Press, Oxford, 1972); Haugland, HANDBOOK OF FLUORESCENT PROBES AND RESEARCH CHEMICALS (Molecular Probes, Eugene, 1992) Pringsheim, FLUORESCENCE AND PHOSPHORESCENCE (Interscience Publishers, New York, 1949); and the like. Further, there is extensive guidance in the literature for derivatizing fluorophore molecules for covalent attachment via common reactive groups that can be added to a nucleic acid, as exemplified by the following references: Haugland (supra); Ullman et al., U.S. Pat. No. 3,996,345; Khanna et al., U.S. Pat. No. 4,351,760. Thus, it is well within the abilities of those of skill in the art to choose an energy exchange pair for a particular application and to conjugate the members of this pair to a probe molecule, such as, for example, a nucleic acid, peptide or other polymer.

In view of the well-developed body of literature concerning the conjugation of small molecules to nucleic acids, many other methods of attaching donor/acceptor pairs to nucleic acids will be apparent to those of skill in the art. For example, rhodamine and fluorescein dyes are conveniently attached to the 5'-hydroxyl of an nucleic acid at the conclusion of solid phase synthesis by way of dyes derivatized with a phosphoramidite moiety (see, for example, Woo et al., U.S. Pat. No. 5,231,191; and Hobbs, Jr., U.S. Pat. No. 4,997,928).

More specifically, there are many linker moieties and methodologies for attaching groups to the 5'- or 3'-termini of nucleic acids, as exemplified by the following references: Eckstein, editor, Nucleic acids and Analogues: A Practical Approach (IRL Press, Oxford, 1991); Zuckerman et al., Nucleic Acids Research, 15: 5305-5321 (1987) (3'-thiol group on nucleic acid); Sharma et al., Nucleic Acids Research, 19: 3019 (1991) (3'-sulfhydryl); Giusti et al., PCR Methods and Applications, 2: 223-227 (1993) and Fung et al., U.S. Pat. No. 4,757,141 (5'-phosphoamino group via Aminolink TM II available from P.E. Biosystems, CA.) Stabinsky, U.S. Pat. No. 4,739,044 (3-aminoalkylphosphoryl group); Agrawal et al., Tetrahedron Letters, 31: 1543-1546 (1990) (attachment via phosphoramidites linkages); Sproat et al., Nucleic Acids Research, 15: 4837 (1987) (5-mercapto group); Nelson et al., Nucleic Acids Research, 17: 7187-7194 (1989) (3'-amino group), and the like.

Means of detecting fluorescent labels are well known to those of skill in the art. Thus, for example, fluorescent labels can be detected by exciting the fluorophore with an appropriate wavelength of light and detecting the resulting fluorescence. The fluorescence can be detected visually, by means of photographic film, by the use of electronic detectors such as charge coupled solid supports (CCDs) or photomultipliers and the like. Similarly, enzymatic labels may be detected by providing the appropriate substrates for the enzyme and detecting the resulting reaction product.

Though exemplified by reference to detection of a fluorescent labeled nucleic acid, the solid supports of this invention are useful for the detection of analyte molecules. When the polymer is functionalized with a binding group, the solid support will capture onto the surface analytes that bind to the particular group. Unbound materials can be washed off, and the analyte can be detected in any number of ways including, for example, a gas phase ion spectrometry method, an optical method, an electrochemical method, atomic force microscopy and a radio frequency method. Exemplary optical methods include, for example, detection of fluorescence, luminescence, chemiluminescence, absorbance, reflectance, transmittance, birefringence or refractive index (e.g., surface plasmon resonance, ellipsometry, quartz crystal microbalance, a resonant mirror method, a grating coupler waveguide method (e.g., wavelength-interrogated optical sensor ("WIOS") or interferometry). Optical methods include microscopy (both confocal and non-confocal), imaging methods and non-imaging methods. Immunoassays in various formats (e.g., ELISA) are popular methods for detection of analytes captured on a solid phase. Electrochemical methods include voltammetry and amperometry methods. Radio frequency methods include multipolar resonance spectroscopy or interferometry. Optical methods include microscopy (both confocal and non-confocal), imaging methods and non-imaging methods. Immunoassays in various formats (e.g., ELISA) are popular methods for detection of analytes captured on a solid phase. Electrochemical methods include voltammetry and amperometry methods. Radio frequency methods include multipolar resonance spectroscopy.

Conditions that favor hybridization between an oligomer of the present invention and target nucleic acid molecules can be determined empirically by those skilled in the art, and can include optimal incubation temperatures, salt concentrations, length and base compositions of oligonucleotide analogue probes, and concentrations of oligomer and nucleic acid molecules of the sample. Preferably, hybridization is performed in the presence of at least one millimolar magnesium ion and at a pH that is above 6.0. In some embodiments, it may be necessary or desirable to treat a sample to render nucleic acid molecules in the sample single-stranded prior to hybridization. Examples of such treatments include, but are not limited to, treatment with base (preferably followed by neutralization), incubation at high temperature, or treatment with nucleases.

In addition, because the salt dependence of hybridization to nucleic acids is largely determined by the charge density of the backbone of a hybridizing oligonucleotide analogue, increasing the ratio of pPNA monomers in a HypNA-pPNA oligomer or a SerNA-pPNA oligomer of the present invention can increase the salt dependence of hybridization. This can be used to advantage in the methods of the present invention where it can in some aspects be desirable to be able to increase the stringency of hybridization by changing salt conditions, for example, or release a hybridized nucleic acid by reducing the salt concentration. In yet other aspects of the present invention, it can be desirable to have high-affinity binding of an oligonucleotide analogue of the present invention to a nucleic acid in very low salt. In this case, maintaining a ratio of close to 1:1 of HypNA to pPNA monomers in an oligonucleotide analogue of the present invention is advantageous.

The high degree of specificity of oligomers of the present invention in binding to target nucleic acid molecules allow the practitioner to select hybridization conditions that can favor discrimination between nucleic acid sequences that comprise a stretch of sequence that is completely complementary to at least a portion of one or more oligomer and target nucleic acid molecules that comprise a stretch of sequence that comprises a small number of non-complementary bases within a substantially complementary sequence. For example, hybridization or wash temperatures can be selected that permit stable hybrids between oligomer of the present invention and target nucleic acid molecules that are completely complementary along a stretch of sequence but promote dissociation of hybrids between oligomer of the present invention and target nucleic acid molecules that are not completely complementary, including those that comprise one or two base mismatches along a stretch of complementary sequence. The selection of a temperature for hybridization and washes can be dependent, at least in part, on other conditions, such as the salt concentration, the concentration of oligomer and target nucleic acid molecules, the relative proportions of oligomer to target nucleic acid molecules, the length of the oligomers to be hybridized, the base composition of the oligomer and target nucleic acid molecules, the monomer composition of the oligonucleotide analogue molecules, etc. In addition, when selecting for conditions that favor stable hybrids of completely complementary molecules and disfavor stable hybrids between oligomer and target nucleic acid molecules that are mismatched by one or more bases, additional conditions can be taken into account, and, where desirable, altered, including but not limited to, the length of the oligonucleotide analogue to be hybridized, the length of the stretch of sequence of complementarity between oligomer and target nucleic acid molecules, the number of non-complementary bases within a stretch of sequence of complementarity, the identity of mismatched bases, the identity of bases in the vicinity of the mismatched bases, and the relative position of any mismatched bases along a stretch of complementarity. Those skilled in the art of nucleic acid hybridization would be able to determine favorable hybridization and wash conditions in using oligomer of the present invention for hybridization to target nucleic acid molecules, depending on the particular application. "Favorable conditions" can be those favoring stable hybrids between oligomer and target nucleic acid molecules that are, at least in part, substantially complementary, including those that comprise one or more mismatches.

"Favorable conditions" can be those favoring stable hybrids between oligomer and target nucleic acid molecules that are, at least in part, completely complementary and disfavor or destabilize hybrids between molecules that are not completely complementary.

Using methods such as those disclosed herein, the melting temperature of oligomer of the present invention hybridized to target nucleic acid molecules of different sequences can be determined and can be used in determining favorable conditions for a given application. It is also possible to empirically determine favorable hybridization conditions by, for example, hybridizing target nucleic acid molecules to oligomer that are attached to a solid support and detecting hybridized complexes.

Target nucleic acid molecules that are bound to solid supports or oligomeric probes of the present invention can be conveniently and efficiently separated from unbound nucleic acid molecules of the survey population by the direct or indirect attachment of oligomer probes to a solid support. A solid support can be washed at high stringency to remove nucleic acid molecules that are not bound to oligomer probes. However, the attachment of oligomer probes to a solid support is not a requirement of the present invention. For example, in some applications bound and unbound nucleic acid molecules can be separated by centrifugation through a matrix or by phase separation or some by other forms of separation (for example, differential precipitation) that can optionally be aided by chemical groups incorporated into the oligomer probes (see, for example, U.S. Pat. No. 6,060,242 issued May 9, 2000, to Nie et al.).

In an exemplary embodiment, a solid support of the invention is utilized in a real time PCR assay such as those described in commonly owned, copending United States Patent Application No. 13/399,872.

In other methods of the invention, the present invention is directed to a method for preparing a solid support having a probe molecule bound thereto, the method comprising contacting the solid support comprising an azide or alkyne moiety covalently bound to the outer surface of the solid support (as described herein above) with the polymer comprising A, B and C subunits described herein above.

In other embodiments, the methods further comprise contacting a Cu(I) catalyst with the solid support and the polymer. Further embodiments comprise contacting a probe molecule having an amine functional group with the solid support comprising a polymer bound thereto to prepare a solid support comprising a probe molecule bound thereto. Such methods have utility in any number of applications, such as preparation of DNA microarrays and the like.

All of the U.S. patents, U.S. patent application publications, U.S. patent applications, foreign patents, foreign patent applications and non-patent publications referred to in this specification are incorporated herein by reference, in their entirety to the extent not inconsistent with the present description.

From the foregoing it will be appreciated that, although specific embodiments of the invention have been described herein for purposes of illustration, various modifications may be made without deviating from the spirit and scope of the invention. Accordingly, the invention is not limited except as by the appended claims.

The following examples are provided for purposes of illustration, not limitation.

### EXAMPLES

### EXAMPLE 1

### SYNTHESIS OF BOC-NH-PEG₅₇-ACET-(4-AZIDO)ANILIDE

4-Azidoaniline hydrochloride (51 mg, 300 µmol, Sigma-Aldrich) was dissolved in water (10 mL) and 1N NaOH (10 mL), then was extracted into EtOAc (3 x 20 mL). The organic phase was washed with water, then with saturated NaCl, and then dried over Na₂SO₄. Evaporation gave the free base of azidoaniline as an amber oil. Glassware was wrapped in foil throughout to minimize exposure to room light. In a separate flask (oven dried) was placed Boc-amino-PEG₅₇-acetic acid NHS ester, average MW 2800 (280 mg, 100 µmol, Laysan Bio, Arab, AL) along with dry DMF (250 µL) and TEA (27 µL, 200 µmol). To this was added the azidoaniline in a small amount of DMF. The flask was purged with Ar, then sealed, covered with foil, and shaken overnight. By TLC (DCM/MeOH/TEA, 80:20:1) a single UV-active product was observed (R_{f}=0.25) along with unreacted azidoaniline (R_{f}=0.88). The reaction mixture was diluted with EtOAc (5 mL) and product was precipitated by dropwise addition to vortexing hexane and left in a freezer at -20C overnight. The precipitate was collected on a glass frit, washed with hexane, then redissolved in EtOAc and reprecipitated as before. After collection, the product was redried from MeOH to a constant weight (189 mg, 65%). The beige, hygroscopic solid was sealed and stored in the dark until use. ¹H-NMR (400MHz, DMSO-d6) *δ*: 9.36 (s, 1H), 7.69 (d, 2H), 7.08 (d, 2H), 6.62 (dd, 1H), 4.07 (s, 2H), 3.4-3.6 (m, 230H), 2.91 (dd, 2H), 1.37 (s, 9H); ¹³C-NMR *δ*: 172.24, 172.17, 168.80, 156.11, 136.19, 134.73, 121.71, 119.91, 73.18, 51.87, 40.08, 28.78.

### EXAMPLE 2

### SYNTHESIS OF BOC-NH-PEG₄₄-ACET-(4-AZIDO)ANILIDE

4-Azidoaniline hydrochloride (100 mg, 600 µmol, Sigma-Aldrich) was dissolved in water (20 mL) and 1N NaOH (20 mL), then was extracted into EtOAc (3 x 40 mL). The extract was handled as above to give the free base. In a separate flask (oven dried) was placed Boc-amino-PEG₄₄-acetic acid NHS ester, average MW 2000 (400 mg, 200 µmol, Laysan Bio, Arab, AL) along with dry DMF (1000 µL) and TEA (55 uL, 400 µmol). To this was added the azidoaniline in a small amount of DMF. The flask was purged with Ar, sealed, covered, and shaken overnight. By TLC (DCM/MeOH/TEA, 80:20:1) a single UV-active product was observed (R_{f}=0.38) along with unreacted azidoaniline (R_{f}=0.88). The reaction mixture was diluted with EtOAc (10 mL) and product was precipitated by dropwise addition to vortexing pentane and left in at a freezer at -20C overnight. The precipitate was collected on a glass frit, washed with hexane, then redissolved in EtOAc and reprecipitated as before. After collection, the product was redried from MeOH to a constant weight (272 mg, 65%). The beige, hygroscopic solid was sealed and stored in the dark until use. ¹H-NMR (DMSO-d6) *δ*: 9.69 (s, 1H), 7.69 (d, 2H), 7.08 (d, 2H), 6.74 (dd, 1H), 4.07 (s, 2H), 3.53 (s, 2H), 3.46-3.52 (m, 178H), 3.05 (dd, 2H), 1.37 (s, 9H).

### EXAMPLE 3

### SYNTHESIS OF N-(4-AZIDOBENZOYL)POLYALLYLAMINE

The procedure was adapted from Sugawara et al., Macromolecules 27, 7809-14, (1994). A solution of KHCO₃ (0.28 g, 2.8 mmol) in water (42 mL) was prepared. To this was added polyallylamine hydrochloride, MW 120-200K (Alfa Aesar, 0.69 g, ∼9.4 mmol amine), 4-azidobenzoic acid (TCI, 0.38 g, 2.8 mmol), and DMF (14 mL). The mixture was stirred and cooled in an ice bath. Then a solution of EDC (0.59 g, 3.1 mmol) in a mixture of DMF (1 mL) and water (0.5 mL) was added slowly, dropwise. The reaction mixture was stirred to RT overnight, shielded from ambient light. Overnight, the pH of the solution was 6.7. The solution was transferred to a large dialysis tube (Snakeskin MWCO 7000, Pierce) and dialyzed in water (14L) for 24 hrs. The water was changed 2x, for a total dialysis time of 72 hrs. The dialysate was frozen in microcentrifuge tubes and lyophilized (Speedvac) to give 0.70 g of the azidobenzoyl polymer. ¹H-NMR (D₂O) *δ*: 7.87 (s, 2H), 7.03 (d, 2H), 3.01 (br s, 8.8H), 1.96 (br s, 4.4H), 1.48 (br s, 4.4H). Based on the ratio of aromatic to aliphatic (polymer backbone) protons, the azidobenzoyl substitution was ∼1 per 4.4 amines, or 23%.

### EXAMPLE 4

### SURFACE PRETREATMENT OF GLASS SLIDES

Glass microscope slides (1" x 3" x 1 mm) were sonicated for 20 minutes in a glass staining jar containing 0.5 wt% solution of SDS, and rinsed thoroughly with DI water. They were next sonicated for 20 minutes in a mixture of 29% NH₄OH, 30% H₂O₂, and DI water in 1:1:5 v/v ratio, and rinsed thoroughly with DI water. The slides were then sonicated for 20 minutes in a mixture of 38% HCl. 30% H₂O₂, and DI water in 1:1:6 v/v ratio, and rinsed thoroughly with DI water. The pretreated slides were stored in DI water in a capped jar. Prior to use, the slides were removed from its water storage, blow-dried with argon, and baked at 110 °C for 5 minutes. The pretreated glass slides exhibited water contact angles < 10 °.

### EXAMPLE 5

### SILYLATION OF GLASS SLIDES WITH 3-AMINOPROPYL DIISOPROPYLETHOXYSILANE

Five pretreated glass slides were immersed into a mixture anhydrous EtOH (30 mL), 3-aminopropyl diisopropylethoxysilane (500 µL) and triethylamine (TEA, 20 □L) in a screw-capped polypropylene staining tube. The tube was swirled gently on an orbital shaker for 2-3 hours. The slides were removed from the reaction mixture, rinsed with plenty of 95% EtOH, blown dry with argon, and annealed in a 110 °C oven for 5 minutes. The silylated glass slides exhibited water contact angles of 55.8° ±1.8°, and they were used immediately after preparation.

### EXAMPLE 6

### GENERAL PROCEDURE FOR THE PREPARATION OF POLYMER COMPRISING ONE OR MORE AZIDE OR ALKYNE SUBUNITS AND N,N-DIMETHYLACRYLAMIDE AND PENTAFLUOROPHENYL ACRYLATE SUBUNITS

Subunits are purified by vacuum distillation at 58-60 °C/3.5 mm Hg and 42-43 °C/5 torr, respectively, prior to use. To a mixture containing azide or alkyne containing subunits (molar ratio according to desired polymer), DMA (991.3 mg, 10.0 mmol), PFPA (238.11 mg, 1.0 mmol), 2,2'-azobis(2,4-dimethylvaleronitrile) (1.0 mg), and ACN (3.0 mL) in a glass ampoule, ultra-pure argon is bubbled for 1 minute. Then the ampoule is sealed and placed in a 55 °C oil bath for 22 hrs. The seal is broken, the solvent is removed under reduced pressure, and the residue iss dissolved in a minimum amount of THF. The THF solution iss added dropwise into 10x volume of *n*-pentane with constant stirring. The precipitated polymer iss centrifuged and the supernatant discarded. The polymer is triturated in pentane (40 mL), filtered, and vacuum dried at 40°C.

This general procedure is applicable for the preparation of various copolymers having alkyne or azide subunits, DMA and PFPA. The ratio of subunits is varied to obtain polymers of different ratios.

### EXAMPLE 7

### GENERAL PROCEDURE FOR THE SURFACE IMMOBILIZATION OF POLYMERS ON SOLID SUBSTRATES VIA CLICK CHEMISTRY

A polymer prepared according to example 6 is contacted with a solid substrate comprising an alkyne or azide moiety (depending on whether the polymer contains a alkyne or azide) on the outer surface in a solution comprising Cu(I). Solvent and remaining reactants are removed , for example by washing, to obtain a solid support comprising the polymer covalently linked via a triazole moiety. The water contact angle ranges from about 50° to 70°.

Since the polymer is attached to the solid substrate via an alkyne or azide functional group, the activated ester is available for conjugation to a capture probe in an orthogonal manner.

### EXAMPLE 8

### GENERAL PROCEDURE FOR THE SURFACE IMMOBILIZATION OF POLYMERS ON SOILD SUBSTRATES VIA ACTIVATED ESTERS

In a polypropylene slide staining tube containing ACN (30 mL) is added TEA (150 µL) and a polymer prepared as described in Example 6. To this solution, four aminosilylated microscope glass slides are immersed and tumbled gently for 4-16 hours. They are removed, rinsed with plenty of acetonitrile, and blow-dried with argon. The water contact angle ranges from about 50° to 70°.

Since the polymer is attached to the solid substrate via an activated ester, the alkyne or azide functional groups are available for conjugation to a capture probe via click chemistry.

### EXAMPLE 9

### PREPARATION OF POLYMER COMPRISING ALKYNE OR AZIDE, N,N-DIMETHYLACRYLAMIDE, AND 2-VINYL-4,4'-DIMETHYLAZLACTONE SUBUNITS

2-Vinyl-4,4'-dimethylazlactone (VAL, Polysciences, Inc.) is purified by vacuum distillation at 26-27 °C/ 600 millitorr. A mixture of redistilled DMA (4.80 g, 48.5 mmol), VAL (0.74 g, 5.39 mmol), alkyne or azide subunit (molar ratio according to desired polymer) and AIBN (15.9 mg, 0.097 mmol) in ACN (30 mL), is placed in a 150-mL 14/20 three-neck flask equipped with a water-cooled condenser, a 20 cm 19-gauge SS bleeding needle connected to ultra-pure Ar, and a venting 19 gauge SS needle connected to a mineral oil bubbler. Argon is bubbled through the solution for 20 minutes, then the flask is immersed in an oil bath at 71 °C with constant stirring for 3-4 hours. The solvent is removed under reduced pressure at 60 °C. The residue is dried under vacuum at 50°C for 2-3 hours to yield a glassy polymer coated onto the flask wall. The polymer is then redissolved in methylethylketone (15 mL). With constant stirring, petroleum ether is added dropwise until the solution turned cloudy. This cloudy suspension is then added dropwise into an excess of petroleum ether (200 mL) with vigorous stirring. The resulting supernatant is discarded and the residual polymer is triturated in fresh petroleum ether (200 mL) for 10 minutes. The precipitated polymer is filtered, rinsed with petroleum ether, and vacuum dried. Polymers containing different ratios of subunits are prepared in a similar manner by adjusting the ration of monomer subunits.

### EXAMPLE 10

### GRAFTING OF AZIDOBENZOYL-(10 MOL%)-POLY(ALLYLAMINE) ONTO COC SLIDES FROM SOLUTION BY UV, AND SUBSEQUENT SURFACE IMMOBILIZATION OF POLYMER THERETO

A grafting solution was prepared by dissolving *N*-azidobenzoyl-poly(allylamine) (4.0 mg), containing about 10 mol% 4-azidobenzoyl groups, in DI water (180 µL). The COC slide (1" x 3" x 1 mm) was rinsed thoroughly with 95% EtOH and blow-dried with Ar. The dry COC slide exhibited water contact angles greater than 85° prior to UV-grafting. An aliquot of the polymer solution (90 µL) was transferred onto the center of the COC slide and it was spread to cover the whole COC slide by laying down a Quartz slide (1" x 3" x 1 mm) on top of it. A total of two samples were prepared for UV grafting. The sandwich assembles were placed 5 cm underneath a 365 nm UV light source (BondWang, Electro-Lite Corp., 10 mW/cm²) and exposed to UV irradiation for 15 minutes. The sandwich assembles were immersed in DI water and the COC slides were separated and rinsed well with DI water. The slides were then tumbled in 0.5 N HCl (30 mL) for 60 minutes. The 0.5 N HCl was replaced once with an additional 60 min tumbling, then they were then rinsed with DI water and blow-dried with Ar. The contact angle was 31.7° ± 3.7°.

To anhydrous acetonitrile (30 mL) in a polypropylene staining tube is added TEA (150 µL) and a polymer prepared according to Example 6. The two washed and dry UV-grafted COC slides are immersed in this solution and tumbled gently for 4-16 hours, then were removed, rinsed well with acetonitrile, and blow-dried with argon.

The resulting surface immobilized polymer comprises alkyne or azide moieties available for conjugation to a capture probe via click chemistry. Polymers are immobilized to solid substrates using an analogous procedure wherein the surface comprises an azide or alkyne, rather than amine.

This procedure can be applied to various azidobenzoyl-poly(allylamine) compositions having a range of 4-azidobenzoyl substitution levels, for subsequent UV-grafting onto COC or COP surfaces.

### EXAMPLE 11

### GRAFTING OF DRY FILMS OF BOC-AMINO-PEG₅₇-ACET-(4-AZIDO)ANILIDE ONTO COP SLIDES BY UV, DEPROTECTION OF AMINO GROUPS, AND SUBSEQUENT SURFACE IMMOBILIZATION OF A REPRESENTATIVE POLYMER

A grafting solution was prepared by dissolving Boc-amino-PEG₅₇-acet-(4-azido)anilide (20.1 mg) in DI water (200 µL). A COP slide (1" x 3" x 1 mm) was rinsed thoroughly with 95% EtOH and blow-dried with Ar. The dry slide exhibited water contact angles greater than 90°. One side of the slide was treated by corona discharge in open air, passing the slide 7 times at a distance of 0.5 cm under a Corona Treater (Electro-Technic Products, Inc., Model BD-20). The corona-treated surface exhibited water contact angle of 40.0° ±1.9°. To the center of the corona-treated surface an aliquot of the grafting solution (100 µL) was placed and spread to cover the whole surface with a stainless steel spatula. The grafting solution coated the surface uniformly and dried to form a thin film after evaporation at ambient temperature overnight in a dark box. With the dry film facing up, the slide was then placed 5 cm underneath a 365 nm UV light source (BondWang, Electro-Lite Corp., 10 mW/cm²) and exposed to UV irradiation for 15 minutes. The UV-irradiated surface was rinsed well with acetonitrile and blown dry with argon to give a grafted surface with water contact angle of 57.1° ± 1.6°. To deprotect the grafted amino groups, the slide was immersed in 5% trifluoroacetic acid in MeOH and tumbled for 60 minutes. The slide was then rinsed with plenty of MeOH and blow-dried with Ar to give an amine surface with water contact angles of 61.9° ± 3.7°.

The slide is subsequently immersed in a solution of comprising a polymer prepared according to Example 6 and TEA (150 µL) in ACN (30 mL). The slide is tumbled for 60 minutes, rinsed with plenty of acetonitrile, and blown dry with argon to give a surface with water contact angle of 53° to 70°.

The resulting surface immobilized polymer comprises alkyne or azide moieties available for conjugation to a capture probe via click chemistry. Polymers are immobilized to solid substrates using an analogous procedure wherein the surface comprises an azide or alkyne, rather than amine.

### EXAMPLE 12

### AMINOSILYLATTON OF 100Å SiO₂ SPUTTERED SURFACES AND SUBSEQUENT IMMOBILIZATION OF A REPRESENTATIVE POLYMER

One face of a COC or COP slide was treated by plasma etching and subsequent SiO₂ sputtering with palate at 80°C (Hionix, Inc., San Jose, CA). The average thickness of deposited SiO₂ was 100Å and water contact angles were less than 10°. The silica-sputtered COC and COP slides were immersed in a solution of 3-aminopropyl diisopropylethoxysilane (250 µL) and TEA (20 µL) in anhydrous EtOH (30 mL). The slides were tumbled gently for 2 hours, rinsed well with EtOH, and blow-dried with argon to give water contact angles of 58.2° ± 1.4° and 55.9° ± 2.4° for the COC and COP slides, respectively.

An aminosilylated COC or COP slide is individually immersed in acetonitrile (30 mL) containing triethylamine (150 µL). To the immersed COC or COP slide, a polymer prepared according to Example 6 is added (28.6 mg for COC, or 23.7 mg for COP). The COC or COP slide is tumbled overnight, then rinsed with plenty of acetonitrile and blown dry with argon. The water contact angle is determined.

### EXAMPLE 13

### UV-INITIATED INTERFACIAL POLYMERIZATION OF A REPRESENTATIVE POLYMER ON COC SURFACES, WITHOUT A CROSSLINKER

*N,N*-dimethylacrylamide (DMA), pentafluorophenyl acrylate (PFPA) and alkyne or azide containing subunits are purified by vacuum distillation as described previously. A solution of monomers is prepared by dissolving DMA (800 mg, 8.07 mmol), PFPA (68.0 mg, 0.286 mmol), azide or alkyne subunit (molar ratio varied depending on desired polymer composition) and benzophenone (31.8 mg, 0.175 mmol) in acetonitrile (1.0 mL). An aliquot of the monomer solution (150 µL) is placed at the center of the COC slide and then spread to cover the whole COC slide by laying down a PTFE slide (1" x 3" x 1 mm) on top of it. The sandwich assembly is then inverted, with COC slide facing up, and placed 5 cm underneath a 365 nm UV light source (BondWang, Electro-Lite Corp., 10 mW/cm²) and exposed to UV irradiation for 15 minutes. The PTFE slide is then peeled off and the COC slide is rinsed with acetonitrile thoroughly, then blow-dried with argon to give a grafted surface having a water contact angle of 50° to 70°. The procedure is generally applicable for the preparation of grafted copolymers of DMA and PFPA having various monomer molar ratios.

### EXAMPLE 14

### UV-INITIATED INTERFACIAL POLYMERIZATION OF A REPRESENTATIVE POLYMER ON COC SURFACES WITH A CROSSLINKER

The general procedure of Example 13 is applied. The monomer solution is prepared by dissolving DMA (794.4 mg, 8.01 mmol), PFPA (668 mg, 0.281 mmol), methylene-*bis*-acrylamide (MBA, 40.0 mg, 0.259 mmol), alkyne or azide monomer (molar ration depending on desired polymer) and benzophenone (32.0 mg, 0.176 mmol) in ACN (10.0 mL). An aliquot of the monomer solution (50 µL) is applied at the center of the COC slide and then spread to cover the whole COC slide by laying down a PTFE slide, as described above. The sandwich assembly is inverted and irradiated with UV (as above). The procedure is generally applicable for other polymers.

### EXAMPLE 15

### PREPARATION OF OLIGONUCLEOTIDE ARRAYS AND DEVICES

Spotting solutions of 20 µM amine-modified oligonucleotides in 50 mM sodium phosphate (pH 8.5) are prepared in a 384-well plate. Oligos are then spotted onto a solid support prepared above (comprising activated esters available for bioconjugation) in the desired pattern by an array spotter (Array-it SpotBot3), with an appropriate spotting pin selected for the desired spot size. Two arrays are spotted per slide at points ¼ and ¾ of the slide length, and centered in relation to the slide width. Following spotting, the slides are incubated at 75% relative humidity for 4-18 hours, then rinsed with a stream of DI water and blown dry with argon.

Spotting of oligonucleotides comprising azide or alkyne functionality is performed in an analogous manner except a solid support comprising an azide or alkyne available for conjugation is used and the spotting is performed under the appropriate click conditions (e.g., in the presence of Cu(I)).

Following drying, slides are cut in half, resulting in two 1" x 1.5" chips with the spotted array centered on each. A small single-chamber device is assembled in which the spotted slide formed the bottom. A pre-cut double-sided PSA gasket of appropriate dimensions is placed on the slide, leaving the array-spotted portion exposed along with a roughly circular area of fixed dimension around it. On top of this gasket, a polycarbonate lid with two pre-drilled filling ports is placed. The resulting assembly is laminated at room temperature in order to insure proper adhesion during thermocycling.

Multiplex PCR solutions comprising primer and probe mix, buffer, enzyme, and target DNA are premixed in a tube and then added to the chamber described above. Typical reaction chamber volumes are 25-40 µL. Following addition of the PCR reaction solution the ports in the ports in the polycarbonate lid of the chip are sealed with an optically clear film.

Devices filled with PCR reaction solutions are tested in a custom thermocycling apparatus, which allows for imaging of the surface with a digital camera though an epifluoresence microscope during the course of thermocycling. Typical hybridization times for cleaved fluorescent DNA-flaps (and for full probes) is less than 2 minutes when cooled below their hybridization temperatures (Tₘ). Surfaces are characterized by measuring the fluorescence intensity of the cleaved flaps (or full probes) that hybridize to the capture probe array. In this manner, surface stability is measured in buffer under typical thermocycling conditions. PCR in the device is also conducted, with a run typically comprising activation at 95°C for the desired time, 40 cycles of thermocycling from 95°C to 60°C, with 15 sec. dwell time at 95°C and 60 sec. dwell time at 60°C. At certain, chosen cycles, the chamber is chilled below the Tₘ of the probes, allowing for hybridization following the 60°C extension step.

Automated image analysis software is utilized to locate the arrayed spots and to quantitate the signal by measuring pixel intensity. The average pixel intensity outside the actual spot area is subtracted from the average pixel intensity inside the spot, resulting in a background-subtracted pixel intensity for the spot regions. These intensities are monitored over the course of thermocycling for the detection of cleaved DNA-flaps specific to the capture probes.

All of the U.S. patents, U.S. patent application publications, U.S. patent applications, foreign patents, foreign patent applications and non-patent publications referred to in this specification and/or attached Application Data Sheet are incorporated herein by reference, including U.S. Application No. 61/713,329, filed October 12, 2012, in their entirety to the extent not inconsistent with the present description.

From the foregoing it will be appreciated that, although specific embodiments of the invention have been described herein for purposes of illustration, various modifications may be made without deviating from the spirit and scope of the invention. Accordingly, the invention is not limited except as by the appended claims.

The present invention provides in embodiments:
1. A solid support comprising a polymer immobilized to an outer surface of a solid substrate, wherein the polymer comprises B, D and E subunits, wherein:
   the B subunit, at each occurrence, independently comprises a hydrophilic functional group;
   the D subunit, at each occurrence, independently comprises a reactive group having a reactivity specific for covalent conjugation to a capture probe or the D subunit comprises a covalent bond to a capture probe; and
   the E subunit, at each occurrence, independently subunit comprises a reaction product of two complementary click functional groups, wherein the reaction product comprises a covalent bond to either the outer surface of the solid substrate or to an optional linker (L⁴) between the E subunit and the outer surface of the solid substrate.
2. The solid support of embodiment 1, wherein the polymer has the following structure (VI):

   T³-(B)_{q}(D)ᵣ(E)ₛ-T⁴ (VI)

   wherein:
   T³ and T⁴ are each independently absent or polymer terminal groups selected from H, alkyl and an initiator residue; and
   q, r and s are each independently an integer from 1 to 350,000.
3. The solid support of any of embodiments 1 or 2, wherein the reaction product can be formed by reaction of an alkyne, amine, alkylsilyl-protected alkyne, azide, nitrile, thiol, alkene, maleimide, epoxide, aziridine or thiirane functional group with a complementary click reactive group.
4. The solid support of any of embodiments 1-3, wherein at least one E subunit is at a terminal position in the polymer.
5. The solid support of any of embodiments 1-4, wherein the E subunit comprises, at each occurrence, independently the following structure (VI): 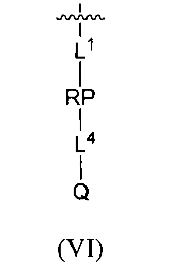 wherein:
   L¹ is an optional linker up to 100 atoms in length;
   RP is the reaction product;
   L⁴ is an optional linker up to 100 atoms in length; and
   Q represents the outer surface of the solid substrate.
6. The solid support of any of embodiments 1-5, wherein the E subunit has, at each occurrence, independently the following structure (VII): 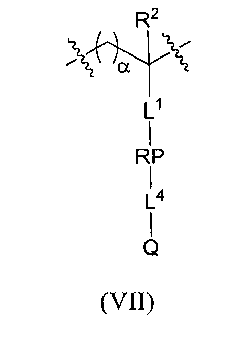 wherein:
   L¹ is an optional linker up to 100 atoms in length;
   RP is the reaction product;
   L⁴ is an optional linker up to 100 atoms in length;
   R² is H or alkyl; and
   Q represents the outer surface of the solid substrate.
7. The solid support of any of embodiments 5 or 6, wherein L¹ comprises alkylene, ester, ether or dithio moieties, or combinations thereof.
8. The solid support of any of embodiments 5 or 6, wherein L¹ is absent.
9. The solid support of any of embodiments 5-8, wherein α is 1.
10. The solid support of any of embodiments 5-9, wherein R² is H.
11. The solid support of any of embodiments 1-10, wherein L⁴ comprises a silicon-oxygen bond, an alkylene chain, a polymer or combinations thereof.
12. The solid support of embodiment 11, wherein the polymer is polyethylene glycol.
13. The solid support of embodiment 12, wherein the polyethylene glycol comprises from 1 to 50,000 monomer subunits.
14. The solid support of embodiment 13, wherein the polyethylene glycol comprises from 55 to 90 monomer subunits.
15. The solid support of any of embodiments 1-14, wherein L⁴ has one of the following structures: 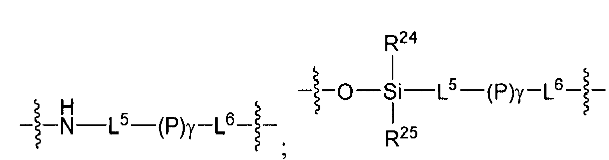 or 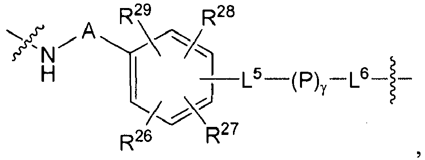 wherein:
   L⁵ and L⁶ are each independently optional linkers comprising alkylene, alkylene oxide, imide, ether, ester or amide moieties, or combinations thereof;
   R²⁴ and R²⁵ are each independently H, hydroxyl, alkyl, alkoxy or -OQ, wherein Q is the outer surface of the solid substrate;
   R²⁶, R²⁷, R²⁸ and R²⁹ are each independently, H, alkyl, halo, nitrile, nitro or ammonium;
   P represents a polymer subunit;
   A is a direct bond or -S(O)₂-; and
   γ is an integer ranging from 1 to 2000,
   where L⁴ is bound to the solid substrate via the terminal nitrogen or oxygen atom.
16. The solid support of embodiment 15, wherein P is -CH₂- or -OCH₂CH₂-.
17. The solid support of embodiment 15, wherein L⁴ has one of the following structures: 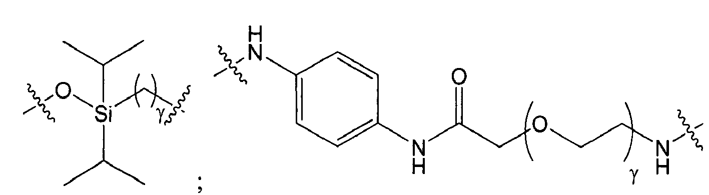 or 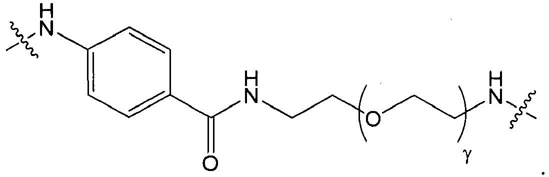
18. The solid support of embodiment 17, wherein γ ranges from 1 to 90.
19. The solid support of any of embodiments 1-10, wherein L⁴ is absent.
20. The solid support of any of embodiment 1-19, wherein the reaction product is a triazole.
21. The solid support of any of embodiments 1-20, wherein the E subunit has, at each occurrence, independently one of the following structures: 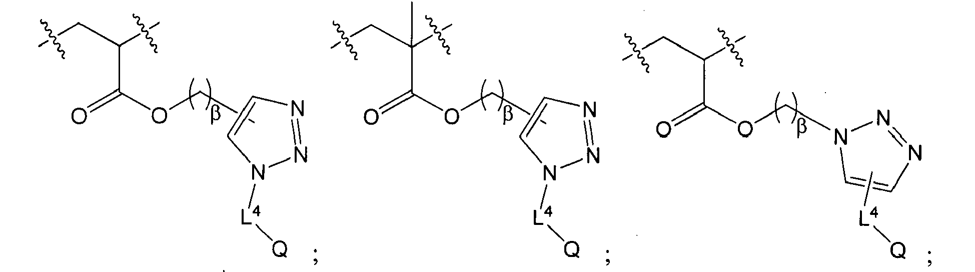 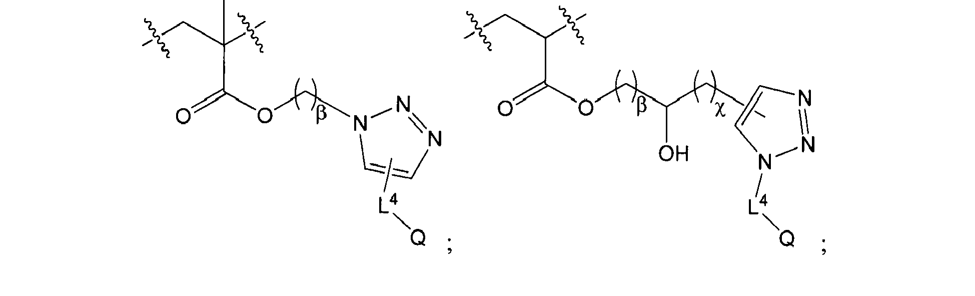 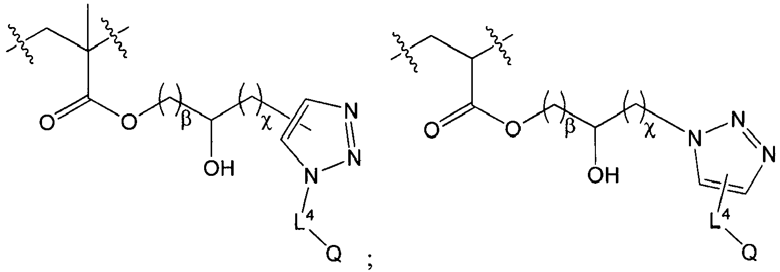 or 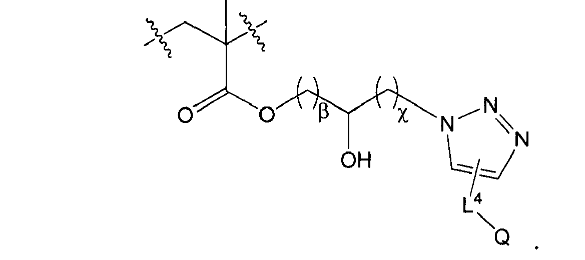 wherein:
   β and χ are each independently integers ranging from 1 to 5;
   L⁴ is an optional linker; and
   Q represents the solid substrate.
22. The solid support of any of embodiments 1-21, wherein at least one E subunit is at a terminal position covalently bound to T⁴.
23. The solid support of embodiment 22, wherein T⁴ is H.
24. The solid support of embodiment 21, wherein L⁴ comprises one or more polyethylene glycol repeating units.
25. The solid support of any of embodiments of embodiments 1-24, wherein the B subunit has, at each occurrence, independently the following structure (V): 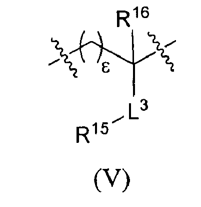 wherein:
   R¹⁵ is the hydrophilic functional group;
   R¹⁶ is hydrogen or alkyl
   L³ is an optional linker up to 100 atoms in length; and
   ε is an integer ranging from 0 to 10.
26. The solid support of embodiment 25, wherein L³ comprises alkylene, ester, alkylene oxide, amide, imide, ether or dithio moieties, or combinations thereof.
27. The solid support of embodiment 25, wherein L³ is absent.
28. The solid support of any of embodiments 25-27, wherein ε is 1.
29. The solid support of any of embodiments 25-28, wherein R¹⁶ is H.
30. The solid support of any one of embodiments 1-29, wherein R¹⁵ has, at each occurrence, independently one of the following structures: 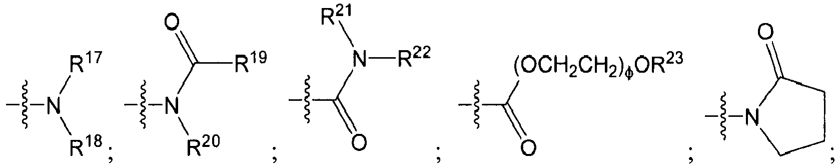 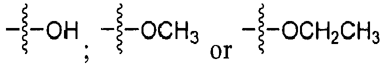 wherein:
   R¹⁷, R¹⁸, R¹⁹, R²⁰, R²¹, R²² and R²³ are each independently H, alkyl or hydroxyl alkyl; and
   φ is an integer ranging from 1 to 200.
31. The solid support of embodiment 30, wherein R¹⁷, R¹⁸, R¹⁹, R²⁰, R²¹, R²² and R²³ are each independently H or methyl.
32. The solid support of any one of embodiments 1-31, wherein at least one R¹⁵ has the following structure: 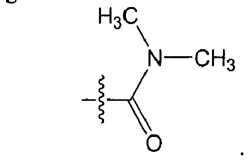
33. The solid support of any one of embodiment 1-32, wherein the B subunit has, at each occurrence, independently one of the following structures: 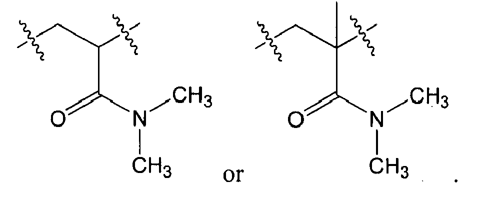
34. The solid support of any of embodiments of embodiments 1-33, wherein the D subunit has, at each occurrence, independently the following structure (IV): 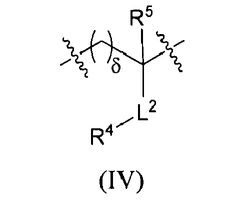 wherein:
   R⁴ is the second reactive group;
   R⁵ is hydrogen or alkyl
   L² is an optional linker up to 100 atoms in length; and
   δ is an integer ranging from 0 to 10.
35. The solid support of embodiment 34, wherein L² comprises alkylene, ester, alkylene oxide, amide, imide, ether or dithio moieties, or combinations thereof.
36. The solid support of embodiment 34, wherein L² is absent.
37. The solid support of any one of embodiments 34-36, wherein δ is 1.
38. The solid support of any one of embodiments 34-37, wherein R⁵ is H.
39. The solid support of any one of embodiments 1-38, wherein the reactive group has, at each occurrence, independently a reactivity specific for covalent bond formation with an amine group in the capture probe.
40. The solid support of any one of embodiments 1-39, wherein the reactive group is, at each occurrence, independently a N-hydroxysuccinimide (NHS) ester, N-hydroxysulfosuccinimide (sulfo-NHS) ester, succinimidyl acetylthioacetate (SATA), carbodiimide, hydroxymethyl phosphine, maleimide, arylester, imidoester, isocyanate, psoralen, vinyl sulfone, pyridyl disulfide, azlactone or benzophenone.
41. The solid support of embodiment 40, wherein the reactive group is, at each occurrence, independently a NHS ester, azlactone or arylester.
42. The solid support 41, wherein the reactive group has, at each occurrence, independently one of the following structures: 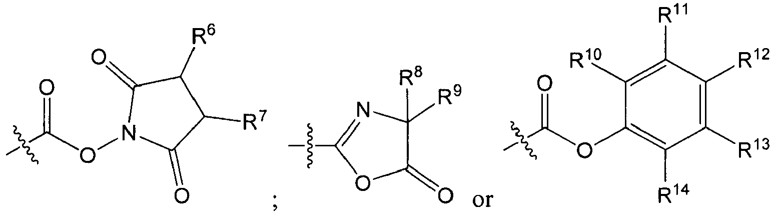 wherein:
   R⁶, R⁷, R⁸ and R⁹ are each independently H or alkyl; and
   R¹⁰, R¹¹, R¹², R¹³ and R¹⁴ are each independently, H, an electron withdrawing group, -NCS, -NCO, -CO₂H, -SO₃H, -L'-poly or salts thereof, wherein L' is an optional linker up to 100 atoms in length and poly is a water soluble polymer.
43. The solid support of embodiment 42, wherein each of R⁶, R⁷, R⁸ and R⁹ are H.
44. The solid support of embodiment 43, wherein at least one of R¹⁰, R¹¹, R¹², R¹³ or R¹⁴ is an electron withdrawing group.
45. The solid support of embodiment 44, wherein each of R¹⁰, R¹¹, R¹², R¹³ or R¹⁴ is an electron withdrawing group.
46. The solid support of any one of embodiments 44-45, wherein the electron withdrawing group is halogen, nitro or nitrile.
47. The solid support of embodiment 46, wherein the electron withdrawing group is fluoro.
48. The solid support of embodiment 47, wherein the second reactive group has the following structure: 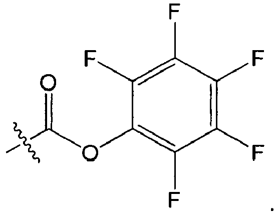
49. The solid support of any one of embodiments 1-48, wherein the D subunit has, at each occurrence, independently one of the following structures: 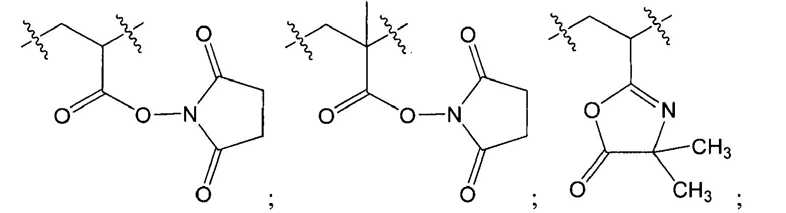 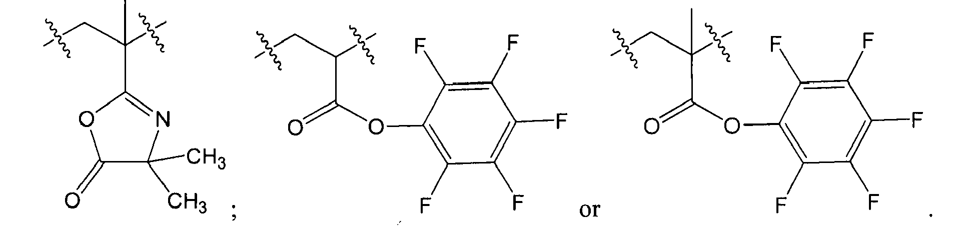
50. The solid support of embodiment 49, wherein the D subunit has, at each occurrence, independently one of the following structures: 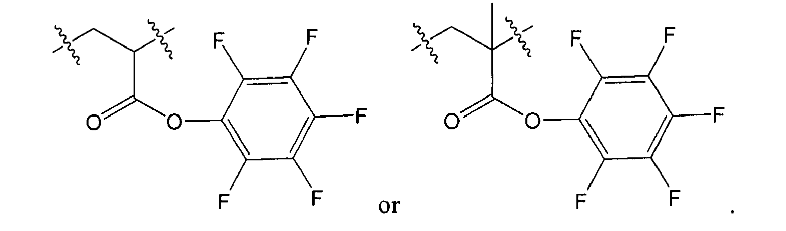
51. The solid support of embodiment 42, wherein poly is polyethylene glycol, polyacrylamide, poly(dimethylacrylamide), poly(acrylic acid), polyvinyl alcohol, polyvinyl pyrrolidone, poly(methyl vinyl ether), poly(ethyl vinyl ether), poly(*N*-vinylformamide), (poyl(*N*-vinyl acetamide) or (poly(*N*-methyl-*N-*vinylacetamide).
52. The solid support of any of any one of embodiments 1-51, wherein the D subunit is at a terminal position covalently bound to T².
53. The solid support of embodiment 52, wherein T² is H.
54. The solid support of any one of embodiments 1-33, wherein the D subunit comprises a covalent bond to a capture probe.
55. The solid support of embodiment 54, wherein the D subunit has, at each occurrence, independently the following structure (VIII): 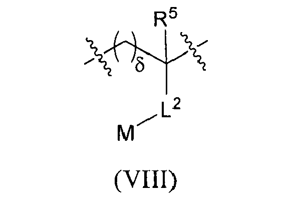 wherein:
   M is the capture probe;
   R⁵ is hydrogen or alkyl
   L² is an optional linker up to 100 atoms in length; and
   δ is an integer ranging from 0 to 10.
56. The solid support of embodiment 55, wherein L² comprises alkylene, ester, carbonyl, alkylene oxide, amide, imide ether or dithio moieties, or combinations thereof.
57. The solid support of any of embodiments 55 or 56, wherein δ is 1.
58. The solid support of any of embodiments 55-57, wherein R⁵ is H.
59. The solid support of any of embodiments 57-58, wherein the D subunit has, at each occurrence, independently the following structure: 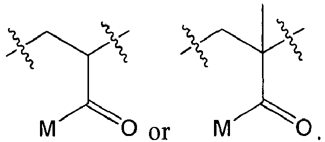
60. The solid support of any one of embodiments 1-59, wherein a surface of the solid support has one of the following structures: 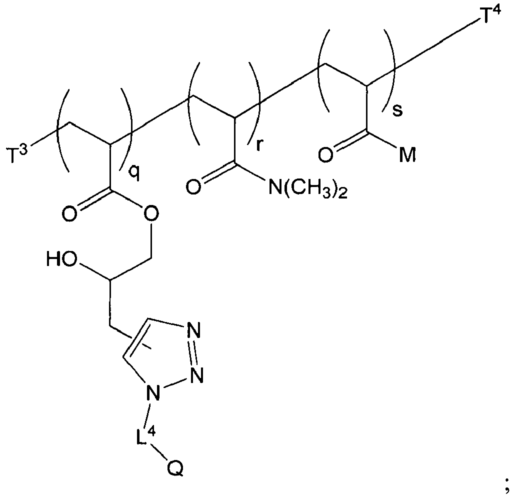 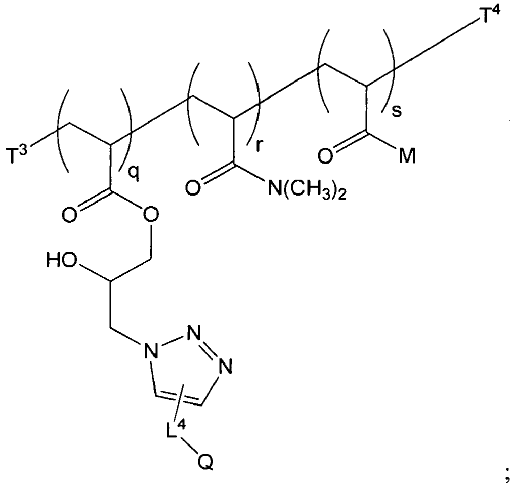 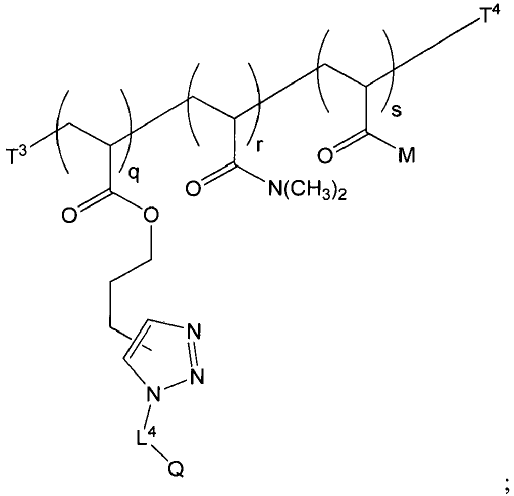 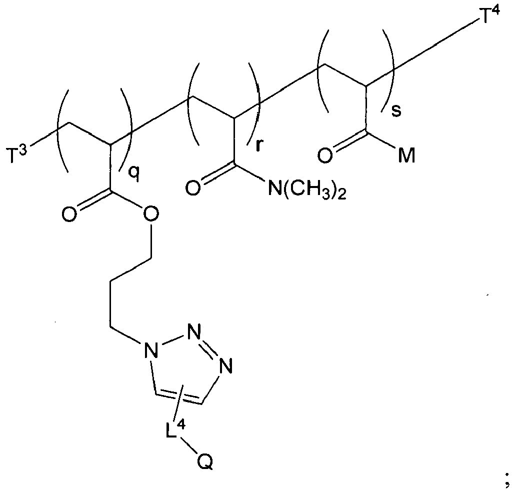 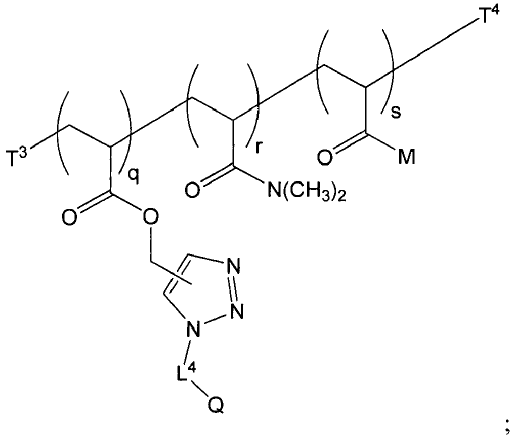 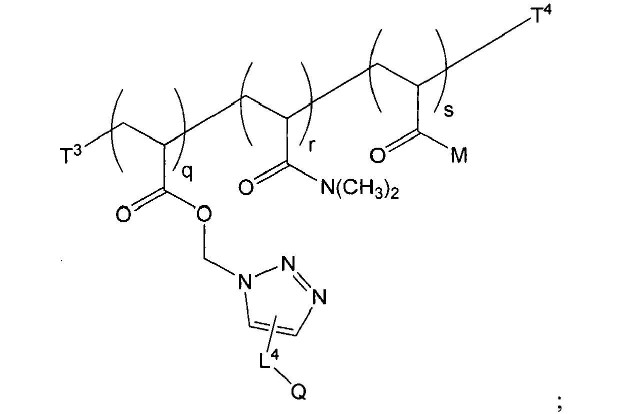 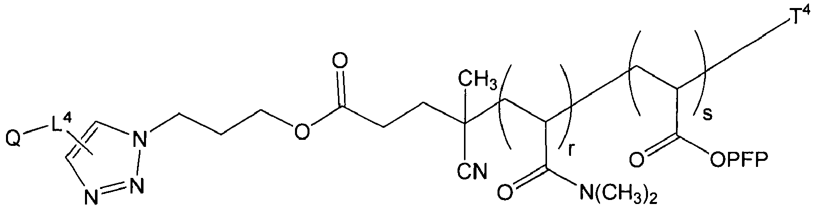 or 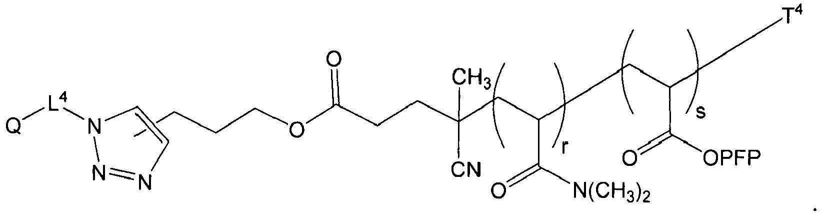 wherein:
   the B, D and E subunits are present at least once in the polymer;
   T³ and T⁴ are each independently absent or polymer terminal groups selected from H, alkyl and an initiator residue;
   q, r and s are each independently an integer from 1 to 350,000;
   L⁴ is an optional linker;
   M represents the capture probe; and
   Q represents the outer surface of the solid substrate.
61. The solid support of embodiment 60, wherein L⁴ is as defined in any of embodiments 11-18.
62. The solid support of any of embodiments 54-61, wherein the capture probe is covalently bound to the D subunit via a nitrogen atom.
63. The solid support of any one of embodiments 54-62, wherein the capture probe is a peptide, protein, carbohydrate, polynucleotide, oligonucleotide or polypeptide.
64. The solid support of embodiment 63, wherein the capture probe is a polynucleotide.
65. The solid support of embodiment 64, wherein the capture probe is DNA.
66. The solid support of any one of embodiments 1-65, wherein the solid support has a water contact angle ranging from 50° to 90°.
67. The solid support of any of embodiments 1-66, wherein the solid support comprises an organic polymer.
68. The solid support of embodiment 67, wherein the solid substrate comprises poly(styrene), poly(carbonate), poly(ethersulfone), poly(ketone), poly(aliphatic ether), poly(aryl ether), poly(amide) poly(imide), poly(ester) poly(acrylate), poly(methacrylate), poly(olefin), poly(cyclic olefin), poly(vinyl alcohol) or copolymers, halogenated derivatives or crosslinked derivatives thereof.
69. The solid support of embodiment 68, wherein the halogenated derivatives are halogenated poly(aryl ether), halogenated poly(olefin) or halogenated poly(cyclic olefin).
70. The solid support of embodiment 68, wherein the solid substrate comprises a cyclic poly(olefin).
71. The solid support of any of embodiment 1-66, wherein the solid substrate comprises an oxide.
72. The solid support of embodiment 71, wherein the solid substrate comprises silicon, fused silica, glass, quartz, indium-tin oxide, titanium dioxide, aluminum oxide or combinations thereof.
73. The solid support of any one of embodiments 1-66, wherein the solid substrate comprises an organic polymer, wherein the solid support comprises an outer surface having an oxide layer adhered thereto.
74. The solid support of any one of embodiment 1-73, wherein the solid substrate is substantially optically transparent.
75. The solid support of any one of embodiments 1-74, wherein the solid substrate is substantially optically transparent between about 400 nm and about 800 nm.
76. The solid support of any one of embodiments 1-75, wherein the solid substrate is at least about 90% optically transparent.
77. The solid support of any one of embodiments 1-76, wherein the solid support comprises a systematic array of distinct locations, each distinct location independently comprising at least one of the polymers conjugated thereto.
78. The solid support of embodiment 77, wherein each distinct location independently comprises a plurality of the polymers conjugated thereto.
79. The solid support of any one of embodiments 77-78, wherein at least one polymer at each distinct location comprises a capture probe covalently bound thereto via a D subunit.
80. The solid support any one of embodiments 77-79, wherein each distinct location comprises at least one capture probe bound thereto, wherein the capture probe is structurally distinct from at least one capture probe bound at each of the other distinct locations.
81. The solid support of embodiment 77, wherein each distinct location comprises a plurality of structurally distinct analyte molecules bound thereto.
82. The solid support of any of embodiments 77-80, wherein the capture probe is a peptide, protein, carbohydrate, polynucleotide, oligonucleotide, oligopeptide or polypeptide.
83. The solid support of embodiment 82, wherein the capture probe is a polynucleotide.
84. The solid support of embodiment 83, wherein the capture probe is DNA.
85. The solid support of any one of embodiments 83 or 84, wherein the polynucleotide or DNA comprises a sequence complementary to a sequence of a target polynucleotide or DNA.
86. A polymer comprising A, B and C subunits, wherein:
   the A subunit, at each occurrence, independently comprises a first reactive group having a reactivity specific for covalent bond formation with a target functional group on a solid substrate;
   the B subunit, at each occurrence, independently comprises a hydrophilic functional group; and
   the C subunit, at each occurrence, independently comprises a second reactive group having a reactivity specific for covalent conjugation to a capture probe,
   wherein the reactivity of the first reactive group and the second reactive group are orthogonal to each other.
87. The polymer of embodiment 86, wherein the polymer has the following structure (I):

   T¹-(A)ₓ(B)_{y}(C)_{z}-T² (I)

   wherein:
   T¹ and T² are each independently absent or polymer terminal groups selected from H, alkyl and an initiator residue; and
   x, y and z are independently an integer from 1 to 350,000.
88. The polymer of embodiments 86 or 87, wherein the polymer comprises an A subunit at a terminal position of the polymer.
89. The polymer of any one of embodiments 86-88, wherein the first reactive group has, at each occurrence, independently click reactivity.
90. The polymer of embodiment 89, wherein the first reactive group is, at each occurrence, independently specific for reaction with an azide.
91. The polymer of embodiment 90, wherein the first reactive group is, at each occurrence, independently an alkyne.
92. The polymer of embodiment 89, wherein the first reactive group is, at each occurrence, independently specific for reaction with an alkyne.
93. The polymer of embodiment 92, wherein the first reactive group is, at each occurrence, independently an azide.
94. The polymer of any one of embodiments 86-93, wherein the A subunit has, at each occurrence, independently the following structure (II): 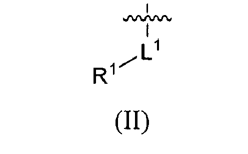 wherein:
   R¹ is the first reactive group; and
   L¹ is an optional linker up to 100 atoms in length.
95. The polymer of any one of embodiments 86-94, wherein the A subunit has, at each occurrence, independently the following structure (III): 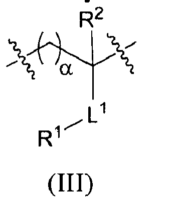 wherein:
   R¹ is the first reactive group;
   R² is hydrogen or alkyl
   L¹ is an optional linker up to 100 atoms in length; and
   α is an integer ranging from 0 to 10.
96. The polymer of any one of embodiments 94 or 95, wherein L¹ comprises alkylene, ester, alkylene oxide, amide, imide, ether or dithio moieties, or combinations thereof.
97. The polymer of any one of embodiments 94 or 95, wherein L¹ is absent.
98. The polymer of any one of embodiments 95-97, wherein α is 1.
99. The polymer of any of embodiments 95-98, wherein R² is H.
100. The polymer of any one of embodiments 94-99, wherein R¹ is an alkyne, alkylsilyl-protected alkyne, azide, nitrile, thiol, alkene, maleimide, epoxide, aziridine or thiirane functional group.
101. The polymer of any one of embodiments 94-99, wherein R¹ is an alkyne or azide functional group.
102. The polymer of any one of embodiments 86-101, wherein the A subunit has, at each occurrence, independently one of the following structures: 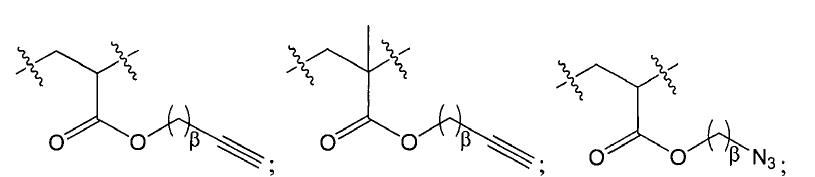 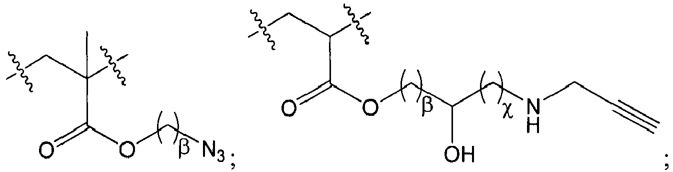 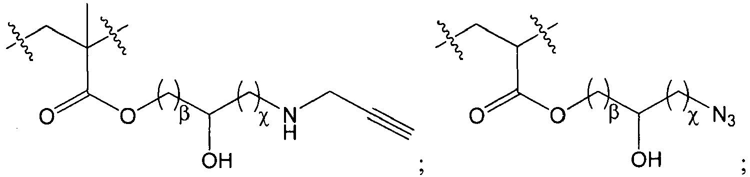 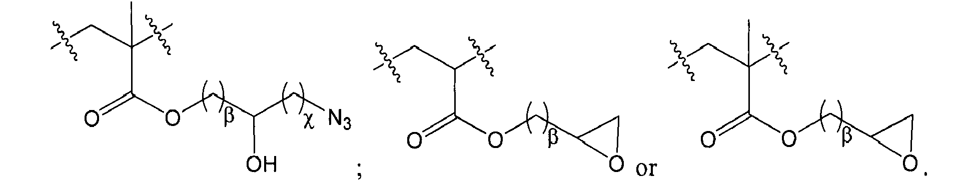 wherein β and χ are each independently integers ranging from 1 to 5.
103. The polymer of embodiment 102, wherein β is 1 or 3.
104. The polymer of embodiment 102, wherein χ is 1.
105. The polymer of embodiment 102, wherein the A subunit has, at each occurrence, independently one of the following structures: 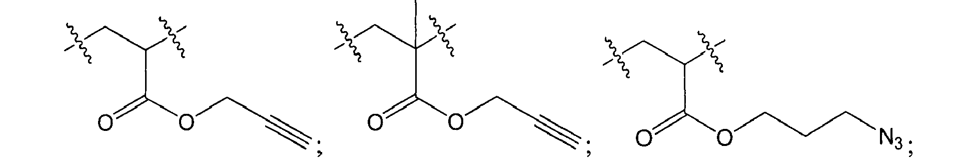 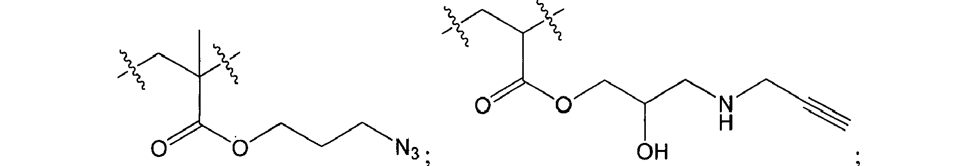 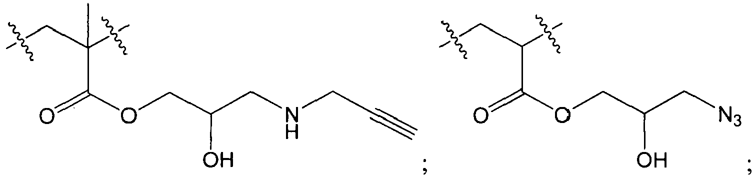 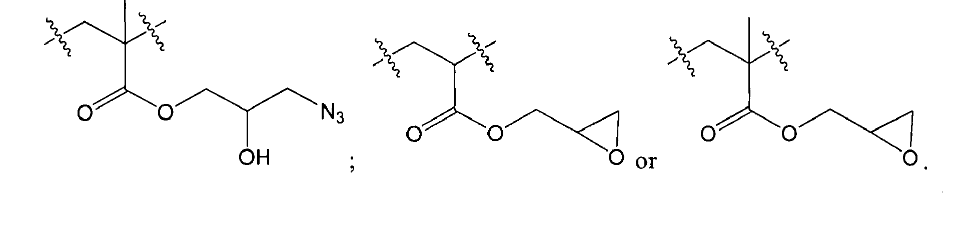
106. The polymer of any of embodiments 86-105, wherein at least one A subunit is at a terminal position covalently bound to T¹.
107. The polymer of embodiment 106, wherein T¹ is H.
108. The polymer of any one of embodiments 86-88, wherein the A subunit has, at each occurrence, independently the following structure: 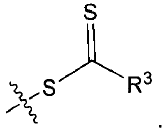 wherein:
   R³ is aryl.
109. The polymer of embodiment 108, wherein the A subunit has the following structure: 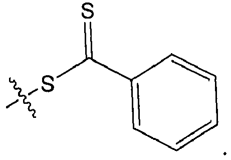
110. The polymer of any one of embodiments 86-109, wherein the C subunit has, at each occurrence, independently the following structure (IV): 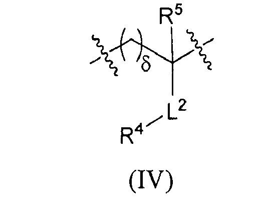 wherein:
   R⁴ is the second reactive group;
   R⁵ is hydrogen or alkyl
   L² is an optional linker up to 100 atoms in length; and
   δ is an integer ranging from 0 to 10.
111. The polymer of embodiment 110, wherein L² comprises alkylene, ester, alkylene oxide, amide, imide, ether or dithio moieties, or combinations thereof.
112. The polymer of embodiment 110, wherein L² is absent.
113. The polymer of any one of embodiments 110-112, wherein δ is 1.
114. The polymer of any one of embodiments 110-113, wherein R⁵ is H.
115. The polymer of any one of embodiments 86-114, wherein the reactivity of the second reactive group is, at each occurrence, independently specific for an amine group in the capture probe.
116. The polymer of any one of embodiments 86-115, wherein the second reactive group is, at each occurrence, independently a N-hydroxysuccinimide (NHS) ester, N-hydroxysulfosuccinimide (sulfo-NHS) ester, succinimidyl acetylthioacetate (SATA), carbodiimide, hydroxymethyl phosphine, maleimide, arylester, imidoester, isocyanate, psoralen, vinyl sulfone, pyridyl disulfide, azlactone or benzophenone.
117. The polymer of embodiment 116, wherein the second reactive group is, at each occurrence, independently a NHS ester, azlactone or arylester.
118. The polymer of embodiment 117, wherein the second reactive group has, at each occurrence, independently one of the following structures: 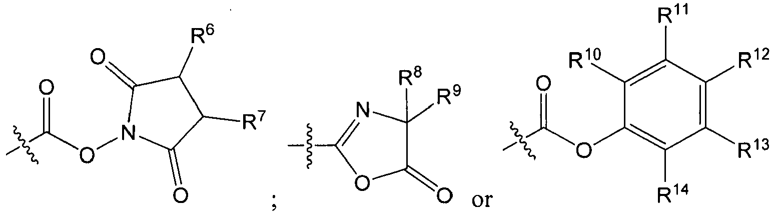 wherein:
   R⁶, R⁷, R⁸ and R⁹ are each independently H or alkyl; and
   R¹⁰, R¹¹, R¹², R¹³ and R¹⁴ are each independently, H, an electron withdrawing group, -NCS, -NCO, -CO₂H, -SO₃H, -L'-poly or salts thereof, wherein L' is an optional linker up to 100 atoms in length and poly is a water soluble polymer.
119. The polymer of embodiment 118, wherein each of R⁶, R⁷, R⁸ and R⁹ are H.
120. The polymer of any one of embodiments 118-119, wherein at least one of R¹⁰, R¹¹, R¹² R¹³ or R¹⁴ is an electron withdrawing group.
121. The polymer of embodiment 120, wherein each of R¹⁰, R¹¹, R¹² R¹³ or R¹⁴ is an electron withdrawing group.
122. The polymer of any one of embodiments 118-121, wherein the electron withdrawing group is halogen, nitro or nitrile.
123. The polymer of embodiment 122, wherein the electron withdrawing group is fluoro.
124. The polymer of embodiment 123, wherein the second reactive group has the following structure: 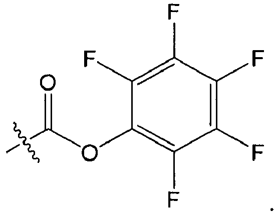
125. The polymer of any one of embodiments 86-124, wherein the C subunit has, at each occurrence, independently one of the following structures: 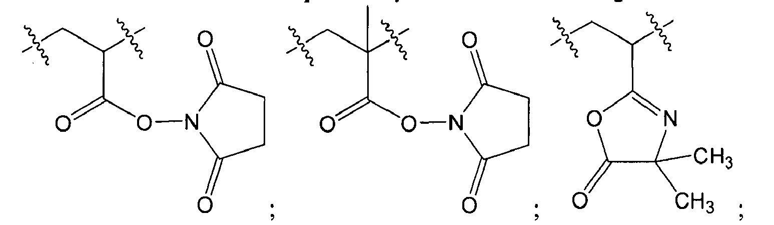 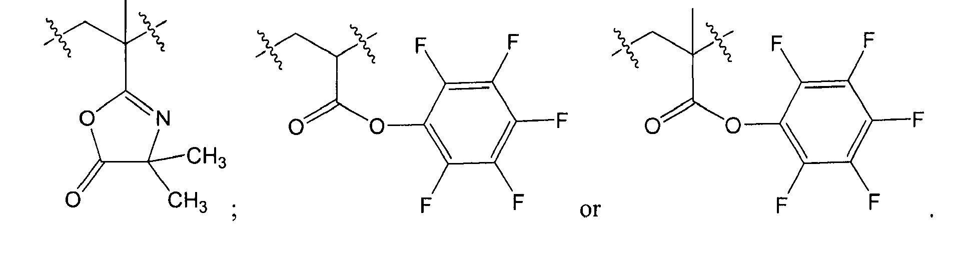
126. The polymer of embodiment 125, wherein the C subunit has, at each occurrence, independently one of the following structures: 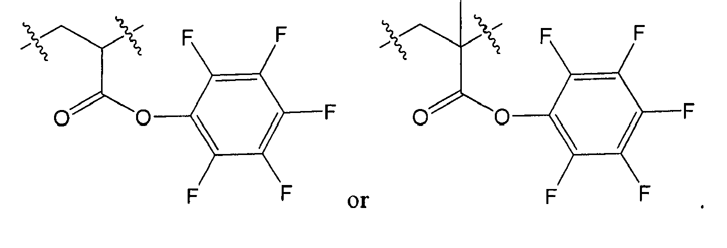
127. The polymer of embodiment 118, wherein poly is polyethylene glycol, polyacrylamide, poly(dimethylacrylamide), poly(acrylic acid), polyvinyl alcohol, polyvinyl pyrrolidone, poly(methyl vinyl ether), poly(ethyl vinyl ether), poly(*N*-vinylformamide), (poyl(*N*-vinyl acetamide) or (poly(*N*-methyl-*N-*vinylacetamide).
128. The polymer of any one of embodiments 86-127, wherein at least one C subunit is at a terminal position covalently bound to T².
129. The polymer of embodiment 128, wherein T² is H.
130. The polymer of any one of embodiments 86-127, wherein the B subunit has, at each occurrence, independently the following structure (V): 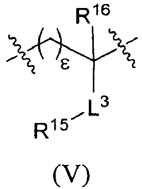 wherein:
   R¹⁵ is the hydrophilic functional group;
   R¹⁶ is hydrogen or alkyl
   L³ is an optional linker up to 100 atoms in length; and
   ε is an integer ranging from 0 to 10.
131. The polymer of embodiment 130, wherein L³ comprises alkylene, ester, alkylene oxide, amide, imide, ether or dithio moieties, or combinations thereof.
132. The polymer of embodiment 130, wherein L³ is absent.
133. The polymer of any one of embodiments 130-132, wherein ε is 1.
134. The polymer of any one of embodiments 130-133, wherein R¹⁶ is H.
135. The polymer of any one of embodiments 86-134, wherein the hydrophilic functional group has, at each occurrence, independently one of the following structures: 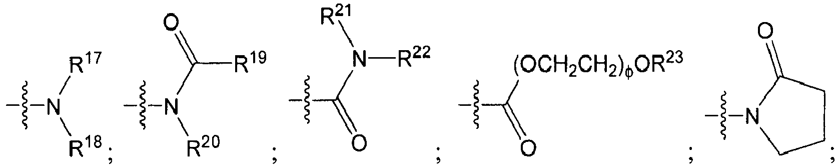 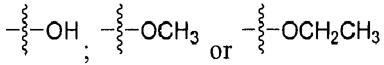 wherein:
   R¹⁷, R¹⁸, R¹⁹, R²⁰, R²¹, R²² and R²³ are each independently H, alkyl or hydroxyl alkyl; and
   φ is an integer ranging from 1 to 200.
136. The polymer of embodiment 135, wherein R¹⁷, R¹⁸, R¹⁹, R²⁰, R²¹, R²² and R²³ are each independently H or methyl.
137. The polymer of embodiment 135, wherein the hydrophilic functional group has the following structure: 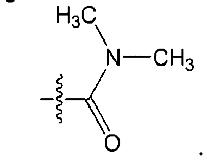
138. The polymer of any one of embodiments 86-137, wherein the B subunit has, at each occurrence, independently one of the following structures: 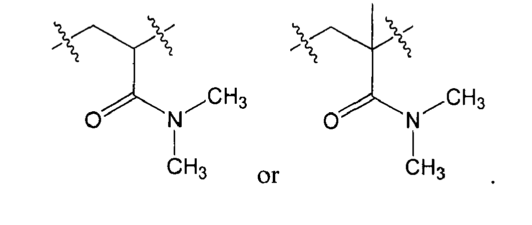
139. The polymer of embodiment 86, wherein the polymer has one of the following structures: 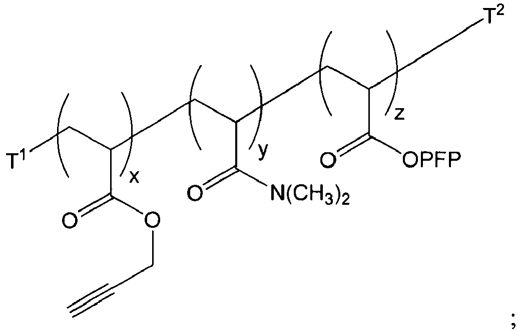 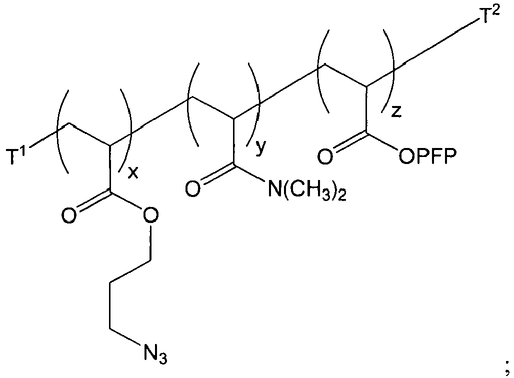 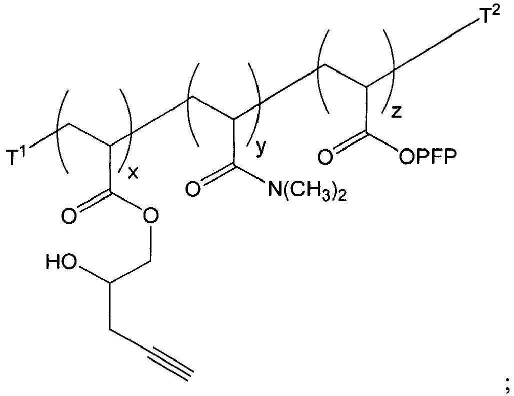 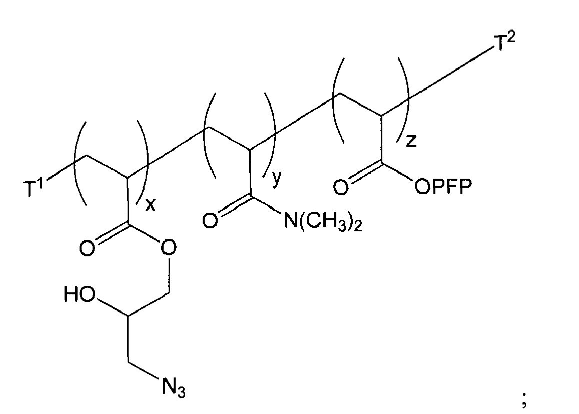 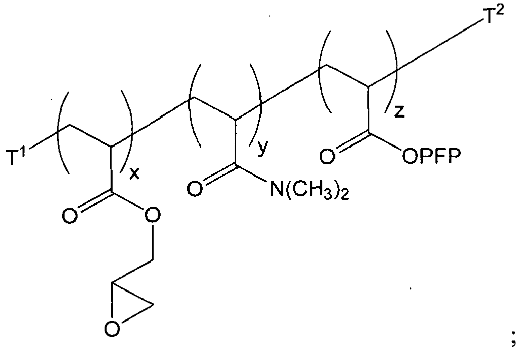 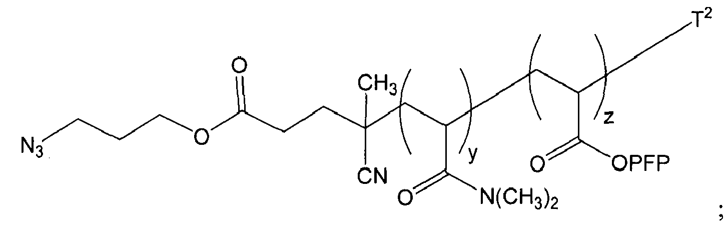 or 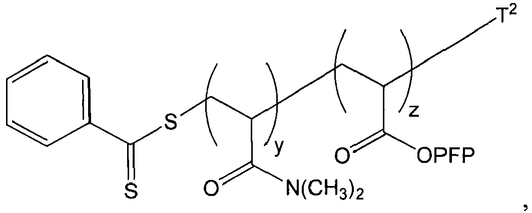 wherein:
   the A, B and C subunits are present at least once in the polymer;
   OPFP represents pentafluorophenoxy;
   T¹ and T² are each independently absent or polymer terminal groups selected from H and alkyl;
   x, y and z are each independently an integer from 1 to 350,000.
140. The polymer of any one of embodiments 86-139, wherein at least 30% of the subunits in the polymer are B subunits.
141. The polymer of any one of embodiments 86-140, wherein at least 75% of the subunits in the polymer are B subunits.
142. The polymer of any one of embodiments 86-141, wherein at least 90% of the subunits in the polymer are B subunits.
143. The polymer of any one of embodiments 86-142, wherein at least 95% of the subunits in the polymer are B subunits.
144. The polymer of any one of embodiments 86-143, wherein the polymer comprises only one A subunit.
145. The polymer of embodiment 144, wherein the A subunit is at a terminal end of the polymer.
146. The polymer of any one of embodiments 86-145, wherein the capture probe is a polynucleotide, an oligonucleotide, a peptide, a polypeptide or a carbohydrate.
147. The polymer of embodiment 146, wherein the capture probe is a polynucleotide.
148. The polymer of embodiment 147, wherein the capture probe is DNA.
149. A solid substrate comprising an outer surface, wherein the solid substrate comprises an azide or alkyne moiety covalently bound to the outer surface.
150. The solid substrate of embodiment 149, wherein the solid substrate has one of the following structures: 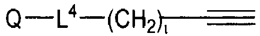 or wherein:
   Q represents the outer surface of the solid substrate;
   L⁴ is an optional linker;
   and 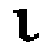 is an integer ranging from 0 to 10.
151. The solid substrate of embodiment 150, wherein L⁴ is present.
152. The solid substrate of embodiment 150, wherein L⁴ is as defined in any of embodiments 11-18.
153. The solid substrate of any of embodiments 149-152, wherein 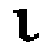 is 1,2 or 3.
154. A method for preparing a solid support having a capture probe bound thereto, the method comprising contacting the solid support of any of embodiments 149-153 with the polymer of any of embodiments 86-148 to obtain a solid support comprising a polymer bound thereto.
155. The method of embodiment 154, further comprising contacting a Cu(I) catalyst with the solid support of any of embodiments 149-153 and the polymer of any of embodiments 86-148.
156. The method of any of embodiments 154 or 155, further comprising contacting a capture probe having an amine functional group with the solid support comprising a polymer bound thereto.
157. A method for determining the presence or absence of a target analyte molecule, the method comprising:
   a) providing a solid support according to any of embodiments 1-85, wherein the D subunit comprises a capture probe covalently bound thereto;
   b) contacting an analyte probe with the solid support; and
   c) detecting the presence or absence of a signal produced from interaction of the capture probe with the analyte probe.
158. The method of embodiments 157, wherein the solid support is the solid support of any of embodiments 54-65.
159. The method of any of embodiments 157 or 158, wherein the capture probe is a polynucleotide.
160. The method of any of embodiments 157-159, wherein the target analyte molecule is a polynucleotide.
161. The method of any of embodiments 157-160, wherein the signal is a fluorescent signal.
162. The method of embodiment 161, wherein the fluorescent signal is produced as a result of specific hybridization of the analyte probe with a capture probe.
163. The method of any of embodiments 157-162, wherein the analyte probe comprises a fluorophore or a fluorophore quencher.
164. A compound having the following structure (IX): 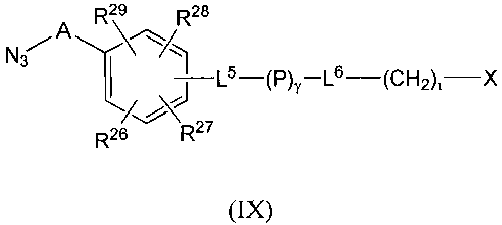 wherein:
   X is an azide or alkyne moiety;
   L⁵ and L⁶ are each independently optional linkers comprising alkylene, alkylene oxide, imide, ether, ester or amide moieties, or combinations thereof;
   R²⁶, R²⁷, R²⁸ and R²⁹ are each independently, H, alkyl, halo, nitrile, nitro or ammonium;
   P is -(OCH₂CH₂)- or -(CH₂)-;
   A is a direct bond or -S(O)₂-;
   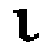 is an integer ranging from 0 to 10; and
   γ is an integer ranging from 1 to 2000.
165. The compound of embodiment 164, wherein each of R²⁶, R²⁷, R²⁸ and R²⁹ are H.
166. The compound of any of embodiments 164 or 165, wherein A is a direct bond.
167. The compound of embodiment 164, wherein the compound has one of the following structures: 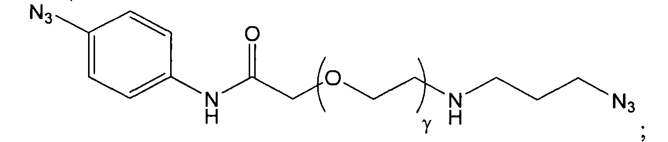 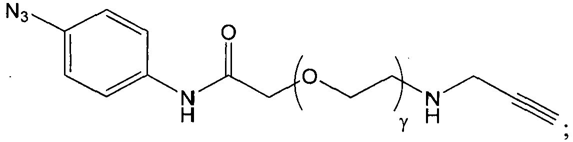 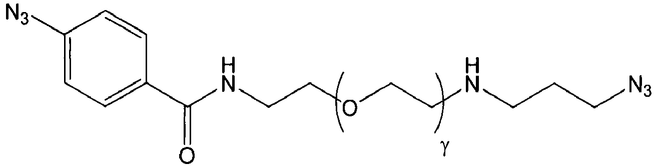 or 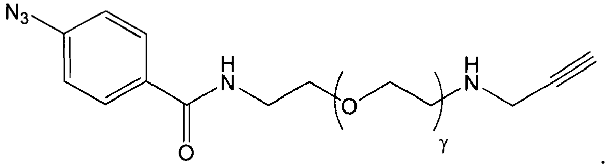
168. The compound of embodiment 167, wherein γ ranges from 1 to 90.

## Claims

1. A solid support comprising a polymer immobilized to an outer surface of a solid substrate, wherein the polymer comprises B, D and E subunits, and has the following structure:
T³-(B)q(D)ᵣ(E)ₛ-T⁴ (VI)
wherein:
the B subunit, at each occurrence, independently comprises a hydrophilic functional group;
the D subunit, at each occurrence, independently comprises a reactive group having a reactivity specific for covalent conjugation to a capture probe or the D subunit comprises a covalent bond to a capture probe;
the E subunit, at each occurrence, independently subunit comprises a reaction product of two complementary click functional groups, wherein the reaction product comprises a covalent bond to either the outer surface of the solid substrate or to an optional linker (L⁴) between the E subunit and the outer surface of the solid substrate;
T³ and T⁴ are each independently absent or polymer terminal groups selected from H, alkyl and an initiator residue; and
q, r and s are each independently an integer from 1 to 350,000,
wherein each of the B, D and E subunits may occur at any position in the polymer.

2. The solid support of claim 1, wherein the E subunit has, at each occurrence, independently the following structure (VII): 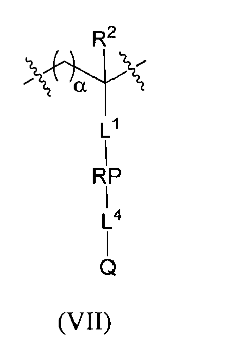 wherein:
L¹ is an optional linker up to 100 atoms in length;
RP is the reaction product;
L⁴ is an optional linker up to 100 atoms in length;
R² is H or alkyl;
α is an integer ranging from 0 to 10; and
Q represents the outer surface of the solid substrate.

3. The solid support of any of claims 1-2, wherein:
A) L⁴ comprises a silicon-oxygen bond, an alkylene chain, a polymer or combinations thereof;
B) L⁴ comprises a polymer, and the polymer is polyethylene glycol;
C) L⁴ comprises a polymer, and the polymer is polyethylene glycol comprising from 1 to 50,000 monomer subunits;
D) L⁴ comprises a polymer, and the polymer is polyethylene glycol comprising from 55 to 90 monomer subunits;
E) L⁴ has one of the following structures: 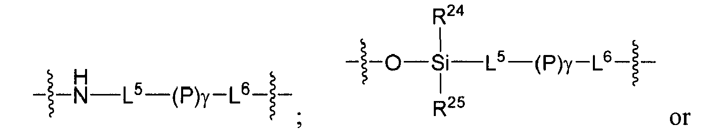 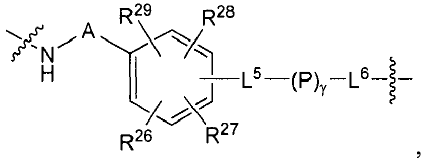 wherein:
L⁵ and L⁶ are each independently optional linkers comprising alkylene, alkylene oxide, imide, ether, ester or amide moieties, or combinations thereof;
R²⁴ and R²⁵ are each independently H, hydroxyl, alkyl, alkoxy or -OQ, wherein Q is the outer surface of the solid substrate;
R²⁶, R²⁷, R²⁸ and R²⁹ are each independently, H, alkyl, halo, nitrile, nitro or ammonium;
P represents a polymer subunit;
A is a direct bond or -S(O)₂-; and
γ is an integer ranging from 1 to 2000,
where L⁴ is bound to the solid substrate via the terminal nitrogen or oxygen atom;
F) L⁴ has one of the following structures: 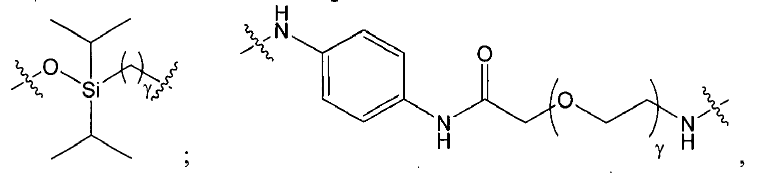 wherein γ ranges from 1 to 90; or
G) L⁴ is absent.

4. The solid support of any of claim 1-3, wherein the reaction product is a triazole; or wherein the E subunit has, at each occurrence, independently one of the following structures: 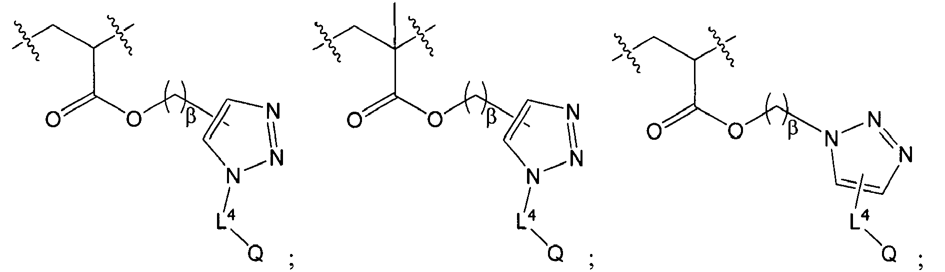 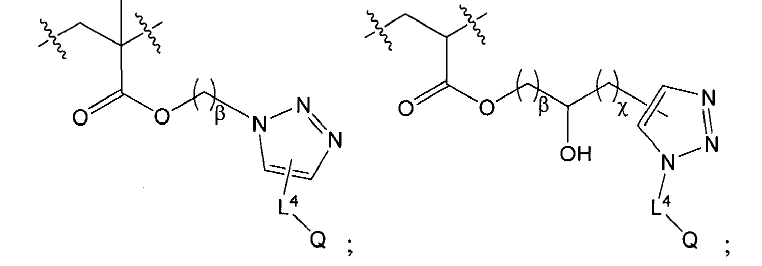 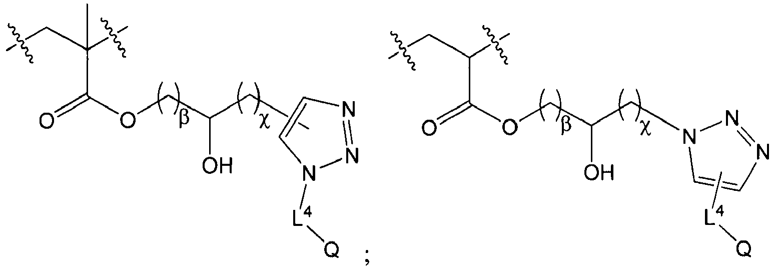 or 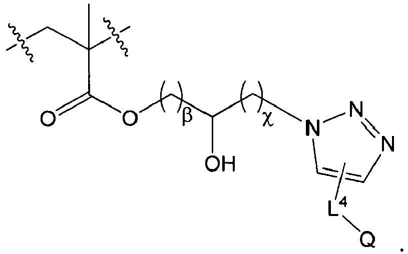 wherein:
β and χ are each independently integers ranging from 1 to 5;
L⁴ is an optional linker; and
Q represents the solid substrate.

5. The solid support of any of claims of claims 1-4, wherein the B subunit has, at each occurrence, independently the following structure (V): 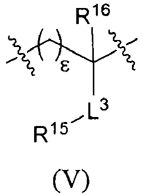 wherein:
R¹⁵ is the hydrophilic functional group;
R¹⁶ is hydrogen or alkyl
L³ is an optional linker up to 100 atoms in length; and
ε is an integer ranging from 0 to 10.

6. The solid support of any of claims of claims 1-5, wherein the D subunit has, at each occurrence, independently the following structure (IV): 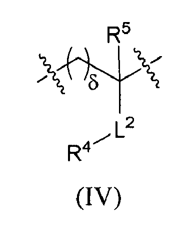 wherein:
R⁴ is the second reactive group;
R⁵ is hydrogen or alkyl
L² is an optional linker up to 100 atoms in length; and
δ is an integer ranging from 0 to 10.

7. The solid support of any one of claims 1-6, wherein the reactive group has, at each occurrence, independently one of the following structures: 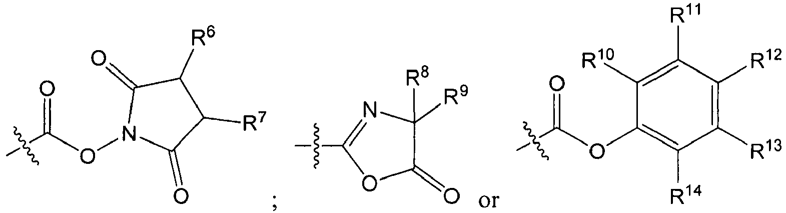 wherein:
R⁶, R⁷, R⁸ and R⁹ are each independently H or alkyl; and
R¹⁰, R¹¹, R¹², R¹³ and R¹⁴ are each independently, H, an electron withdrawing group, -NCS, -NCO, -CO₂H, -SO₃H, -L'-poly or salts thereof, wherein L' is an optional linker up to 100 atoms in length and poly is a water soluble polymer.

8. The solid support of any one of claims 1-7, wherein the D subunit has, at each occurrence, independently the following structure (VIII): 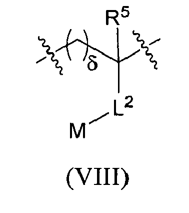 wherein:
M is a capture probe;
R⁵ is hydrogen or alkyl
L² is an optional linker up to 100 atoms in length; and
δ is an integer ranging from 0 to 10.

9. The solid support of any one of claims 1-8, wherein a surface of the solid support has one of the following structures: 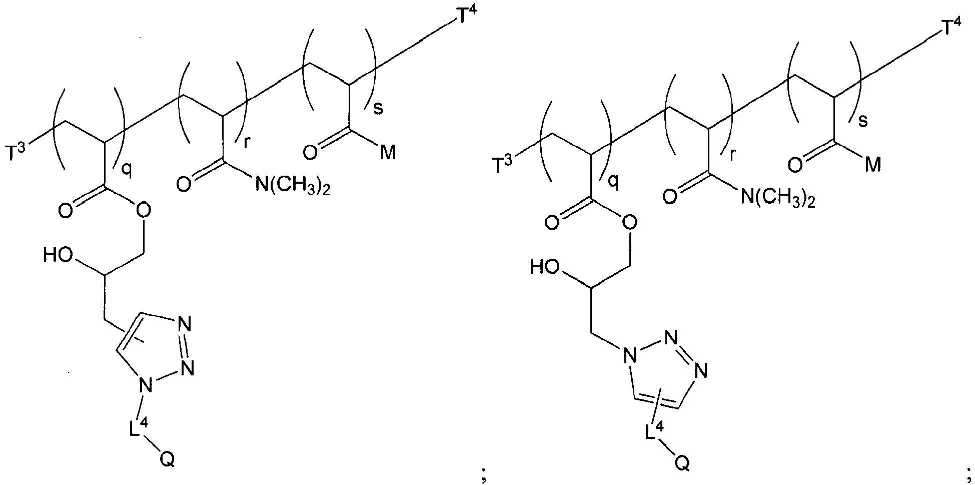 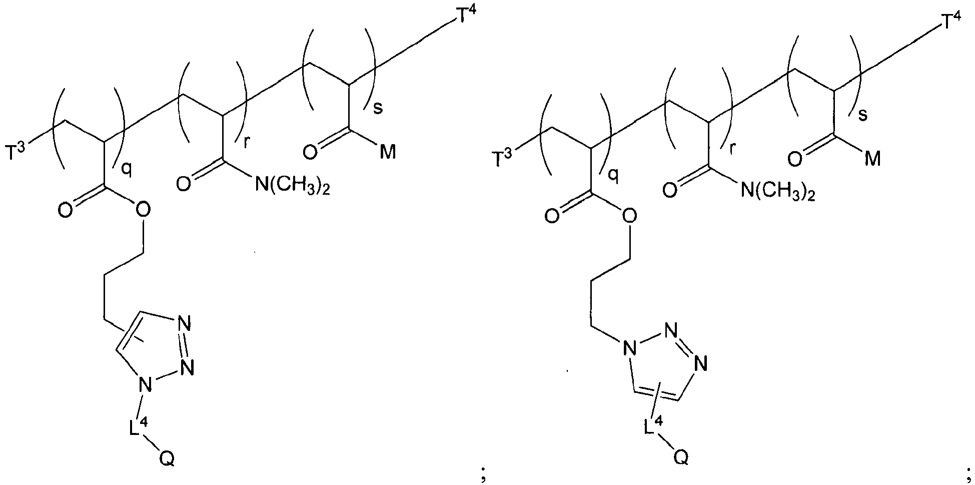 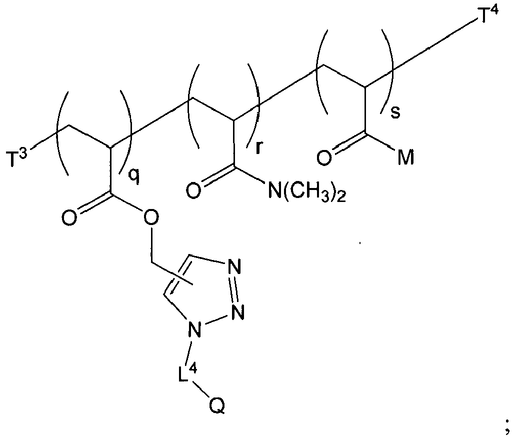 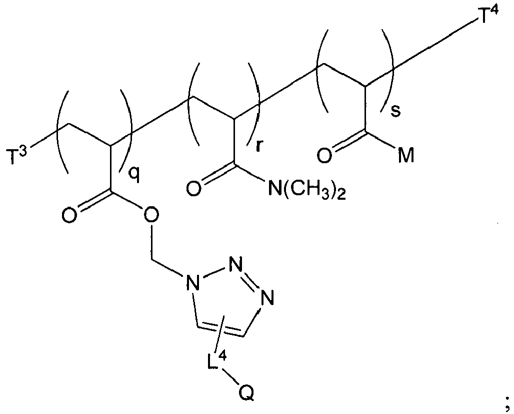 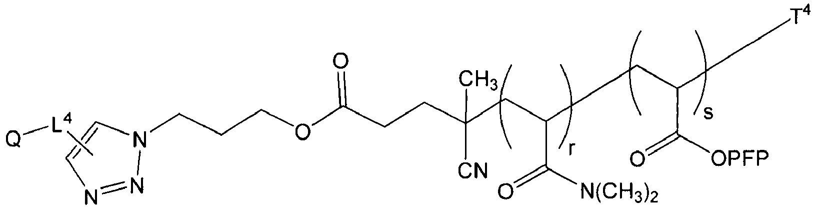 or 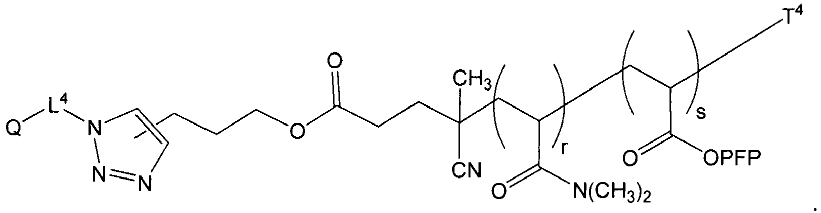 wherein:
the B, D and E subunits are present at least once in the polymer;
T³ and T⁴ are each independently absent or polymer terminal groups selected from H, alkyl and an initiator residue;
q, r and s are each independently an integer from 1 to 350,000;
L⁴ is an optional linker;
M represents the capture probe; and
Q represents the outer surface of the solid substrate.

10. A polymer comprising A, B and C subunits, and having the following structure (I):
T¹-(A)ₓ(B)y(C)_{z}-T² (I)
wherein:
the A subunit, at each occurrence, independently comprises a first reactive group having a reactivity specific for covalent bond formation with a target functional group on a solid substrate;
the B subunit, at each occurrence, independently comprises a hydrophilic functional group;
the C subunit, at each occurrence, independently comprises a second reactive group having a reactivity specific for covalent conjugation to a capture probe;
T¹ and T² are each independently absent or polymer terminal groups selected from H, alkyl and an initiator residue;
x, y and z are independently an integer from 1 to 350,000
wherein the reactivity of the first reactive group and the second reactive group are orthogonal to each other, and each of the A, B and C subunits may occur at any position in the polymer.

11. The polymer of claim 10, wherein:
A) the A subunit has, at each occurrence, independently the following structure (III): 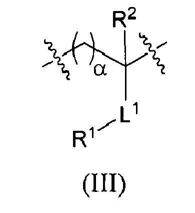 wherein:
R¹ is the first reactive group;
R² is hydrogen or alkyl
L¹ is an optional linker up to 100 atoms in length; and
α is an integer ranging from 0 to 10;
B) the A subunit has, at each occurrence, independently one of the following structures: 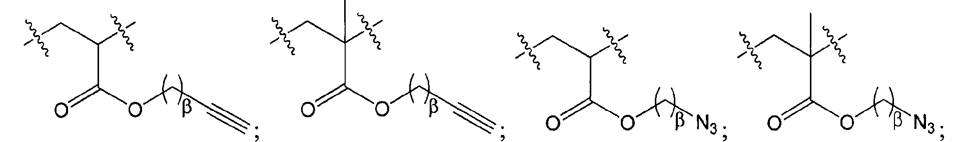 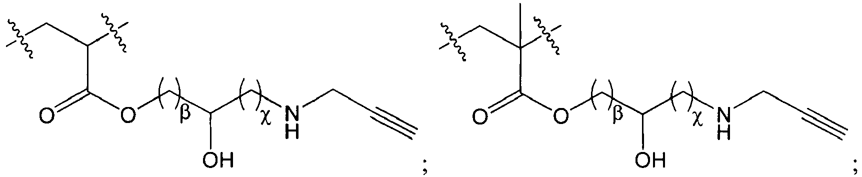 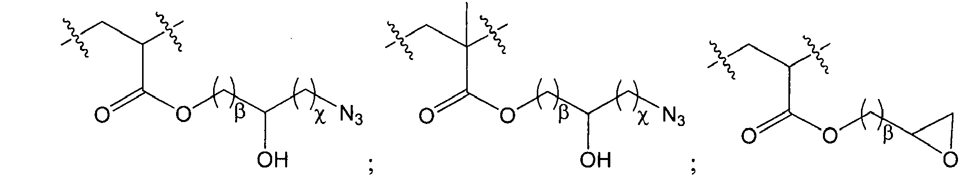 or 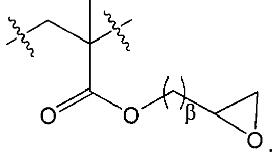 wherein β and χ are each independently integers ranging from 1 to 5; or
C) the A subunit has, at each occurrence, independently one of the following structures:

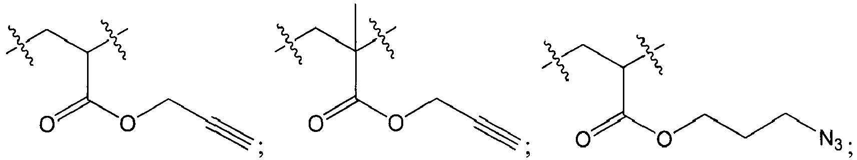

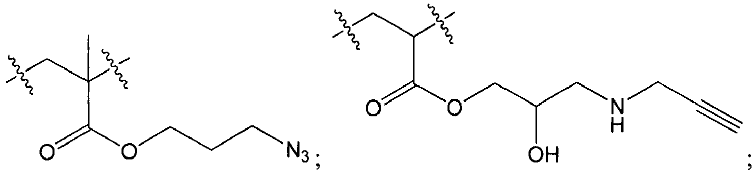

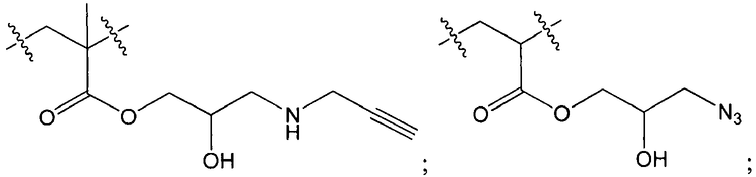

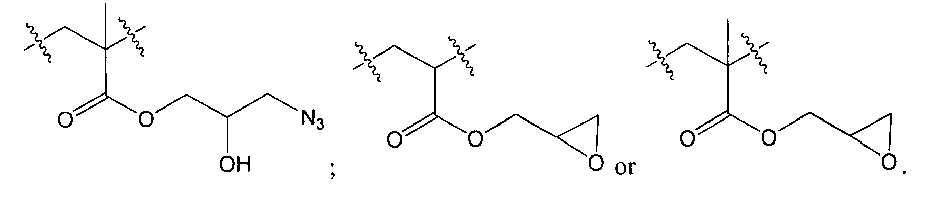

12. The polymer of any one of claims 10-11, wherein the C subunit has, at each occurrence, independently the following structure (IV): 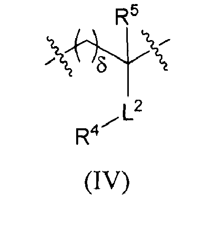 wherein:
R⁴ is the second reactive group;
R⁵ is hydrogen or alkyl
L² is an optional linker up to 100 atoms in length; and
δ is an integer ranging from 0 to 10.

13. The polymer of any one of claims 10-12, wherein the B subunit has, at each occurrence, independently the following structure (V): 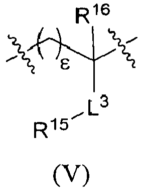 wherein:
R¹⁵ is the hydrophilic functional group;
R¹⁶ is hydrogen or alkyl
L³ is an optional linker up to 100 atoms in length; and
ε is an integer ranging from 0 to 10.

14. The polymer of claim 10, wherein the polymer has one of the following structures: 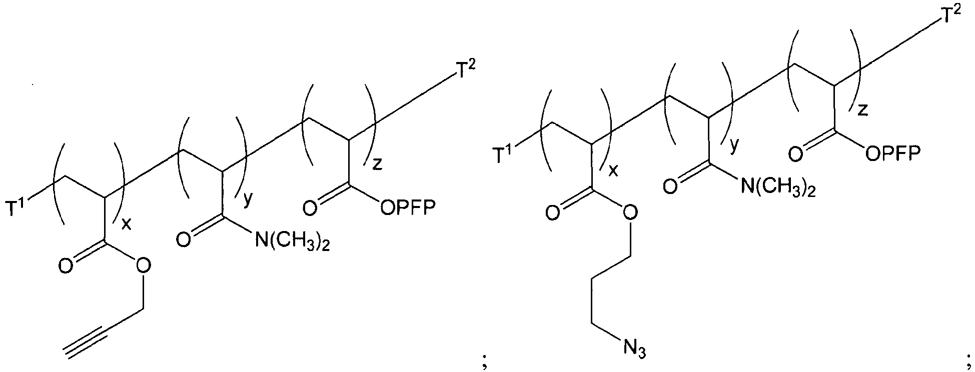 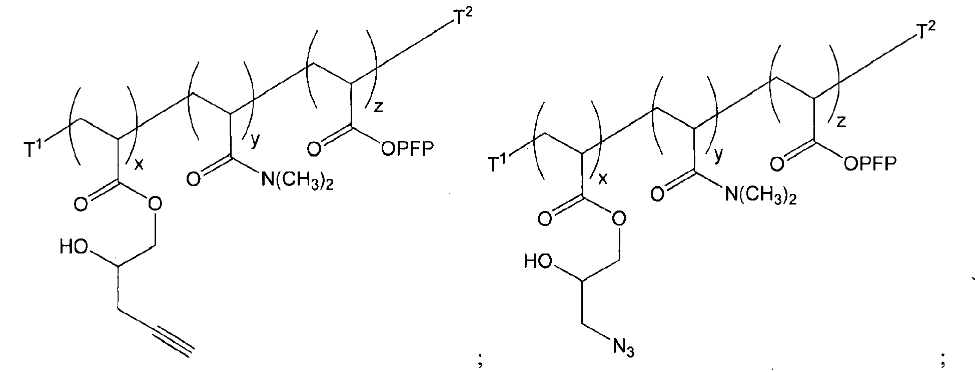 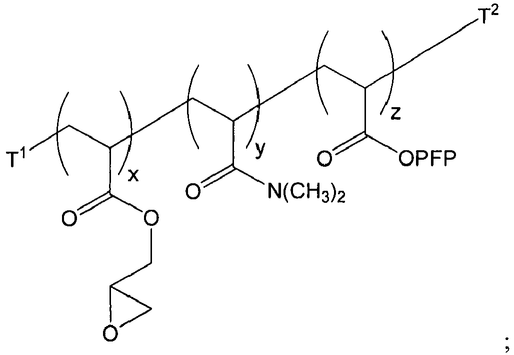 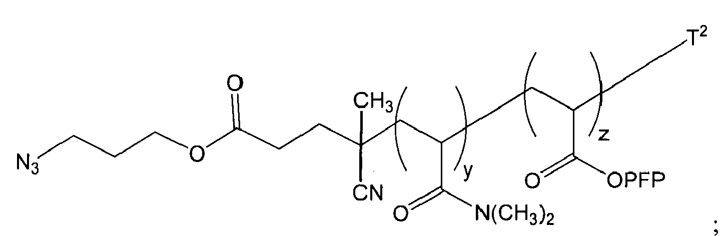 or 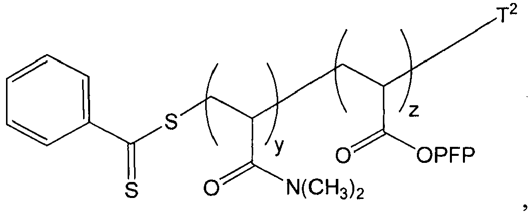 wherein:
the A, B and C subunits are present at least once in the polymer;
OPFP represents pentafluorophenoxy;
T¹ and T² are each independently absent or polymer terminal groups selected from H and alkyl;
x, y and z are each independently an integer from 1 to 350,000.

15. A method for determining the presence or absence of a target analyte molecule, the method comprising:
a) providing a solid support according to any of claims 1-9, wherein the D subunit comprises a capture probe covalently bound thereto;
b) contacting an analyte probe with the solid support; and
c) detecting the presence or absence of a signal produced from interaction of the capture probe with the analyte probe.
